# EUROPEAN PATENT APPLICATION

(11) **EP 3 184 536 A1**
(43) Date of publication of application: **28.06.2017**
(21) Application number: 15201829.7
(22) Date of filing: 22.12.2015
(51) Int. Cl.: C07H 3/04, A61K 31/7016, A61K 31/702, A61K 31/7028, A61K 31/715, A61K 31/739, C07H 3/06, C07H 3/08, C07H 15/12, A61P 31/04

(54) **OLIGOSACCHARIDE COMPOSITION FOR BINDING TO LECTINS**

(71) Applicant: Westfälische Wilhelms-Universität Münster, 48149 Münster (DE)
(72) Inventor: Müthing, Johannes, 33611 Bielefeld (DE); Pohlentz, Gottfried, 48329 Havixbeck (DE)
(74) Representative: Schiweck, Weinzierl & Koch Patentanwälte Partnerschaft mbB

(57) **Abstract**

The present invention relates to a method of preparing an oligosaccharide composition, said method comprising the steps of:
(a) providing a pectin; and
(b) performing, in any order, conversion of carboxylic acid groups to the corresponding alcohols and cleavage of glycosidic bonds under conditions suitable to give an oligosaccharide composition.

The present invention also relates to an oligosaccharide composition obtainable or being obtained by using such a method of preparing an oligosaccharide composition.

The present invention further relates to a method of preparing a coupling product of an oligosaccharide composition.

The present invention is also directed to a coupling product of an oligosaccharide composition.

The present invention also relates to pharmaceutical compositions comprising the oligosaccharide composition or the coupling product of an oligosaccharide composition as well as medical uses and diagnostic uses.

## Description

### FIELD OF THE INVENTION

The present invention relates to methods of preparing an oligosaccharide composition and an oligosaccharide composition obtainable or being obtained by such methods. Furthermore, the invention relates to methods of preparing a coupling product of an oligosaccharide composition such as, for example, a neoglycolipid composition. The present invention also relates to a coupling product of an oligosaccharide composition such as, for example, a neoglycolipid composition, which are obtainable or being obtained by such methods. In addition, the present invention relates to a pharmaceutical composition comprising an oligosaccharide composition and a pharmaceutical composition comprising a coupling product of an oligosaccharide composition. Also, the invention relates to applications of such oligosaccharide composition, coupling product of an oligosaccharide composition and pharmaceutical composition in medicine. The present invention also relates to a method of determining a lectin in a sample using a neoglycolipid composition of the invention and a kit suitable for determining of a lectin.

### BACKGROUND OF THE INVENTION

Lectin-producing pathogens and diseases caused by lectin-producing pathogens are a worldwide health risk.

An example for a lectin-producing pathogen is enterohemorrhagic *Escherichia coli* (EHEC), which produces Shiga toxin as a lectin. Diseases caused by EHEC such as, for example, hemorrhagic colitis and the hemolytic-uremic syndrome (HUS) represents a substantial health risk, since so far no causal treatment of such diseases is available (Trachtmann H, Austin C, Lewinski M, Stahl RAK. Renal and neurological involvement in typical Shiga toxin-associated HUS. 2012. Nat Rev Nephrol 8, 658-669, PetruzzielloPellegrini TN, Moslemi-NaeiniM, Marsden PA. New insights into Shiga toxin-mediated endothelial dysfunction in hemolytic uremic syndrome. 2013 Virulence 4, 556-563, Salvadori M, Bertoni E. Update on hemolytic uremic syndrome: diagnostic and therapeutic recommendations. 2013 World J Nephrol 2, 56-76). In particular, such deficiency of causal treatment options became clear during the last large EHEC outbreak of 2011 in Germany which was caused by an EHEC strain of O104:H4 serotype (Bielaszewska M, Mellmann A, Zhang W, Köck R, Fruth A Bauwens A, Peters G, Karch H. Characterisation of the Escherichia coli strain associated with an outbreak of haemolytic araemic syndrome in Germany, 2011: a microbiological study. 2011 Lancet Infect Dis 11, 671-676, Karch H, Denamur E. Dobrindt U. Finlay BB, Hengge R, Johannes L, Ron EZ, Tonjum T, Sansonetti PJ, Vicente M. The enemy within us: lessons from the 2011 European Escherichia coli O104:H4 outbreak. 2012 EMBO Mol Med 4, 841-848).

EHEC represents a subgroup of Shiga toxin producing *Escherichia coli* (STEC) which are pathogenic for humans. Another subgroup is formed by STEC which are pathogenic for animals. Such STEC which are pathogenic for animals may, for example, cause edema disease in pigs (Tseng M, Fratamico PM, Manning SD, Funk JA. Shiga toxin-producing Escherichia coli in swine: the public health perspective. 2014 Anim Health Res Rev 15, 63-75).

Humanpathogenic EHEC and swinepathogenic STEC produce Shiga toxins. Shiga toxins are cytotoxic proteins which belong to the group of lectins. Various subtypes of Shiga toxins are known. Their cytotoxic properties are caused by the binding of Shiga toxins to the cells. In particular, the initial step of cell damage is the binding of Shiga toxins to glycosphingolipids which are localized in the outer layer of the plasma membrane of the cells and protrude from the cell surface to the environment. By such binding a toxin-receptor complex is formed which is then internalized and which transports the toxin to its site of action.

Glycosphingolipids having neutral uncharged saccharide chains of the so-called globo series are specific receptors for Shiga toxins (Bauwens A, Betz J, Meisen I, Kemper B, Karch H, Müthing J. Facing glycosphingolipid-Shiga toxin interaction: dire straits for endothelial cells of the human vasculature. 2013 Cell Mol Life Sci 70, 425-457, Müsken A, Souady J, Dreisewerd K, Zhang W, Distler U, Peter-Katalinic J, Miller-Prodraza H, Karch H, Müthing J. Application of thin-layer chromatography/infrared matrix-assisted laser desorption/ionization orthogonal time-of-flight mass spectrometry to structural analysis of bacteria-binding glycosphingolipids selected by affinity detection. 2010 Rapid Commun Mass Spectrom 24, 1032-1038). For example, the Shiga toxins of subtypes Stx1a and Stx2a predominantly bind to the glycosphingolipid globotriaosylceramide (Gb3Cer) having the structure Galα1-4Galβ1-4Glcβ1-1Cer, wherein "Gal" denotes galactose, "Glc" denotes glucose, "Cer" denotes ceramide and "α1-4", "β1-4" and β1-1 denote the stereochemistry and the site of the linkage between the parts of the molecule (Müthing J, Meisen I, Zhang W, Bielaszewska M, Mormann M, Bauerfeind R, Schmidt MA, Friedrich AW, Karch H. Promiscuous Shiga toxin 2e and its intimate relationship to Forssman. 2012 Glycobiology 22, 849-862). To a lesser extent the Shiga toxins of subtypes Stx1 a and Stx2a bind to the glycosphingolipid globotetraosylceramide having the structure GalNAcβ1-3Galα1-4Galβ1-4Glcβ1-1Cer.

In Gb3Cer the epitope Galα1-4Galβ1-4Glc, which is also denoted as Pk-trisaccharide, is the central binding moiety for Shiga toxin. As this epitope provides binding of the Shiga toxin to the cell, treating a subject infected with a Shiga toxin producing pathogen with a compound comprising such Galα1-4Galβ1-4Glc moiety should lead to binding of the Shiga toxin *in vivo* thereby competing with/preventing binding of the Shiga toxin to the glycosphingolipids of the cell and thus preventing damage of the cell. The interactions between the Shiga toxin and the cell wall should be competitively inhibited by administering such a compound. In other words, the Shiga toxin should be neutralized

It has been found that the highly stereospecific configuration of the Galα1-4Galβ1-4Glc epitope is important for binding of Shiga toxin. In this respect, Shiga toxin does not bind to β1-4-glycosidic bound galactose and even does not recognize the isomeric Galα1-3Gal epitope. However, preparation of such highly stereospecific epitope and thus Shiga toxin receptors comprising such epitope via a chemical or chemo-enzymatic synthesis, in particular on a multigram scale using a recombinant α1-4-galactosyltransferase and UDP-galactose (Kamath VP, Yeske RE, Gregson JM, Ratcliffe RM, Fang YR, Palcic MM. Large-scale chemical and chemo-enzymatic synthesis of a spacer-containing Pk-trisaccharide. 2004 Carbohydr Res 339, 1141-1146.) requires a lot of technical effort and cost. Such technical effort becomes particularly noticeable when it is required to provide larger quantities for therapeutic applications. As a result, administering compounds comprising the Galα1-4Galβ-4Glc epitope for the treatment of a subject infected with a Shiga toxin producing pathogen is connected with large technical effort and cost for the synthesis of such compounds.

There is hence a need for providing methods of preparing compounds suitable for binding to Shiga toxin and/or other lectins which can be performed easily and a need for providing compounds suitable for binding to Shiga toxin and/or other lectins which are easily accessible.

### SUMMARY OF THE INVENTION

This need is addressed by the present invention as defined in the claims, described in the description, and illustrated in the Examples and Figures.

The present invention relates to a method of preparing an oligosaccharide composition, said method comprising the steps of:
(a) providing a pectin; and
(b) performing, in any order, conversion of carboxylic acid groups to the corresponding alcohols and cleavage of glycosidic bonds under conditions suitable to give an oligosaccharide composition.

In an embodiment of the method of preparing an oligosaccharide composition, said method comprises the steps of:
(a) providing a pectin;
(b1) conversion of carboxylic acid groups of the pectin to the corresponding alcohols; and
(b2) cleavage of glycosidic bonds of the conversion product of step (b1) under conditions suitable to give an oligosaccharide composition.

In another embodiment of the method of preparing an oligosaccharide composition, said method comprises the steps of:
(a) providing a pectin;
(b1) cleavage of glycosidic bonds of the pectin under conditions suitable to give an oligomeric pectin cleavage product; and
(b2) conversion of carboxylic acid groups of the cleavage product of step (b1) to the corresponding alcohols to give an oligosaccharide composition.

The present invention further relates to an oligosaccharide composition obtainable or being obtained by any one of the methods of preparing an oligosaccharide composition disclosed herein.

The present invention also relates to a method of preparing a coupling product of an oligosaccharide composition, said method comprising the steps of:
(a) providing an oligosaccharide composition as disclosed herein, in particular an oligosaccharide composition obtained by any one of the methods of preparing an oligosaccharide composition as disclosed herein; and
(b) forming a coupling product of an oligosaccharide composition from the oligosaccharide composition and one or more of a coupling partner.

The present invention further relates to a method of preparing a coupling product of an oligosaccharide composition, said method comprising the steps of:
(a1) providing a pectin;
(a2) cleavage of glycosidic bonds of the pectin under conditions suitable to give an oligosaccharide composition;
(b) forming a coupling product of an oligosaccharide composition from the oligosaccharide composition and one or more of a coupling partner; and
(c) conversion of carboxylic acid groups of the coupling product of step (b) to the corresponding alcohols.

In some embodiments the coupling product of an oligosaccharide composition is a neoglycolipid composition. In some embodiments the method of preparing a coupling product of an oligosaccharide composition is a method of preparing a neoglycolipid composition, said method comprising the steps of:
(a) providing an oligosaccharide composition as disclosed herein, in particular an oligosaccharide composition obtained by any one of the methods of preparing an oligosaccharide composition as disclosed herein; and
(b) forming a neoglycolipid composition from the oligosaccharide composition and one or more of a phosphatidylethanolamine having a structure of: 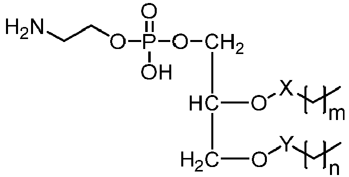 or any suitable salt thereof, wherein m and n is each, independently, an integer of from 8 to 20, wherein X and Y is each, independently, CH₂ or C=O; and wherein a carbon-nitrogen bond is formed between an oligosaccharide and a phosphatidylethanolamine.

In some embodiments the method of preparing a coupling product of an oligosaccharide composition is a method of preparing a neoglycolipid composition, said method comprising the steps of:
(a1) providing a pectin;
(a2) cleavage of glycosidic bonds of the pectin under conditions suitable to give an oligosaccharide composition;
(b) forming a neoglycolipid composition from the oligosaccharide composition and one or more of a phosphatidylethanolamine having a structure of: 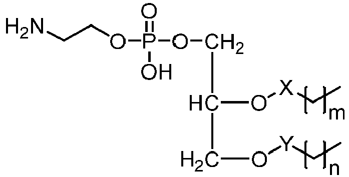 or any suitable salt thereof, wherein m and n is each, independently, an integer of from 8 to 20, wherein X and Y is each, independently, CH₂ or C=O; and wherein a carbon-nitrogen bond is formed between an oligosaccharide and a phosphatidylethanolamine; and
(c) conversion of carboxylic acid groups of the neoglycolipid composition of step (b) to the corresponding alcohols.

The present invention further relates to a coupling product of an oligosaccharide composition obtainable or being obtained by any one of the methods of preparing a coupling product of an oligosaccharide composition described herein. In a preferred embodiment the coupling product of an oligosaccharide composition is a neoglycolipid composition. A preferred embodiment relates to a neoglycolipid composition obtainable or being obtained by any one of the methods of preparing a neoglycolipid composition described herein. The neoglycolipid composition may comprise one or more of a neoglycolipid having the structure: 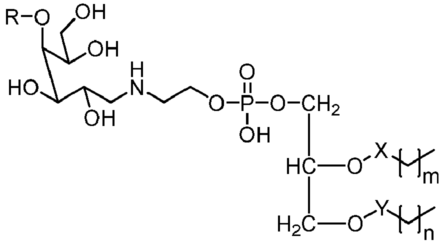 or any pharmaceutically acceptable salt thereof, wherein m and n is each, independently, an integer of from 8 to 20, wherein X and Y is each, independently, CH₂ or C=O; and wherein R is a residue comprising of from 1 to 19 saccharide units.

The present invention further relates to pharmaceutical compositions, medical uses, methods of treatment, methods of determining a lectin and a kit suitable for determining a lectin.

### BRIEF DESCRIPTION OF THE DRAWINGS

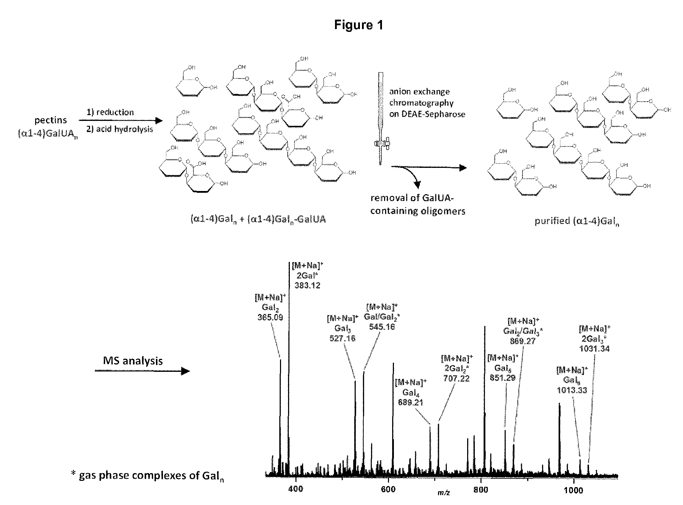

Figure 1 depicts schematically the preparation of an oligosaccharide composition according to the present invention from pectins as starting material and analysis of the oligosaccharides using mass spectrometry. "Galₙ" denotes oligomers of galactose comprising n galactose units, wherein "Gal" means galactose. "GalUAₙ" denotes oligomers of galacturonic acid comprising n galacturonic acid units, wherein "GalUA" means galacturonic acid. "α1-4" denotes the configuration of the glycosidic bonds between the saccharide units. Furthermore, Figure 1 depicts a mass spectrum of an oligosaccharide composition of the invention.
Figure 2 depicts the reaction of the oligosaccharides with a phosphatidylethanolamine to prepare neoglycolipids. "Galₙ" denotes oligomers of galactose comprising n galactose units, wherein "Gal" means galactose. "PE" denotes a phosphatidylethanolamine. "Galₙ-PE" denotes a neoglycolipid in which a galactose oligomer Galₙ is coupled to the phosphatidylethanolamine PE. The reaction is monitored by thin layer chromatography, wherein the formed neoglycolipids are visualized using orcinol as staining reagent. The integer n is from 2 to 9.
Figure 3 depicts a fragment ion mass spectrum and fragmentation scheme of neoglycolipid Gal₄PE comprising four galactose units coupled to a phosphatidylethanolamine.
Figure 4 depicts schematically a thin layer chromatography overlay assay. In the left chart a primary antibody and a secondary antibody are used in a reference method for detecting glycosphingolipids (GSLs). In the right chart a primary antibody and a secondary antibody are used for detecting a Shiga toxin in accordance with the present invention.
Figure 5 depicts thin layer chromatography overlay assays using a neoglycolipid composition comprising neoglycolipids Galₙ-PE of the present invention. In the left picture the neoglycolipids are detected using an anti-Gb3Cer antibody. In the right picture the neoglycolipids are detected using Shiga toxin of subtype Stx2a and an anti-Stx2 antibody. "Gb3Cer" denotes globotriaosylceramide and Gb4Cer denotes globotetraosylceramide.
Figure 6 depicts specific binding patterns of different Shiga toxin subtypes detected by thin layer chromatography overlay assays using a neoglycolipid composition comprising neoglycolipids Galₙ-PE according to the present invention.
Figure 7 depicts the mass spectrometric analysis of thin layer chromatography bands obtained by a thin layer chromatography overlay assay carried out using a neoglycolipid composition of the present invention and Shiga toxin subtype Stx2a.
Figure 8 depicts the mass spectrometric analysis of thin layer chromatography bands obtained by a thin layer chromatography overlay assay carried out using a neoglycolipid composition of the present invention and Shiga toxin subtype Stx1 a.
Figure 9 depicts the mass spectrometric analysis of thin layer chromatography bands obtained by a thin layer chromatography overlay assay carried out using a neoglycolipid composition of the present invention and Shiga toxin subtype Stx2e (human).
Figure 10 depicts binding patterns of Shiga toxin subtypes Stx2a, Stx1a and Stx2e (human) obtained by a thin layer chromatography overlay assay using a neoglycolipid composition according to the present invention.
Figure 11 depicts binding patterns of Shiga toxin subtype Stx2a from different strains of various serotypes of Shiga toxin producing *Escherichia coli* (STEC) obtained by a thin layer chromatography overlay assay using a neoglycolipid composition according to the present invention.
Figure 12 depicts binding patterns of Shiga toxin subtypes Stx1 a and Stx2e (human) from different strains of various serotypes of Shiga toxin producing *Escherichia coli* (STEC) obtained by a thin layer chromatography overlay assay using a neoglycolipid composition according to the present invention.
Figure 13 depicts schematically the binding of Shiga toxin (Stx) to globotriaosylceramide (Gb3Cer) receptors for Shiga toxin on the cell surface and the binding of Shiga toxin to oligosaccharides of an oligosaccharide composition of the present invention, thus preventing Shiga toxin from binding to globotriasosyl ceramide receptors of the cells.
Figure 14 depicts schematically the binding of Shiga toxin (Stx) to globotriaosylceramide (Gb3Cer) receptors for Shiga toxin on the cell surface and the binding of Shiga toxin to neoglycolipids of a neoglycolipid composition of the present invention formulated into liposomes, thus preventing Shiga toxin from binding to globotriaosylceramide receptors of the cells.
Figure 15 depicts the concentration-dependent Stx1a- and Stx2a-mediated cytotoxicity towards Vero-B4 cells determined using a cytotoxicity assay.
Figure 16 depicts the size distribution of liposomes with varying cholesterol content (denoted as "% Chol") containing a neoglycolipid composition of the present invention comprising neoglycolipids Galₙ-PE. The size distribution has been determined using dynamic light scattering.
Figure 17 depicts the influence of a neoglycolipid composition of the present invention formulated into liposomes comprising neoglycolipids Galₙ-PE and of globotriaosylceramide (Gb3Cer) on the cytotoxicity towards Vero-B4 cells mediated by Shiga toxin of subtype Stx1 a. The left side of the bar chart shows reference samples in which the cells have not been incubated with the Shiga toxin. The right side of the bar chart shows samples in which the cells have been incubated with Shiga toxin of subtype Stx1 a.
Figure 18 depicts the influence of a neoglycolipid composition of the present invention formulated into liposomes comprising neoglycolipids Galₙ-PE, of globotriaose coupled to a phosphatidylethanolamine (Gb3-PE) and of globotriaosylceramide (Gb3Cer) on the cytotoxicity towards Vero-B4 cells mediated by Shiga toxin of subtype Stx2a. The left side of the bar chart shows reference samples in which the cells have not been incubated with the Shiga toxin. The right side of the bar chart shows samples in which the cells have been incubated with Shiga toxin of subtype Stx2a.

### DETAILED DESCRIPTION OF THE INVENTION

Unless otherwise defined, scientific and technical terms used in connection with the disclosure shall have the meanings that are commonly understood by those of ordinary skill in the art. Further, unless otherwise required by context, singular terms shall include pluralities and plural terms shall include the singular. The nomenclatures utilized in connection with, and the laboratory procedures and techniques of, analytical chemistry, synthetic organic chemistry, and medicinal and pharmaceutical chemistry described herein are those well-known and commonly used in the art. Standard techniques are used for chemical syntheses, chemical analyses, pharmaceutical preparation, formulation, and delivery, and treatment of patients.

The following terms, definitions and abbreviations further apply:
The term "pectin", also known as pectic polysaccharides, in general denotes a complex polysaccharide associated with plant cell walls. A pectin comprises an α1-4 linked polygalacturonic acid backbone intervened by rhamnose residues and modified with neutral sugar side chains and non-sugar components such as acetyl, methyl, and ferulic acid groups. Because of the presence of neutral sugar side chains and some other non-sugar components, the structure of pectins is very complex; essentially no two molecules have identical structures. Rhamnose, galactose, arabinose, and xylose are the most common neutral sugar components of pectins. The less common ones are glucose, mannose, and fucose. Isolated pectin has a molecular weight of typically 60-130,000 g/mol, varying with origin and extraction conditions. Methylation may occur at carboxyl groups of galacturonic acid residues. In nature, around 80 percent of carboxyl groups of galacturonic acid are esterified with methanol. This proportion is decreased to a varying degree during pectin extraction. The degree of methyl-esterification is defined as the percentage of carboxyl groups (galacturonic acid residues) esterified with methanol. A pectin with a degree of esterification ("DE") above 50% is considered a high methoxyl ("HM") pectin and one with a DE below 50% is referred to as low methoxyl ("LM") pectin. Most of the natural pectins are HM with a few exceptions such as sunflower pectin. Pectin may contain some non-sugar components. Ferulic acid esters have been found in sugar beet pectin. They are linked to the arabinose and galactose residues in the neutral sugar side chains. The galacturonic acid residues in the pectin backbone are α1-4 linked. Both hydroxyl groups of D-galacturonic acid at carbon atoms 1 and 4 are in the axial position. The resulting linkage is therefore trans 1-4. Commercial pectins are mainly extracted from apple pomace or orange peels under hot acid conditions followed by alcohol precipitation.

The term "glycosidic bond" denotes a covalent bond that joins the anomeric carbon atom, i.e. the carbon atom of the hemiacetal group, of a saccharide unit and a hydroxyl group of another saccharide unit.

The term "saccharide unit" in general relates to a monosaccharide comprised in an oligosaccharide. In this regard, the term "saccharide unit" as used herein may also be referred to as "monosaccharide unit". For example, a saccharide unit as referred to herein may include galacturonic acid, galacturonic acid methyl ester and/or galactose, which derive from the pectin. Further, a saccharide unit as referred to herein may include rhamnose, arabinose, xylose, glucose, mannose and/or fucose, which represent further saccharides also contained in naturally occurring pectin.

The term "oligosaccharide" in general denotes a chemical compound composed of two or more saccharide units which are linked to each other via glycosidic bonds. An "oligosaccharide composition" as referred to herein may comprise oligosaccharides having different lengths which means that the oligosaccharides differ in respect to the number of their saccharide units. An oligosaccharide comprised in an oligosaccharide composition of the present invention may, for example, have from 2 to about 25 saccharide units, from 2 to about 20 saccharide units, from 2 to about 15 saccharide units, from 2 to about 10 saccharide units, from 3 to about 7 saccharide units, from 3 to about 6 saccharide units, or from 4 to 5 saccharide units. Specific examples of oligosaccharides, of which two or more may be present in the oligosaccharide composition, may comprise oligosaccharides having 2, 3, 4, 5, 6, 7, 8, 9, 10, 11, 12, 13, 14, 15, 16, 17, 18, 19, 20, 21, 22, 23, 24 or 25 saccharide units. According to a purely illustrative example, an oligosaccharide composition of the present invention may comprise two oligosaccharides such as a first oligosaccharide having 2 saccharide units and a second oligosaccharide having from 3 to 25 saccharide units. According to another purely illustrative example, the oligosaccharide composition may comprise three oligosaccharides, such as a first oligosaccharide having 2 saccharide units, a second oligosaccharide having 3 saccharide units and a third oligosaccharide having from 4 to 25 saccharide units. According to a still further example an oligosaccharide composition may comprise 4 oligosaccharides such as a first oligosaccharide having 2 saccharide units, a second oligosaccharide having 3 saccharide units, a third oligosaccharide having 4 saccharide units and a fourth oligosaccharide having from 5 to 25 saccharide units. It is also possible that two oligosaccharides present in the oligosaccharide composition have the same number of saccharide units so that, for example, a fourth and a fifth oligosaccharide have the same number of oligosaccharides. The amounts of oligosaccharides having a different number of saccharide units of the oligosaccharide composition may form a Gaussian distribution.

As known to a person skilled in the art the term "lectin" denotes a carbohydrate-binding protein which binds specifically to sugar moieties. In general, lectins perform recognition on the cellular and molecular level and play numerous roles in biological recognition phenomena involving cells, carbohydrates and proteins. For example, lectins also mediate attachment and binding of bacteria and viruses to their intended targets. Typically, lectins are proteins or glycoproteins having a molecular weight of from 8,500 to 300,000 which consist of 2 to 8 subunits. Lectins may be dissolved or released from a pathogen such as a bacterium. An example for a lectin released from a bacterium is Shiga toxin. On the other hand, lectins may be bound to the surface of the cell. For example, a lectin may be bound to the cell membrane. Examples for lectins bound to a cell membrane are the PapG adhesins which are located on the P-fimbriae of uropathogenic *Escherichia coli* (UPEC). Another example for a lectin which is an adhesin is SadP (streptococcal adhesin P) of *Streptococcus suis* (Kouki A, Haataja S, Loimaranta V, Pulliainen AT, Nilsson UJ, Finne J. 2011, Journal of Biological Chemistry 286(45), 38854-38864); Kouki A, Pieters RJ, Nilsson UJ, Loimaranta V, Finne J, Haataja S. Bacterial Adhesion of Streptococcus suis to Host Cells and Its Inihibition by Carbohydrate Ligands. 2013 Biology, 2, 918-935). The term "adhesin" as used herein in general denotes a cell surface component or appendage of bacteria that facilitate adhesion or adherence to other cells or surfaces.

The term "Shiga toxin" denotes a member of a family of related toxins which belong to the lectins. In general the toxin has two subunits and is one of the AB₅ toxins. The B subunit is a pentamer that binds to specific glycolipids on the host cell, in particular globotriaosylceramide (Gb3Cer). Following this binding, the A subunit is internalized and cleaved into two parts. The A1 component then binds to the ribosome, disrupting protein synthesis. For the purposes of the present disclosure the term "Shiga toxin" refers to any Shiga toxin independently of the source of the toxin. As non-limiting examples, Shiga toxins are found in bacteriae *Shigellae dysenteriae* and Shiga toxin producing *Escherichia coli* (STEC). Various subtypes of Shiga toxin are known. For example, the Shiga toxin from *Shigella dysenteriae* is also known as "true Shiga toxin" and abbreviated with "Stx". From Shiga toxin producing *Escherichia coli* (STEC) the Shiga toxin 1 (Stx1) and Shiga toxin 2 (Stx2) are known. From Shiga toxin 1 the subtypes Stx1 a, Stx1 c and Stx1 d are known. From Shiga toxin 2 the subtypes Stx2a, Stx2b, Stx2c, Stx2d, Stx2e, Stx2f and Stx2g are known. Stx, Stx1, Stx2, Stx1 a, Stx1 c, Stx1 d, Stx2a, Stx2b, Stx2c, Stx2d, Stx2e, Stx2f and Stx2g are encompassed by the term "Shiga toxin" as used herein. However, the term "Shiga toxin" as used herein is not limited to these subtypes. Shiga toxin 1 (Stx1) and Shiga toxin 2 (Stx2) may be also referred to as "Shiga-like toxin 1" and "Shiga-like toxin 2", respectively. Shiga toxins may be detected using Vero cell toxicity test. Therefore, further names for Shiga toxins are "verotoxins" or "verocytotoxins" which derives from the hypersensitivity of Vero cells to Shiga toxins. Accordingly, Shiga toxin producing *Escherichia coli* (STEC) is frequently denoted as "verotoxin producing *Escherichia coli* (VTEC)" or "verocytotoxin producing *Escherichia coli* (VTEC)" (European Centre for Disease Prevention and Control and European Food Saftey Authority. Shiga toxin/verotoxin-producing *Escherichia coli* in humans, food and animals in the EU/EEA, with special reference to the German outbreak strain STEC 0104. Stockholm: ECDC; 2011).

It was an object of the present invention to provide methods of preparing compounds suitable for binding to Shiga toxin and/or other lectins which can be performed easily, and to provide compounds suitable for binding to Shiga toxin and/or other lectins which are easily accessible.

Thus, the present invention relates to a method of preparing an oligosaccharide composition, said method comprising the steps of:
(a) providing a pectin; and
(b) performing, in any order, conversion of carboxylic acid groups to the corresponding alcohols and cleavage of glycosidic bonds under conditions suitable to give an oligosaccharide composition.

As a pectin is the starting material for the preparation of the oligosaccharide composition the oligosaccharide composition obtainable or being obtained by any one of the methods described herein above and below can be denoted as a "pectin-derived oligosaccharide composition". Similarly, any one of the methods described herein can be denoted as a method for preparing a pectin-derived oligosaccharide composition. The term "providing a pectin" as used within the context of the invention described herein means to arrange any pectin for a chemical reaction, such as, for example, conversion of carboxylic acid groups to the corresponding alcohols and/or cleavage of glycosidic bonds. For example, "providing a pectin" may include placing the pectin in the reaction vessel, combining the pectin with a suitable reaction medium and/or combining the pectin with a reactant. The pectin may be obtained from any source, such as, for example, from a commercial supplier. However, the term "providing a pectin" in general does not extend to obtaining the pectin, for example, by chemical reaction. The conversion of carboxylic acid groups to the corresponding alcohols can be also referred to as a reduction of the carboxylic acid groups to the corresponding alcohol groups.

The operations under step (b) can be performed in any order. Hence, the pectin may be first subjected to a conversion of the carboxylic acids groups to the corresponding alcohols. Then the glycosidic bonds of the obtained conversion product may be cleaved. Accordingly, an embodiment of the present invention provides a method of preparing an oligosaccharide composition or pharmaceutically acceptable salt or tautomer thereof, said method comprising the steps of:
(a) providing a pectin;
(b1) conversion of carboxylic acid groups of the pectin to the corresponding alcohols; and
(b2) cleavage of glycosidic bonds of the conversion product of step (b1) under conditions suitable to give an oligosaccharide composition.

Alternatively, the glycosidic bonds of the pectin may be cleaved first and then the carboxylic acid groups of the obtained cleavage product may be converted into the corresponding alcohols. Accordingly, another embodiment of the present invention provides a method for preparing an oligosaccharide composition or pharmaceutically acceptable salt or tautomer thereof, said method comprising the steps of:
(a) providing a pectin;
(b1) cleavage of glycosidic bonds of the pectin under conditions suitable to give an oligomeric pectin cleavage product; and
(b2) conversion of carboxylic acid groups of the cleavage product of step (b1) to the corresponding alcohols to give an oligosaccharide composition.

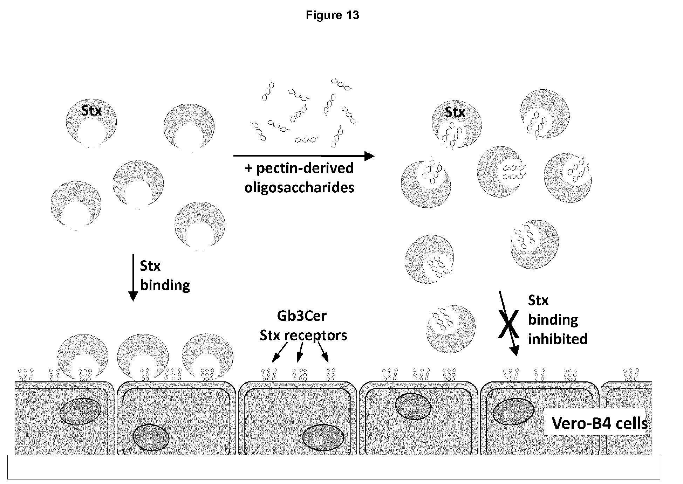

The oligosaccharide composition of the invention comprises oligosaccharides suitable for binding to lectins. As shown in Figure 13, in a purely illustrative example the oligosaccharides of the oligosaccharide composition bind to Shiga toxin. Such binding of the oligosaccharides to the Shiga toxin prevents/inhibits binding of the Shiga toxin to the cell. Hence, the cell is protected from damage by the Shiga toxin.

The methods of preparing an oligosaccharide composition described herein use a pectin as starting material. Pectins are readily available from natural sources and thus easily accessible starting materials. In this respect, pectins are commercially available in large quantities at comparably low cost. Furthermore, the cleavage of glycosidic bonds and the conversion of carboxylic acid groups into the corresponding alcohols of the methods of preparing an oligosaccharide composition of the invention can be performed easily. As set out above, the oligosaccharides comprised in the oligosaccharide composition are suitable for binding to lectins, such as, for example, a Shiga toxin or an adhesin. Since the starting material is a pectin which is readily available and since the method can be performed easily, the resulting oligosaccharide composition is easily accessible.

It has been shown that pectins derived from plants in their original native form do not exhibit any binding to lectins or exhibit only weak, i.e. neglectable, binding to lectins (Olano-Martin E, Williams MR, Gibson GR, Rastall RA. Pectins and pectic-oligosaccharides inhibit Escherichia coli O157:H7 Shiga toxin as directed towards the human colonic cell line HT29. 2003 FEMS Microbiol Lett 218, 101-105). Oligosaccharides derived from pectins have also been tested for their binding properties towards Shiga toxin (Olano-Martin E, Williams MR, Gibson GR, Rastall RA. Pectins and pectic-oligosaccharides inhibit Escherichia coli O157:H7 Shiga toxin as directed towards the human colonic cell line HT29. 2003 FEMS Microbiol Lett 218, 101-105). However, in these oligosaccharides the carboxylic acid groups have not been converted to the corresponding alcohols. It has been found that such oligosaccharides have to be applied in rather high amount thus indicating a relatively low binding activity to Shiga toxin. In the present invention it has been recognized that suitable oligosaccharides can be obtained by converting the carboxylic acid groups to the corresponding alcohols.

The cleavage of glycosidic bonds as performed during the course of any one of the methods of preparing an oligosaccharide composition described herein in general provides oligosaccharides having a different number of saccharide units. Therefore, an oligosaccharide composition obtained by any one of the methods of preparing an oligosaccharide composition described herein may comprise oligosaccharides having a different number of saccharide units. In particular, an oligosaccharide composition obtained as described herein may comprise a plurality of oligosaccharides having a different number of oligosaccharide units. In this regard, the term "plurality of oligosaccharides" denotes "two or more oligosaccharides". An oligosaccharide comprised in an oligosaccharide composition of the present invention may, for example, have from 2 to about 25 saccharide units, from 2 to about 20 saccharide units, from 2 to about 15 saccharide units, from 2 to about 10 saccharide units, from 3 to about 7 saccharide units, from 3 to about 6 saccharide units, or from 4 to 5 saccharide units. Specific examples of oligosaccharides, which may be present in the oligosaccharide composition, may include oligosaccharides having 2, 3, 4, 5, 6, 7, 8, 9, 10, 11, 12, 13, 14, 15, 16, 17, 18, 19, 20, 21, 22, 23, 24 or 25 saccharide units. Two or more oligosaccharides having a different number of saccharide units may be present in the oligosaccharide composition of the invention. As purely illustrative examples, the oligosaccharide composition may comprise two or more, three or more, four or more, five or more, six or more, seven or more, eight or more, nine or more or even ten or more of oligosaccharides having a different number of saccharide units. In the oligosaccharides comprised in the oligosaccharide composition of the present invention the saccharide units may be same or different. As set out above pectin has a polygalacturonic acid backbone. Therefore, the oligosaccharide composition obtained by the methods of the present invention, which include cleavage of glycosidic bonds and conversion of carboxylic acid groups to the corresponding alcohols, may comprise oligosaccharides in which all saccharide units are galactose units. On the other hand, the oligosaccharides may contain other saccharide units than galactose units deriving from the pectin such as, for example, galacturonic acid, galacturonic acid methyl ester, rhamnose, arabinose and/or xylose. An oligosaccharide composition of the present invention is prepared in Example 1 set out below. Figure 1 shows a purely illustrative preparation of an oligosaccharide composition of the present invention. Furthermore, Figure 1 shows a mass spectrum of such oligosaccharide composition in which oligosaccharides having different numbers of saccharide units have been identified.

Any one of such oligosaccharides functions as receptor for a lectin such as, for example, a Shiga toxin, a PapG adhesin or SadP. In this respect, oligosaccharides having different lengths are suitable for binding to a lectin. For the sake of convenience, the oligosaccharides are present in an oligosaccharide composition. If desired, the oligosaccharide composition may be subjected to separation, for example by chromatographic methods, in order to isolate oligosaccharides having different numbers of saccharide units. The isolated oligosaccharides may be stored separately. In respect to further applications, the oligosaccharides having different numbers of saccharide units may also be used separately.

The methods of preparing an oligosaccharide composition of the invention include that a carboxylic acid group -C(O)-OH is converted into the corresponding alcohol -CH₂-OH. In particular, this means that a galacturonic acid unit contained in the pectin or in the oligomeric pectin cleavage product is converted into the corresponding galactose unit. Such conversion can be performed as a full conversion which means that substantially all carboxylic acid groups present in the pectin or in the oligomeric pectin cleavage product are converted into the corresponding alcohol. Alternatively, it is possible to perform an only partial conversion of the carboxylic acid groups of the pectin or of the pectin cleavage product. In this regard, it is possible that only a part of the carboxylic acid groups comprised in the pectin or in the oligomeric pectin cleavage product is converted into the corresponding alcohol. As non-limiting examples, only 95%, 90%, 85%, 80%, 75%, 70%, 65%, 60%, 55% or even only 50% of the carboxylic acid groups comprised in the pectin or in the oligomeric pectin cleavage product may be converted into the corresponding alcohol, each based on 100% of the total number of carboxylic acid groups in the pectin or in the oligomeric pectin cleavage product before said conversion.

In case that only a partial conversion of the carboxylic acid groups to the corresponding alcohols is performed the oligosaccharide composition obtained by any one of the methods for preparing an oligosaccharide composition described herein above and below includes oligosaccharides which comprise both carboxylic acid groups and alcohol groups resulting from the conversion of the carboxylic acid groups into the corresponding alcohols. In other words, in case that only a partial conversion of the carboxylic acid groups into the corresponding alcohols is performed, the oligosaccharide composition or the oligomeric pectin cleavage product obtained by any one of the methods described herein comprises both galacturonic acid units and galactose units. In some of the methods disclosed herein above and below it may be therefore envisaged that the method further comprises, as an optional step, separation of oligosaccharides having non-reduced carboxylic acid groups. Figure 1 shows a purely illustrative preparation of an oligosaccharide composition in which oligomers containing oligomers are removed from the oligosaccharide composition using anion exchange chromatography. In this context the term "non-reduced" carboxylic acid groups denotes carboxylic acid groups which have not undergone conversion to the corresponding alcohols and have been therefore retained as carboxylic acid groups. Such separation may include an at least partial separation of oligosaccharides having non-reduced carboxylic acid groups from the oligosaccharide composition. On the other hand, oligosaccharides having non-reduced carboxylic acids groups may be separated completely from the oligosaccharide composition. In general, the separation of oligosaccharides having non-reduced carboxylic acid groups may be performed any time after the conversion of the carboxylic acid groups into the corresponding alcohols. For example, the separation of oligosaccharides having non-reduced carboxylic acid groups may be performed at the stage of the desired oligosaccharide composition after both steps (b1) and (b2) have been carried out. Accordingly, any one of the methods described herein above and below may comprise further that the oligosaccharide composition is subjected to at least partial separation of oligosaccharides having non-reduced carboxylic acid groups. In case that the conversion of the carboxylic acid groups of the pectin to the corresponding alcohols is performed before the cleavage of the glycosidic bonds, the at least partial separation of oligosaccharides having non-reduced carboxylic acid groups may be already performed after the conversion of carboxylic acid groups of the pectin to the corresponding alcohols and before the cleavage of glycosidic bonds.

The method for performing the separation of oligosaccharides having non-reduced carboxylic acids groups is not particularly limited and any suitable method known to a person skilled in the art may be used. For example, suitable methods for performing the separation of oligosaccharides having non-reduced carboxylic acid groups include, but are not limited to, anion exchange chromatography or extraction. In this regard, aqueous two-phase systems may be considered for extraction, in particular in case that the methods described herein are performed on an industrial scale. Regarding chromatography, besides anion exchange chromatography also other adsorbents which allow for separation of negatively charged oligosaccharides from neutral oligosaccharides, such as, for example, silica gel, may be used. A chromatographic separation of oligosaccharides having non-reduced carboxylic acid groups may, for example, include gradient elution wherein a gradient of polarity in the liquid phase further assists the separation of oligosaccharides having non-reduced carboxylic acid groups from neutral oligosaccharides. As a further method, separation of oligosaccharides having non-reduced carboxylic acid groups using complexing agents which allow for formation of a complex between the complexing agent and the carboxylic acid groups may be envisaged.

In case that the separation of oligosaccharides having non-reduced carboxylic acid groups is performed using anion exchange chromatography, any stationary phase suitable for the separation of compounds containing carboxylic acid groups may be used. For example, stationary phases which may be applied for anion exchange chromatography in any one of the methods disclosed herein include a cross-linked agarose modified with diethylaminoethyl groups, a cross-linked agarose modified with dimethylaminoethyl groups, a cross-linked agarose modified with trimethylaminoethyl groups, a cross-linked cellulose modified with diethylaminoethyl groups, a cross-linked cellulose modified with dimethylaminoethyl groups, or a cross-linked cellulose modified with trimethylaminoethyl groups. For the anion exchange chromatography only one of these stationary phases may be used or any combination of these stationary phases may be used. As a non-limiting example, a commercially available stationary phase which can be used for anion exchange chromatography in any one of the methods described herein is DEAE-Sephadex^{®} A-25 or DEAE-Sephadex^{®} A-50.

The method for performing the conversion of the carboxylic acid groups to the corresponding alcohols is not particularly limited, and any suitable method known to a person skilled in the art may be used. Preferably, in any one of the methods described herein above and below the conversion of the carboxylic acid groups to the corresponding alcohols comprises activating the carboxylic acid groups with a carbodiimide and reducing the obtained conjugate of the carboxylic acid groups and the carbodiimide. Thus, in a first step of such conversion the carboxylic acid group -C(O)-OH is reacted with a carbodiimide to form a conjugate of the carboxylic acid group with the carbodiimide. The conjugate is then reduced to give the corresponding alcohol -CH₂-OH. Preferably, the reducing agent for reducing the conjugate to the alcohol is a borohydride.

From a practical point of view, in some embodiments the methods for preparing an oligosaccharide composition described herein above may be performed in an aqueous medium, preferably an aqueous solution, since the reactants and products are soluble in water and since water is a readily available solvent. In this regard, any one of the steps of cleavage of glycosidic bonds and conversion of carboxylic acid groups to the corresponding alcohols may be performed in an aqueous medium, such as, for example, an aqueous solution. Preferably, both the cleavage of glycosidic bonds and the conversion of carboxylic acid groups to the corresponding alcohols may be performed in an aqueous medium, such as, for example, an aqueous solution. It is therefore preferred that the borohydride to be used for reducing the conjugate to the alcohol is compatible with aqueous conditions. Non-limiting examples for such borohydrides which are relatively stable in the presence of water are lithium borohydride (LiBH₄), sodium borohydride (NaBH₄) and potassium borohydride (KBH₄). Accordingly, in any one of the methods for preparing an oligosaccharide composition described herein the borohydride may be selected from the group consisting of lithium borohydride, sodium borohydride, potassium borohydride and any combination thereof. Preferably, the borohydride is sodium borohydride which is readily available and provides a suitable reactivity and stability.

The carbodiimide, which is used to form the conjugate of the carboxylic acid group and the carbodiimide, is not particularly limited as long as the obtained conjugate is able to undergo reduction to the corresponding alcohol. Suitable carbodiimides which may be used in any one of the methods for preparing an oligosaccharide composition disclosed herein include *N*-cyclohexyl-*N'*-(2-morpholinoethyl)carbodiimide metho-*p*-toluenesulfonate (CMC) and/or 1-ethyl-3-(3-dimethylaminopropyl)carbodiimide (EDC). In this regard, *N*-cyclohexyl-*N'-*(2-morpholinoethyl)carbodiimide metho-*p*-toluenesulfonate (CMC) and 1-ethyl-3-(3-dimethylaminopropyl)carbodiimide (EDC) may be used alone or in combination. In case that the conversion is carried out in an aqueous medium, *N*-cyclohexyl-*N'*-(2-morpholinoethyl)carbodiimide metho-*p*-toluenesulfonate (CMC) is preferred since this carbodiimide is a salt which is readily soluble in water.

In some embodiments of the methods for preparing an oligosaccharide composition described herein the composition obtained by the conversion of the carboxylic acid groups to the corresponding alcohols is purified. In particular, by such purification low molecular weight substances can be separated from the oligosaccharides having a higher molecular weight. For example, by such purification residual reducing agent and products formed from the reducing agent, such as salts, may be separated. Non-limiting purification methods suitable for being used in any one of the methods described herein include dialysis, ultrafiltration and size exclusion chromatography. It is also possible to use a combination of said methods. Preferably, the composition is purified using dialysis. The stage of the methods for preparing an oligosaccharide composition described herein at which the purification is carried out is not particularly limited. For example, in case that the conversion of carboxylic acid groups to the corresponding alcohols is performed before the cleavage of the glycosidic bonds, the purification may be carried out after the conversion of the carboxylic acid groups to the corresponding alcohols and before the cleavage of the glycosidic bonds. Alternatively, in case that the cleavage of glycosidic bonds is performed before the conversion of carboxylic acid groups to the corresponding alcohols, the purification may be carried out after the conversion of the carboxylic acid groups to the corresponding alcohols. In case that further a separation of oligosaccharides having non-reduced carboxylic acid groups is envisaged, the purification may be carried out before or after such separation of oligosaccharides having non-reduced carboxylic acid groups.

As set out above, the purification of the composition obtained by the conversion of the carboxylic acid groups to the corresponding alcohol may be carried out using a method selected from the group consisting of dialysis, ultrafiltration and size exclusion chromatography. In case that dialysis is used, the dialysis may be, as a non-limiting example, carried out against deionized water. For this purpose, the composition to be purified is placed in a dialysis tube which is then placed in the deionized water. The conditions of the dialysis may be appropriately selected by the person skilled in the art. For example, the dialysis time may be selected in order to achieve a desired degree of purity or the deionized water may be exchanged during the dialysis. As a mere non-limiting example, the dialysis tube may have a molecular weight cut-off of 14000 Da. In case that ultrafiltration is used, as mere non-limiting examples the molecular weight cut-off may be 50 kD or 100 kD. In case that size exclusion chromatograph is used, gel permeation chromatography may be employed.

In order to bring the oligosaccharide composition into a transportable and storable form, the oligosaccharide composition may be dried. By such drying the oligosaccharide composition may be obtained in a solid form. The drying method is not particularly limited and any suitable drying method known to a person skilled in the art may be employed. For example, the drying may be carried out using freeze-drying, spray drying, heat drying, vacuum drying, fluidized bed drying and/or spray granulation. Preferably, the drying may be performed using freeze-drying, which is also known as lyophilization. In this regard, lyophilization provides a gentle method for drying the oligosaccharide composition of the present invention.

According to the present invention any one of the methods for preparing an oligosaccharide composition described herein above and below comprises cleavage of glycosidic bonds. In this regard, the method for performing cleavage of glycosidic bonds is not particularly limited, and any method suitable for cleavage of glycosidic bonds known to a skilled person may be used. As set out above, the cleavage of glycosidic bonds may be performed in an aqueous medium. Accordingly, in preferred embodiments of the methods of preparing an oligosaccharide composition disclosed herein the cleavage of glycosidic bonds is performed using hydrolysis of glycosidic bonds. As known to the skilled person the term "hydrolysis" in general means cleavage of a chemical bond by addition of water. Accordingly, a hydrolysis of glycosidic bonds as referred to herein denotes the cleavage of a glycosidic bond by addition of water. In this regard, the hydrolysis of glycosidic bonds in any one of the methods for preparing an oligosaccharide composition is preferably performed in an aqueous medium, in particular in water.

According to a preferred embodiment of the methods for preparing an oligosaccharide composition the hydrolysis of glycosidic bonds is performed in presence of an acid. Thus, the hydrolysis of glycosidic bonds may be performed in an acidic aqueous medium. The acid is not particularly limited. In principle, strong acids such as hydrochloric acid (HCl), sulfuric acid (H₂SO₄) or trifluoroacetic acid may be used. These acids may be used alone or any combination thereof may be used. Trifluoroacetic acid is preferred from the viewpoint that this acid can be readily removed from the oligosaccharide composition during lyophilization and that the trifluoroacetate anion can be easily separated using anion exchange chromatography. The hydrolysis is performed under conditions which provide an oligosaccharide composition. As defined herein above, an oligosaccharide composition as referred to herein may comprise same or different oligosaccharides which differ in the number of the saccharide units. In embodiments of the methods for preparing an oligosaccharide composition described herein the hydrolysis of glycosidic bonds is performed at a temperature of from 70 to 120 °C for 5 min to 3 hours. Preferably, the hydrolysis is performed at a temperature of from 80 to 110 °C for 10 minutes to 60 minutes. More preferably, the hydrolysis is performed at a temperature of from 90 to 95 °C for 30 to 40 minutes. Most preferably, the hydrolysis is performed at a temperature of from 100 to 105 °C for 15 to 20 minutes. The pH of the reaction mixture may be between 2 and 5. Preferably, the pH of the reaction mixture is between 2.1 and 4. More preferably, the pH is between 2.2 and 3.5. Most preferably, the pH is between 2.3 and 3.0.

In any one of the methods of preparing an oligosaccharide composition described herein the hydrolysis of glycosidic bonds may be performed using an enzyme. In case that the cleavage of the glycosidic bond is performed after the conversion of carboxylic acid groups of the pectin to the corresponding alcohols suitable enzymes which are able to mediate cleavage of glycosidic bonds between galactose units of the conversion product, include endo-α-galactosidases. In case that the cleavage of the glycosidic bonds is performed before the reduction of carboxylic acid groups to the corresponding alcohols, i.e. in case that the cleavage is performed on the pectin, suitable enzymes which are able to mediate cleavage of glycosidic bonds between galacturonic acid units of the pectins and which can be used herein include pectinases. Such pectinases which may be useful in the methods described herein comprise pectolyase, pectozyme and polygalacturonidase. Such enzymes may be used alone, or any combination thereof may be employed. Regarding the conditions for an enzymatic hydrolysis of glycosidic bonds in a pectin, the pectinase may be typically used at a temperature of from 40 °C to 55 °C and at a pH of from 3 to 6.5, preferably from 3 to 5. In case that a pectinase is employed, the obtained oligomeric pectin cleavage product is further subjected to conversion of carboxylic acid groups of the cleavage product to the corresponding alcohols to give the oligosaccharide composition. For the further purification of the oligosaccharide composition graphitized carbon may be used. Likewise, the oligosaccharide composition may be purified using a sequence comprising the steps of peracetylation, adsorption chromatography on silica gel and deacetylation.

The compositions obtained by any one of the methods described herein may be analyzed for detecting the number of saccharide units comprised in the oligosaccharides. Accordingly, in any one of the methods for preparing an oligosaccharide composition described herein above and below the composition after cleavage of glycosidic bonds may be analyzed using mass spectrometry (MS), high performance liquid chromatography (HPLC), capillary gel electrophoresis and/or hydrophilic interaction chromatography. These analytic methods allow showing that the obtained composition comprises oligosaccharides and further allow to detect the number of saccharide units in the oligosaccharides. In other words, these analytic methods allow to determine how many monosaccharide units are comprised in the oligosaccharides. In this respect, a mass spectrum of an oligosaccharide composition prepared according to any one of the methods described herein may show oligosaccharides composed of, for example, 2, 3, 4, 5, 6, 7, 8, 9, 10, 11, 12, 13, 14, 15, 16, 17, 18, 19, 20 or even more monosaccharide units. The term "after cleavage of glycosidic bonds" does not necessarily mean that the composition has to be analyzed directly after performing the cleavage. The analysis can be performed any time after the cleavage step. As a non-limiting example, in case that the cleavage of glycosidic bonds is performed after the conversion of carboxylic acid groups into the corresponding alcohols, the analysis may be performed after the conversion of carboxylic acid groups to the corresponding alcohols and even after further steps such as, for example, separating oligosaccharides having non-reduced carboxylic acid groups and/or dialysis. On the other hand, in case that the cleavage of glycosidic bonds is performed before the conversion of carboxylic acid groups to the corresponding alcohols, the analysis may be performed, as a non-limiting example, after the cleavage of the glycosidic bonds and before the conversion of carboxylic acid groups to the corresponding alcohols.

In an embodiment the composition after cleavage of glycosidic bonds is analyzed using mass spectrometry. Mass spectrometry techniques are in general known to the person skilled in the art, and any suitable mass spectrometry technique may be used for the analysis. For example, in any one of the methods described herein the mass spectrometry may be performed using atmospheric pressure chemical ionization (APCI), direct chemical ionization (DCI), electrospray ionization (ESI), fast atom bombardment (FAB), or matrix-assisted laser desorption ionization (MALDI). Preferably, the mass spectrometry is performed using electrospray ionization (ESI) or matrix-assisted laser desorption ionization (MALDI). If electrospray ionization is used, preferably nano electrospray ionization mass spectrometry (nanoESI) is employed.

In an embodiment the composition after cleavage of glycosidic bonds is analyzed using HPLC. In case that HPLC is used for analyzing the composition, the HPLC may be performed using high-performance anion exchange chromatography - pulsed amperometric detection (HPAEC-PAD). In general, this method is known to the skilled person. In case that HPLC is used for performing the analysis, in any HPLC method defined galactose oligomers may be used as standard. Such defined galactose oligomers consist of a defined number of galactose units. In general, such defined galactose oligomers can be prepared by a skilled person using known methods or can be obtained from commercial suppliers, such as, for example, from Elicityl SA, Crolles, France.

In an embodiment the composition after cleavage of glycosidic bonds is analyzed using capillary gel electrophoresis. In case that capillary gel electrophoresis is used for analyzing the composition, the capillary gel electrophoresis may be performed using capillary gel electrophoresis with laser-induced fluorescence (CGEL-LIF).

In an embodiment the composition after cleavage is analyzed using hydrophilic interaction chromatography.

In any one of the methods for preparing an oligosaccharide composition described herein the cleavage of glycosidic bonds may be terminated at a time at which one or more of an oligosaccharide selected from the group consisting of an oligosaccharide having 20 saccharide units, an oligosaccharide having 19 saccharide units, an oligosaccharide having 18 saccharide units, an oligosaccharide having 17 saccharide units, an oligosaccharide having 16 saccharide units, an oligosaccharide having 15 saccharide units, an oligosaccharide having 14 saccharide units, an oligosaccharide having 13 saccharide units, an oligosaccharide having 12 saccharide units, an oligosaccharide having 11 saccharide units, an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units is present in the reaction mixture. The point in time when to terminate the cleavage of glycosidic bonds in order to obtain oligosaccharides having the aforementioned number of saccharide units may be easily determined by terminating the reaction and analyzing the composition by any one of the methods described above. For example, the composition may be analyzed using mass spectrometry. The cleavage of the glycosidic bonds may be terminated by cooling the reaction mixture to about 4 °C. In any one of the methods of preparing an oligosaccharide composition the cleavage of glycosidic bonds may be terminated at a time at which a combined ratio of oligosaccharides having 20 saccharide units, oligosaccharides having 19 saccharide units, oligosaccharides having 18 saccharide units, oligosaccharides having 17 saccharide units, oligosaccharides having 16 saccharide units, oligosaccharides having 15 saccharide units, oligosaccharides having 14 saccharide units, oligosaccharides having 13 saccharide units, oligosaccharides having 12 saccharide units, oligosaccharides having 11 saccharide units, oligosaccharides having 10 saccharide units, oligosaccharides having 9 saccharide units, oligosaccharides having 8 saccharide units, oligosaccharides having 7 saccharide units, oligosaccharides having 6 saccharide units, oligosaccharides having 5 saccharide units, oligosaccharides having 4 saccharide units, oligosaccharides having 3 saccharide units, and oligosaccharides having 2 saccharide units is at least 70 % by weight, preferably at least 80 % by weight, more preferably at least 90 % by weight, still more preferably at least 95 % by weight and most preferably at least 98 % by weight based on 100 % by weight of the combined ratio of all saccharides in the reaction mixture. This ratio can be, for example, determined using HPLC using defined galactose oligomers as standard as described above. For example, the HPLC may be performed using HPAEC-PAD. The balance to 100 % by weight may be formed by oligosaccharides having more than 20 saccharide units, polysaccharides and/or monosaccharides obtained from the pectin. In this regard, the term "all saccharides" encompasses any saccharide present in the reaction mixture including, for example, oligosaccharides, polysaccharides and/or monosaccharides obtained from the pectin.

In an embodiment the cleavage of glycosidic bonds is terminated at a time at which one or more of an oligosaccharide selected from the group consisting of 15 saccharide units, an oligosaccharide having 14 saccharide units, an oligosaccharide having 13 saccharide units, an oligosaccharide having 12 saccharide units, an oligosaccharide having 11 saccharide units, an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units is present in the reaction mixture. In any one of the methods of preparing an oligosaccharide composition the cleavage of glycosidic bonds may be terminated at a time at which a combined ratio of oligosaccharides having 15 saccharide units, oligosaccharides having 14 saccharide units, oligosaccharides having 13 saccharide units, oligosaccharides having 12 saccharide units, oligosaccharides having 11 saccharide units, oligosaccharides having 10 saccharide units, oligosaccharides having 9 saccharide units, oligosaccharides having 8 saccharide units, oligosaccharides having 7 saccharide units, oligosaccharides having 6 saccharide units, oligosaccharides having 5 saccharide units, oligosaccharides having 4 saccharide units, oligosaccharides having 3 saccharide units, and oligosaccharides having 2 saccharide units is at least 70 % by weight, preferably at least 80 % by weight, more preferably at least 90 % by weight, still more preferably at least 95 % by weight and most preferably at least 98 % by weight based on 100 % by weight of the combined ratio of all saccharides in the reaction mixture. This ratio can be, for example, determined using HPLC using defined galactose oligomers as standard as described above. For example, the HPLC may be performed using HPAEC-PAD. The balance to 100 % by weight may be formed by oligosaccharides having more than 15 saccharide units, polysaccharides and/or monosaccharides obtained from the pectin. In this regard, the term "all saccharides" encompasses any saccharide present in the reaction mixture including, for example, oligosaccharides, polysaccharides and/or monosaccharides obtained from the pectin.

In an embodiment the cleavage of glycosidic bonds is terminated at a time at which one or more of an oligosaccharide selected from the group consisting of an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units is present in the reaction mixture. In any one of the methods of preparing an oligosaccharide composition the cleavage of glycosidic bonds may be terminated at a time at which a combined ratio of oligosaccharides having 10 saccharide units, oligosaccharides having 9 saccharide units, oligosaccharides having 8 saccharide units, oligosaccharides having 7 saccharide units, oligosaccharides having 6 saccharide units, oligosaccharides having 5 saccharide units, oligosaccharides having 4 saccharide units, oligosaccharides having 3 saccharide units, and oligosaccharides having 2 saccharide units is at least 70 % by weight, preferably at least 80 % by weight, more preferably at least 90 % by weight, still more preferably at least 95 % by weight and most preferably at least 98 % by weight based on 100 % by weight of the combined ratio of all saccharides in the reaction mixture. This ratio can be, for example, determined using HPLC using defined galactose oligomers as standard as described above. For example, the HPLC may be performed using HPAEC-PAD. The balance to 100 % by weight may be formed by oligosaccharides having more than 10 saccharide units, polysaccharides and/or monosaccharides obtained from the pectin. In this regard, the term "all saccharides" encompasses any saccharide present in the reaction mixture including, for example, oligosaccharides, polysaccharides and/or monosaccharides obtained from the pectin.

In an embodiment the cleavage of glycosidic bonds is terminated at a time at which one or more of an oligosaccharide selected from the group consisting of an oligosaccharide having 7 saccharide units, an oligosaccharide having 6 saccharide units, an oligosaccharide having 5 saccharide units, an oligosaccharide having 4 saccharide units, and an oligosaccharide having 3 saccharide units is present in the reaction mixture. In any one of the methods of preparing an oligosaccharide composition the cleavage of glycosidic bonds may be terminated at a time at which a combined ratio of oligosaccharides having 7 saccharide units, oligosaccharides having 6 saccharide units, oligosaccharides having 5 saccharide units, oligosaccharides having 4 saccharide units, and oligosaccharides having 3 saccharide units is at least 70 % by weight, preferably at least 80 % by weight, more preferably at least 90 % by weight, still more preferably of at least 95 % by weight and most preferably at least 98 % by weight based on 100 % by weight of the combined ratio of all saccharides in the reaction mixture. This ratio can be, for example, determined using HPLC using defined galactose oligomers as standard as described above. For example, the HPLC may be performed using HPAEC-PAD. The balance to 100 % by weight may be formed by oligosaccharides having more than 7 saccharide units, polysaccharides, disaccharides and/or monosaccharides obtained from the pectin. In this regard, the term "all saccharides" encompasses any saccharide present in the reaction mixture including, for example, oligosaccharides, polysaccharides, disaccharides and/or monosaccharides obtained from the pectin.

In an embodiment the cleavage of glycosidic bonds is terminated at a time at which one or more of an oligosaccharide selected from the group consisting of an oligosaccharide having 6 saccharide units, an oligosaccharide having 5 saccharide units, an oligosaccharide having 4 saccharide units, and an oligosaccharide having 3 saccharide units is present in the reaction mixture. In any one of the methods of preparing an oligosaccharide composition the cleavage of glycosidic bonds may be terminated at a time at which a combined ratio of oligosaccharides having 6 saccharide units, oligosaccharides having 5 saccharide units, oligosaccharides having 4 saccharide units, and oligosaccharides having 3 saccharide units is at least 70 % by weight, preferably at least 80 % by weight, more preferably at least 90 % by weight, still more preferably at least 95 % by weight and most preferably at least 98 % by weight based on 100 % by weight of the combined ratio of all saccharides in the reaction mixture. This ratio can be, for example, determined using HPLC using defined galactose oligomers as standard as described above. For example, the HPLC may be performed using HPAEC-PAD. The balance to 100 % by weight may be formed by oligosaccharides having more than 7 saccharide units, polysaccharides, disaccharides and/or monosaccharides obtained from the pectin. In this regard, the term "all saccharides" encompasses any saccharide present in the reaction mixture including, for example, oligosaccharides, polysaccharides, disaccharides and/or monosaccharides obtained from the pectin.

In an embodiment the cleavage of glycosidic bonds is terminated at a time at which one or more of an oligosaccharide selected from the group consisting of an oligosaccharide having 5 saccharide units, and an oligosaccharide having 4 saccharide units is present in the reaction mixture. In any one of the methods of preparing an oligosaccharide composition the cleavage of glycosidic bonds may be terminated at a time at which a combined ratio of oligosaccharides having 5 saccharide units and oligosaccharides having 4 saccharide units is at least 70 % by weight, preferably at least 80 % by weight, more preferably at least 90 % by weight, still more preferably at least 95 % by weight and most preferably at least 98 % by weight based on 100 % by weight of the combined ratio of all saccharides in the reaction mixture. This ratio can be, for example, determined using HPLC using defined galactose oligomers as standard as described above. For example, the HPLC may be performed using HPAEC-PAD. The balance to 100 % by weight may be formed by oligosaccharides having more than 5 saccharide units, disaccharides, trisaccharides, polysaccharides and/or monosaccharides obtained from the pectin.

In some embodiments the oligosaccharides comprise one or more of a saccharide unit selected from the group consisting of galactose, galacturonic acid, and galacturonic acid alkyl ester. In this regard, in the methods described herein galactose units are obtained by conversion of the carboxylic acid groups of glucuronic acid units into the corresponding alcohols. Galacturonic acid units may be present in case that only a part of the galacturonic acid units has been converted into the corresponding alcohols. Furthermore, since pectins contain esterified carboxylic acid groups, the oligosaccharides may contain galacturonic acid alkyl ester units which have not been reduced. The alkyl ester may be a C1-C6 alkyl ester, a C1-C5 alkyl ester, a C1-C4 alkyl ester, a C1-C3 alkyl ester, a C1-C2 alkyl ester or a C1 alkyl ester. Non-limiting examples for suitable alkyl esters include a methyl ester, an ethyl ester, an *n*-propyl ester, an *iso*-propyl ester, an *n*-butyl ester, a *sec*-butyl ester, an *iso*-butyl ester, a *tert*-butyl ester, a pentyl ester and a hexyl ester. Preferably, the alkyl ester is a methyl ester. In this regard, in naturally occurring pectins a part of the carboxylic acid groups is esterified with methanol to form a methyl ester.

The source of the pectin to be employed for the methods of preparing an oligosaccharide composition described herein is not particularly limited. Virtually any pectin may be used. In particular, the pectin may be selected from the group consisting of pectin from apples, pectin from citrus fruits, pectin from vegetables, pectin from carrots, pectin from sugar beets, pectin from fodder beets, pectin from mangold, pectin from sunflower heads, and any combination thereof. The pectin used in the methods of preparing an oligosaccharide composition described herein may be a high methoxyl pectin or a low methoxyl pectin, wherein the term "high methoxyl" denotes a pectin having a degree of esterification (DE) of above 50 % and "low methoxyl" denotes a pectin having a degree of esterification (DE) of below 50 %. As purely illustrative examples, high methoxyl pectin having a degree of esterification of > 70 % (such as, for example, a high methoxyl pectin commercially available from Elicityl SA, Crolles, France), high methoxyl pectin having a degree of esterification of 75 % (such as, for example, a high methoxyl pectin commercially available from Herbstreith & Fox KG, Neuenbürg, Germany), low methoxyl pectin having a degree of esterification of < 5 % (such as, for example, a low methoxyl pectin commercially available from Elicityl SA, Crolles, France) and/or low methoxyl pectin having a degree of esterification of 25 % (such as, for example, a low methoxyl pectin commercially available from Herbstreith & Fox KG, Neuenbürg, Germany) may be employed in any one of the methods of preparing an oligosaccharide composition in accordance with the invention. The inventors have found that low methoxyl pectins provide particularly good results in the conversion of the carboxylic acid groups to the corresponding alcohols.

The present invention further relates to an oligosaccharide composition obtainable or being obtained by any one of the methods for preparing an oligosaccharide composition disclosed herein. Accordingly, the present invention is also directed to an oligosaccharide composition obtainable or being obtained by a method comprising the steps of:
(a) providing a pectin; and
(b) performing, in any order, conversion of carboxylic acid groups to the corresponding alcohols and cleavage of glycosidic bonds under conditions suitable to give an oligosaccharide composition.

In an embodiment the present invention is directed to an oligosaccharide composition obtainable or being obtained by a method comprising the steps of:
(a) providing a pectin;
(b1) conversion of carboxylic acid groups of the pectin to the corresponding alcohols; and
(b2) cleavage of glycosidic bonds of the conversion product of step (b1) under conditions suitable to give an oligosaccharide composition.

In another embodiment the present invention is directed to an oligosaccharide composition or pharmaceutically acceptable salt or tautomer obtainable or being obtained by a method comprising the steps of:
(a) providing a pectin;
(b1) cleavage of glycosidic bonds of the pectin under conditions suitable to give an oligomeric pectin hydrolysis product; and
(b2) conversion of carboxylic acid groups of the hydrolysis product of step (b1) to the corresponding alcohols to give an oligosaccharide composition.

Any oligosaccharide composition described herein may be obtainable or being obtained by any one of the methods of preparing an oligosaccharide composition described herein. As set out above in the context of the methods of preparing an oligosaccharide composition, an oligosaccharide composition described herein may comprise oligosaccharides having a different number of saccharide units.

In an embodiment the oligosaccharide composition comprises one or more of an oligosaccharide selected from the group consisting of an oligosaccharide having 20 saccharide units, and oligosaccharide having 19 saccharide units, an oligosaccharide having 18 saccharide units, an oligosaccharide having 17 saccharide units, an oligosaccharide having 16 saccharide units, an oligosaccharide having 15 saccharide units, an oligosaccharide having 14 saccharide units, an oligosaccharide having 13 saccharide units, an oligosaccharide having 12 saccharide units, an oligosaccharide having 11 saccharide units, an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units. In an embodiment of the oligosaccharide composition a combined ratio of oligosaccharides having 20 saccharide units, oligosaccharides having 19 saccharide units, oligosaccharides having 18 saccharide units, oligosaccharides having 17 saccharide units, oligosaccharides having 16 saccharide units, oligosaccharides having 15 saccharide units, oligosaccharides having 14 saccharide units, oligosaccharides having 13 saccharide units, oligosaccharides having 12 saccharide units, oligosaccharides having 11 saccharide units, oligosaccharides having 10 saccharide units, oligosaccharides having 9 saccharide units, oligosaccharides having 8 saccharide units, oligosaccharides having 7 saccharide units, oligosaccharides having 6 saccharide units, oligosaccharides having 5 saccharide units, oligosaccharides having 4 saccharide units, oligosaccharides having 3 saccharide units, and oligosaccharides having 2 saccharide units is at least 70 % by weight, preferably at least 80 % by weight, more preferably at least 90 % by weight, still more preferably at least 95 % by weight and most preferably at least 98 % by weight based on 100 % by weight of the combined ratio of all saccharides in the oligosaccharide composition. This ratio can be, for example, determined using HPLC using defined galactose oligomers as standard as described above. For example, the HPLC may be performed using HPAEC-PAD. The balance to 100 % by weight may be formed by oligosaccharides having more than 20 saccharide units, polysaccharides and/or monosaccharides obtained from the pectin. In this regard, the term "all saccharides" encompasses any saccharide present in the reaction mixture including, for example, oligosaccharides, polysaccharides and/or monosaccharides obtained from the pectin.

In an embodiment the oligosaccharide composition comprises one or more of an oligosaccharide selected from the group consisting of an oligosaccharide having 15 saccharide units, an oligosaccharide having 14 saccharide units, an oligosaccharide having 13 saccharide units, an oligosaccharide having 12 saccharide units, an oligosaccharide having 11 saccharide units, an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units. In an embodiment of the oligosaccharide composition a combined ratio of oligosaccharides having 15 saccharide units, oligosaccharides having 14 saccharide units, oligosaccharides having 13 saccharide units, oligosaccharides having 12 saccharide units, oligosaccharides having 11 saccharide units, oligosaccharides having 10 saccharide units, oligosaccharides having 9 saccharide units, oligosaccharides having 8 saccharide units, oligosaccharides having 7 saccharide units, oligosaccharides having 6 saccharide units, oligosaccharides having 5 saccharide units, oligosaccharides having 4 saccharide units, oligosaccharides having 3 saccharide units, and oligosaccharides having 2 saccharide units is at least 70 % by weight, preferably at least 80 % by weight, more preferably at least 90 % by weight, still more preferably at least 95 % by weight and most preferably at least 98 % by weight based on 100 % by weight of the combined ratio of all saccharides in the oligosaccharide composition. This ratio can be, for example, determined using HPLC using defined galactose oligomers as standard as described above. For example, the HPLC may be performed using HPAEC-PAD. The balance to 100 % by weight may be formed by oligosaccharides having more than 15 saccharide units, polysaccharides and/or monosaccharides obtained from the pectin. In this regard, the term "all saccharides" encompasses any saccharide present in the reaction mixture including, for example, oligosaccharides, polysaccharides and/or monosaccharides obtained from the pectin.

In an embodiment the oligosaccharide composition comprises one or more of an oligosaccharide selected from the group consisting of an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units. In an embodiment of the oligosaccharide composition a combined ratio of oligosaccharides having 10 saccharide units, oligosaccharides having 9 saccharide units, oligosaccharides having 8 saccharide units, oligosaccharides having 7 saccharide units, oligosaccharides having 6 saccharide units, oligosaccharides having 5 saccharide units, oligosaccharides having 4 saccharide units, oligosaccharides having 3 saccharide units, and oligosaccharides having 2 saccharide units is at least 70 % by weight, preferably at least 80 % by weight, more preferably at least 90 % by weight, still more preferably 95 % by weight and most preferably at least 98 % by weight based on 100 % by weight of the combined ratio of all saccharides in the oligosaccharide composition. This ratio can be, for example, determined using HPLC using defined galactose oligomers as standard as described above. For example, the HPLC may be performed using HPAEC-PAD. The balance to 100 % by weight may be formed by oligosaccharides having more than 10 saccharide units, polysaccharides and/or monosaccharides obtained from the pectin.

In an embodiment the oligosaccharide composition comprises one or more of an oligosaccharide selected from the group consisting of an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units. In an embodiment of the oligosaccharide composition a combined ratio of oligosaccharides having 7 saccharide units, oligosaccharides having 6 saccharide units, oligosaccharides having 5 saccharide units, oligosaccharides having 4 saccharide units, oligosaccharides having 3 saccharide units, and oligosaccharides having 2 saccharide units is at least 70 % by weight, preferably at least 80 % by weight, more preferably at least 90 % by weight, still more preferably at least 95 % by weight and most preferably at least 98 % by weight based on 100 % by weight of the combined ratio of all saccharides in the oligosaccharide composition. This ratio can be, for example, determined using HPLC using defined galactose oligomers as standard as described above. For example, the HPLC may be performed using HPAEC-PAD. The balance to 100 % by weight may be formed by oligosaccharides having more than 7 saccharide units, polysaccharides and/or monosaccharides obtained from the pectin. In this regard, the term "all saccharides" encompasses any saccharide present in the reaction mixture including, for example, oligosaccharides, polysaccharides and/or monosaccharides obtained from the pectin.

In an embodiment the oligosaccharide composition comprises one or more of an oligosaccharide selected from the group consisting of an oligosaccharide having 7 saccharide units, an oligosaccharide having 6 saccharide units, an oligosaccharide having 5 saccharide units, an oligosaccharide having 4 saccharide units, and an oligosaccharide having 3 saccharide units. In an embodiment of the oligosaccharide composition a combined ratio of oligosaccharides having 7 saccharide units, oligosaccharides having 6 saccharide units, oligosaccharides having 5 saccharide units, oligosaccharides having 4 saccharide units, and oligosaccharides having 3 saccharide units is at least 70 % by weight, preferably at least 80 % by weight, more preferably at least 90 % by weight, still more preferably of at least 95 % by weight and most preferably at least 98 % by weight based on 100 % by weight of the combined ratio of all saccharides in the oligosaccharide composition. This ratio can be, for example, determined using HPLC using defined galactose oligomers as standard as described above. For example, the HPLC may be performed using HPAEC-PAD. The balance to 100 % by weight may be formed by oligosaccharides having more than 7 saccharide units, polysaccharides, disaccharides and/or monosaccharides obtained from the pectin. In this regard, the term "all saccharides" encompasses any saccharide present in the reaction mixture including, for example, oligosaccharides, polysaccharides, disaccharides and/or monosaccharides obtained from the pectin.

In an embodiment the oligosaccharide composition comprises one or more of an oligosaccharide selected from the group consisting of an oligosaccharide having 6 saccharide units, an oligosaccharide having 5 saccharide units, an oligosaccharide having 4 saccharide units, and an oligosaccharide having 3 saccharide units. In an embodiment of the oligosaccharide composition a combined ratio of oligosaccharides having 6 saccharide units, oligosaccharides having 5 saccharide units, oligosaccharides having 4 saccharide units, and oligosaccharides having 3 saccharide units is at least 70 % by weight, preferably at least 80 % by weight, more preferably at least 90 % by weight, still more preferably at least 95 % by weight and most preferably at least 98 % by weight based on 100 % by weight of the combined ratio of all saccharides in the oligosaccharide composition. This ratio can be, for example, determined using HPLC using defined galactose oligomers as standard as described above. For example, the HPLC may be performed using HPAEC-PAD. The balance to 100 % by weight may be formed by oligosaccharides having more than 6 saccharide units, polysaccharides, disaccharides and/or monosaccharides obtained from the pectin. In this regard, the term "all saccharides" encompasses any saccharide present in the reaction mixture including, for example, oligosaccharides, polysaccharides, disaccharides and/or monosaccharides obtained from the pectin.

In an embodiment the oligosaccharide composition comprises one or more of an oligosaccharide selected from the group consisting of an oligosaccharide having 5 saccharide units, and an oligosaccharide having 4 saccharide units. In an embodiment of the oligosaccharide composition a combined ratio of oligosaccharides having 5 saccharide units, and oligosaccharides having 4 saccharide units is at least 70 % by weight, preferably at least 80 % by weight, more preferably at least 90 % by weight, still more preferably at least 95 % by weight and most preferably at least 98 % by weight based on 100 % by weight of the combined ratio of all saccharides in the oligosaccharide composition. This ratio can be, for example, determined using HPLC using defined galactose oligomers as standard as described above. For example, the HPLC may be performed using HPAEC-PAD. The balance to 100 % by weight may be formed by oligosaccharides having more than 5 saccharide units, polysaccharides, disaccharides, trisaccharides and/or monosaccharides obtained from the pectin. In this regard, the term "all saccharides" encompasses any saccharide present in the reaction mixture including, for example, oligosaccharides, polysaccharides, disaccharides, trisaccharides and/or monosaccharides obtained from the pectin.

In an embodiment of the oligosaccharide composition the oligosaccharides comprise one or more of a saccharide unit selected from the group consisting of galactose, galacturonic acid, and galacturonic acid methyl ester. The alkyl ester may be a C1-C6 alkyl ester, a C1-C5 alkyl ester, a C1-C4 alkyl ester, a C1-C3 alkyl ester, a C1-C2 alkyl ester or a C1 alkyl ester. Non-limiting examples for suitable alkyl esters include a methyl ester, an ethyl ester, an *n*-propyl ester, an *iso*-propyl ester, an *n*-butyl ester, a *sec*-butyl ester, an *iso*-butyl ester, a *tert*-butyl ester, a pentyl ester and a hexyl ester. Preferably, the alkyl ester is a methyl ester. In this regard, in naturally occurring pectins a part of the carboxylic acid groups is esterified with methyl to form a methyl ester.

As indicated above, the methods for preparing an oligosaccharide composition may comprise that the oligosaccharide composition is dried. Therefore, in an embodiment the oligosaccharide composition is solid. Such solid form of the oligosaccharide composition provides easy handling and storage.

The oligosaccharide composition described herein comprises oligosaccharides each of which represents a receptor molecule for a lectin, in particular for Shiga toxin, a PapG adhesin or SadP. Hence, each oligosaccharide binds to a lectin such as, for example, a Shiga toxin, a PapG adhesin or SadP. Therefore, the oligosaccharide composition of the present invention can be used for inhibition or neutralization of lectins such as, for example a Shiga toxin, a PapG adhesin or SadP (see, for example, Figure 13).

The oligosaccharide composition may also serve as a starting material for the synthesis of coupling products in which the oligosaccharides required for binding to the lectin are coupled with a coupling partner to provide further inhibitors for lectins. Accordingly, the present invention also relates to a method of preparing a coupling product of an oligosaccharide composition, said method comprising the steps of:
(a) providing an oligosaccharide composition described herein, in particular an oligosaccharide composition obtained by any one of the methods of preparing an oligosaccharide composition described herein; and
(b) forming a coupling product of an oligosaccharide composition from the oligosaccharide composition and one or more of a coupling partner.

The term "providing an oligosaccharide composition" as used within the context of the invention described herein means to arrange an oligosaccharide composition described herein for a chemical reaction, such as, for example, for coupling with the coupling partner. For example, the term "providing an oligosaccharide composition" may include placing the oligosaccharide composition in the reaction vessel, combining the oligosaccharide composition with a suitable reaction medium and/or combining the oligosaccharide composition with the coupling partner. On the other hand, the oligosaccharide composition may be already present in a reaction vessel and/or in a reaction medium. This may be, for example, the case when the oligosaccharide composition is prepared before performing the coupling with the coupling partner using, for example, any one of the methods of preparing an oligosaccharide composition described herein. In this case, the term "providing an oligosaccharide composition" may include, for example, combining the oligosaccharide composition with additional reaction medium and/or the coupling partner. The oligosaccharide composition used as a starting material for the formation of a coupling product of an oligosaccharide composition may be any oligosaccharide composition described herein. As denoted by the phrase "in particular", the oligosaccharide composition which is employed for the formation of the coupling product of an oligosaccharide composition may be optionally an oligosaccharide composition obtained by any one of the methods of preparing an oligosaccharide composition described herein. In case that the oligosaccharide composition is obtained by a method of preparing an oligosaccharide composition described herein, any one of the methods of preparing an oligosaccharide composition described herein may be used. In this regard, any condition, such as, for example, reaction time and temperature, method for converting carboxylic groups to the corresponding alcohols, cleavage of glycosidic bonds such as, for example, hydrolysis under acidic or enzymatic conditions, separation of oligosaccharides having non-reduced carboxylic acid groups, purification, analytical method such as, for example, mass spectrometry or HPLC, as described herein in the context of a method for preparing an oligosaccharide composition may be used

Alternatively, the formation of a coupling product of an oligosaccharide composition may be carried out by first cleaving the glycosidic bonds of a pectin leading to an oligosaccharide composition, then forming a coupling product of an oligosaccharide composition using a coupling partner and finally converting carboxylic acid groups to the corresponding alcohols. Accordingly, the present invention further relates to a method of preparing a coupling product of an oligosaccharide composition, said method comprising the steps of:
(a1) providing a pectin;
(a2) cleavage of glycosidic bonds of the pectin under conditions suitable to give an oligosaccharide composition;
(b) forming a coupling product of an oligosaccharide composition from the oligosaccharide composition and one or more of a coupling partner; and
(c) conversion of carboxylic acid groups of the coupling product of step (b) to the corresponding alcohols.

Regarding step (a2), any one of the methods and/or conditions for cleaving glycosidic bonds described herein in the context of any one of the methods for preparing an oligosaccharide composition may be used. For example, the cleavage of glycosidic bonds may be performed using hydrolysis under acidic or enzymatic conditions. Regarding step (c), any one of the methods and/or conditions for the conversion of carboxylic acid groups to the corresponding alcohols described herein in the context of any one of the methods for preparing an oligosaccharide composition may be used. For example, the conversion of carboxylic acid groups to the corresponding alcohols may comprise activating the carboxylic acid groups with a carbodiimide and reducing the obtained conjugate of the carboxylic acid groups and the carbodiimide using a borohydride. Further, any condition, such as, for example, reaction time and temperature, separation of oligosaccharides having non-reduced carboxylic acid groups, purification, analytical method such as, for example, mass spectrometry or HPLC, as described herein in the context of a method for preparing an oligosaccharide composition may be used.

As used herein, the term "coupling partner" denotes a reactant, such as, for example, a compound, which is reacted with the oligosaccharide composition described herein so that the coupling partner is coupled with the oligosaccharides of the oligosaccharide composition. Accordingly, the term "coupling product of an oligosaccharide composition" as used herein denotes a composition which is obtained by reaction of the coupling partner with an oligosaccharide composition as described herein so that the coupling partner is coupled with the oligosaccharides of the oligosaccharide composition. As set out above, the oligosaccharide composition described herein may comprise oligosaccharides having a different number of saccharide units. Accordingly, the coupling product of an oligosaccharide composition may comprise oligosaccharides having a different number of saccharide units coupled to the coupling partner. The coupling partner may be, for example, selected from the group consisting of a lipid anchor, preferably a phosphatidylethanolamine, a Yariv reagent, a silica gel (e.g. SYNSORB Pk, Armstrong GD, Fodor E, Vanmaele R. Investigation of Shiga-like toxin binding to chemically synthesized oligosaccharide sequences. 1991 J. Infect Dis 164, 1160-1167, Takeda T, Yoshino K, Adachi E, Sato Y, Yamagata. In vitro assessment of a chemically synthesized Shiga toxin receptor analog attached to Chromosorb P (synsorb Pk) as a specific absorbing agent of Shiga toxin 1 and 2 1999 Microbiol Immunol 43, 331-337, MacConnachie AA, Todd WTA. Potential therapeutic agents for the prevention and treatment of haemolytic uaemic syndrome in Shiga toxin producing Escherichia coli infection. 2004 Curr Opin Infect Dis 17, 479-482, Clark GC, Basak AK, Titball RW. The rational design of bacterial toxin inhibitors. 2007 Curr Comput Aided Drug Des 3, 1-12, Bundle DR, Kitov P, Read RJ, Ling H, Amrstrong G. Treatment of bacterial infection. 2001 United States Patent, Patent No.: US 6,310,043 B1), a glucose functionalized with a spacer (e.g. STARFISH, Clark GC, Basak AK, Titball RW. The rational design of bacterial toxin inhibitors. 2007 Curr COmput Aided Drug Des 3, 1-12, Kitov PI, Sadowska JM, Mulvey ,G, Armstrong GD, Ling H, Pannu NS, Read RJ, Bundle DR. Shiga-like toxins are neutralized by tailored multivalent carbohydrate lignds. 2000 Nature 403, 669-672, Williams SJ, Davies GJ. Protein-carbohydrate internactions: learning lessons from nature. 2001 Trends Biotchnol 19, 356-362), an acrylamide polymer (e.g. Nishikawa K. Recent progree of Shiga toxin neutralizer for treatment of infections by Shiga toxin-producing Escherichia coli. 2011 Arch Immunol Ther Exp 59, 239-247, Watanabe M. Matsuoka K, Kita E, Igai K, Higashi N, Miyagawa A, Watanabe T, Yanoshita R, Samejima Y, Terunuma D, Natori Y, Nishikawa K. Oral therapeutic agents with highly clustered globotriose for treatment of Shiga toxigenic Escherichia coli infections. 2004 J Infect Dis 189, 360-368, Watanabe M, Igai K, Matsuoka K, Miyagawa A, Watanabe T, Yanoshita R, Samejima Y, Terunuma D, Natori Y, Nishikawa K. Structural analysis of the interaction between Shiga toxin B subunits and linear polymers bearing clustered globotriose residues. 2006 Infect Immun 74, 1984-1988, Watanabe-Takahashi M, Sato T, Dohi T, Noguchi N, Kano F, Murata M, Hamabata T, Natori Y, Nishikawa K. An orally applicable Shiga toxin neutralizer functions in the intestine to inhibit the intracellular transport of the toxin. 2010 Infect Immun 78, 177-183), an acrylamide copolymer, a carbosilane (e.g. SUPER TWIG, Matsuoka K. Terabatake M, Esumi Y, Terunuma D, Kuzuhara H. Synthetic assembly of trisaccharide moieties of globotriaosyl ceramide using carbosilane dendrimers as cores. A new type of functional glyco-material. 1999 Tetrahedron Lett 40, 7839-7842, Nishikawa K, Matsuoka K, Kita E, Okabe N, Mizuguchi M, Hino K, Miyazawa S, Yamasaki C, Aoki J, Takashima S, Yamakawa Y, Nishijima M, Terunuma D, Kuzuhara H, Natori Y. A therapeutic agent with oriented carbohydrates for treatment of infections by Shiga toxin-producing Escherichia coli O157:H7. 2002 Proc. Ntl Acad Sci USA 99, 7669-7674, Nishikawa K. Matsuoka K. Watanabe M. Igai K, Hino K, Hatano K, Yamada A, Abe N, Terunuma D, Kuzuhara H, Natori Y. Identification of the optimal structure required for a Shiga toxin neutralizer with oriented carbohydrates to function in the circulation. 2005 J Infect Dis 191, 2097-2105, Nishikawa K. Recent progress of Shiga toxin neutralizer for treatment of infections by Shiga toxin-producing Escherichia coli. 2011 Arch Immunol Ther Exp 59, 239-247), a chitosan (e.g. Li XB, Wu PX, Cheng SH, Lv X. Synthesis and assessment of globotriose-chitosan conjugate, a novel inhibitor of Shiga toxins produced by Escherichia coli. 2012 J. Med. Chem 55, 2702-2710), a nanoparticle (e.g. Mochalin VN, Shenderova O, Ho D, Gogosi Y. The properties and applications of nanodiamonds. 2012 Nat Nanotechnol 7, 11-23, Reichardt N.C., Martin-Lomas M, Penades S. Glyconanotechnology. 2013 Chem Soc Rev 42, 4358-4376, Chien YY, Jan MD, Adak AK, Tzeng HC, Lin YP, Chen YJ, Wang KT, Chen CT, Chen CC, Lin CC. Globotriose-functionalized diamond nanoparticles as multivalent probes for Shiga-like toxin. 2008 ChemBioChem 9, 1100-1109) and any combination thereof.

In a preferred embodiment the coupling partner is a lipid anchor. Such coupling of the oligosaccharide composition described herein with a lipid anchor leads to formation of a neoglycolipid composition. The term "neoglycolipid composition" refers to a composition comprising one or more oligosaccharides coupled to a lipid anchor. In this regard, a "neoglycolipid" as understood herein is a coupling product of an oligosaccharide with a lipid anchor. Exemplary lipid anchors which are suitable for the present invention are disclosed in US patent no. 5,122,450 of 16 June 1992, the whole disclosure of this document incorporated herein by reference. In this regard, in US 5,122,450 the lipid anchor is referred to as "spacer compound". As non-limiting examples, the lipid anchors used for the preparation of a neoglycolipid composition disclosed herein may include phosphatidyl ethanolamine dipalmitoyl and its analogs, L-alpha-phosphatidyl-L-serine, L-alpha-phosphatidyl-ethanolamine dilauroyl, L-alpha-phosphatidyl-ethanolamine dimyristoyl, L-alpha-phosphatidyl-ethanolamine distearoyl and/or L-alpha-phosphatidyl-ethanolamine-beta-oleoyl-gamma-palmitoyl.

In a particularly preferred embodiment the coupling partner is a phosphatidylethanolamine. Preferably, the phosphatidylethanolamine is a phosphatidylethanolamine having a structure of: 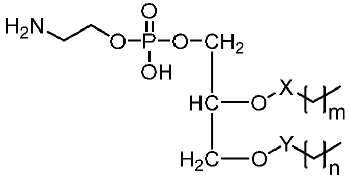 or any suitable salt thereof, wherein m and n is each, independently, an integer of from 8 to 20, wherein X and Y is each, independently, CH₂ or C=O. Accordingly, a preferred embodiment of the present invention relates to a method of preparing a neoglycolipid composition, said method comprising the steps of:
(a) providing an oligosaccharide composition as described herein, in particular an oligosaccharide composition obtained by any one of the methods of preparing an oligosaccharide composition described herein; and
(b) forming a neoglycolipid composition from the oligosaccharide composition and one or more of a phosphatidylethanolamine having a structure of: 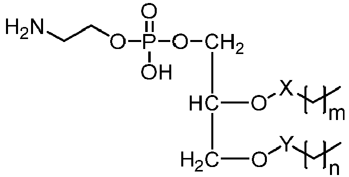
or any suitable salt thereof, wherein m and n is each, independently, an integer of from 8 to 20, wherein X and Y is each, independently, CH₂ or C=O; and wherein a carbon-nitrogen bond is formed between an oligosaccharide and a phosphatidylethanolamine.

The term "providing an oligosaccharide composition" means to arrange an oligosaccharide composition described herein for a chemical reaction, such as, for example, for coupling with the phosphatidylethanolamine. For example, the term "providing an oligosaccharide composition" may include placing the oligosaccharide composition in the reaction vessel, combining the oligosaccharide composition with a suitable reaction medium and/or combining the oligosaccharide composition with the phosphatidylethanolamine. On the other hand, the oligosaccharide composition may be already present in a reaction vessel and/or in a reaction medium. This may be, for example, the case when the oligosaccharide composition is prepared before performing the coupling with the phosphatidylethanolamine using, for example, any one of the methods of preparing an oligosaccharide composition described herein. In this case, the term "providing an oligosaccharide composition" may include, for example, combining the oligosaccharide composition with additional reaction medium and/or the phosphatidylethanolamine. The oligosaccharide composition used as a starting material for the formation of a coupling product of an oligosaccharide composition may be any oligosaccharide composition described herein. As denoted by the phrase "in particular", the oligosaccharide composition which is employed for the formation of the coupling product of an oligosaccharide composition may be optionally an oligosaccharide composition obtained by any one of the methods of preparing an oligosaccharide composition described herein. In case that the oligosaccharide composition is obtained by a method of preparing an oligosaccharide composition described herein, any one of the methods of preparing an oligosaccharide composition described herein may be used for providing the oligosaccharide composition of step (a). In this regard, any condition, such as, for example, reaction time and temperature, method for converting carboxylic groups to the corresponding alcohols, cleavage of glycosidic bonds such as, for example, hydrolysis under acidic or enzymatic conditions, separation of oligosaccharides having non-reduced carboxylic acid groups, purification, analytic method such as, for example, mass spectrometry or HPLC, as described herein in the context of a method for preparing an oligosaccharide composition may be used.

Alternatively, the formation of a neoglycolipid composition may be carried out by first cleaving the glycosidic bonds of a pectin leading to an oligosaccharide composition, then forming a neoglycolipid composition using a phosphatidylethanolamine and finally converting carboxylic acid groups to the corresponding alcohols. Accordingly, another preferred embodiment of the present invention relates to a method of preparing a neoglycolipid composition or pharmaceutically acceptable salt or tautomer thereof, said method comprising the steps of:
(a1) providing a pectin;
(a2) cleavage of glycosidic bonds of the pectin under conditions suitable to give an oligosaccharide composition;
(b) forming a neoglycolipid composition from the oligosaccharide composition and one or more of a phosphatidylethanolamine having a structure of: 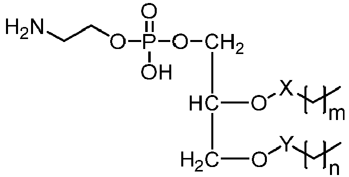 or any suitable salt thereof, wherein m and n is each, independently, an integer of from 8 to 20, wherein X and Y is each, independently, CH₂ or C=O; and wherein a carbon-nitrogen bond is formed between an oligosaccharide and a phosphatidylethanolamine; and
(c) conversion of carboxylic acid groups of the neoglycolipid composition of step (b) to the corresponding alcohols.

Regarding step (a2), any one of the methods and/or conditions for cleaving glycosidic bonds described herein in the context of any one of the methods for preparing an oligosaccharide composition may be used. For example, the cleavage of glycosidic bonds may be performed using hydrolysis under acidic or enzymatic conditions. Regarding step (c), any one of the methods and/or conditions for the conversion of carboxylic acid groups to the corresponding alcohols described herein in the context of any one of the methods for preparing an oligosaccharide composition may be used. For example, the conversion of carboxylic acid groups to the corresponding alcohols may comprise activating the carboxylic acid groups with a carbodiimide and reducing the obtained conjugate of the carboxylic acid groups and the carbodiimide using a borohydride. Further, any condition, such as, for example, reaction time and temperature, separation of oligosaccharides having non-reduced carboxylic acid groups, purification, analytical method such as, for example, mass spectrometry or HPLC, as described herein in the context of a method for preparing an oligosaccharide composition may be used.

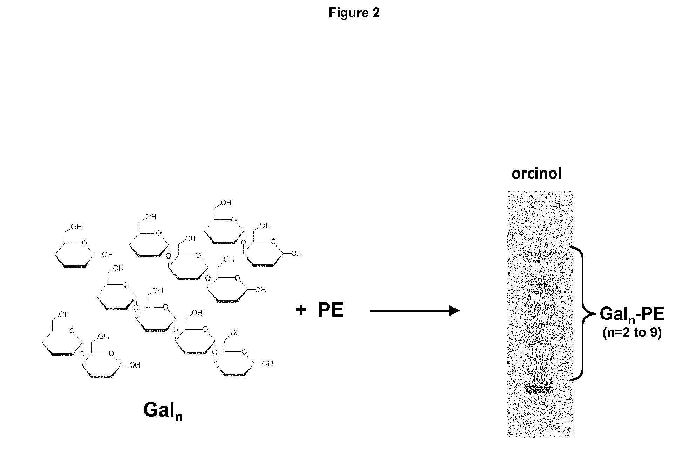

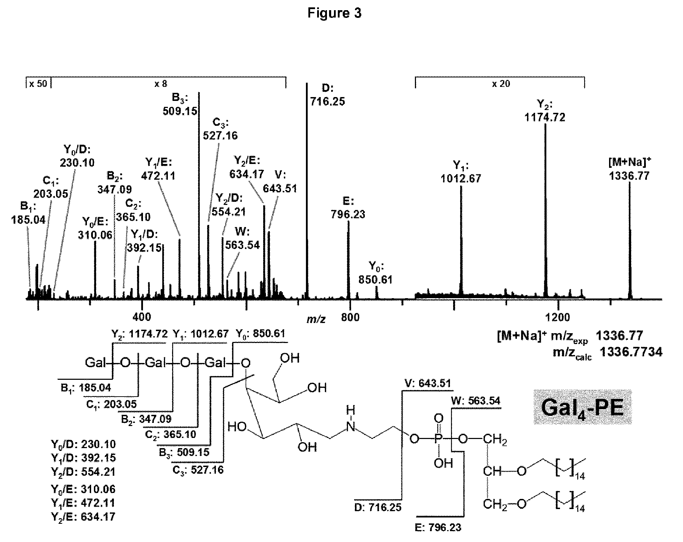

In below Example 3 a neoglycolipid composition as disclosed herein is prepared from an oligosaccharide composition in accordance with the present invention. Figure 2 schematically illustrates reaction of an oligosaccharide composition in accordance with the present invention with a phosphatidylethanolamine (denoted as "PE") to form neoglycolipids. Figure 3 shows a mass spectrum and fragmentation pattern of a neoglycolipid having an oligosaccharide of four galactose units coupled to a phosphatidylethanolamine.

As set out above, the coupling product of an oligosaccharide composition described herein may comprise oligosaccharides having a different number of saccharide units coupled to the coupling partner. Accordingly, any neoglycolipid composition described herein may comprise oligosaccharides having a different number of saccharide units coupled to the phosphatidylethanolamine.

The conditions for forming a neoglycolipid composition of step (b) are not particularly limited. Any method and/or condition suitable for coupling an oligosaccharide with a phosphatidylethanolamine known to a person skilled in the art may be used for forming the neoglycolipid composition from the oligosaccharide composition in step (b) of any one of the methods of forming a neoglycolipid composition described herein. As a non-limiting example, in any one of the methods for preparing a neoglycolipid composition described herein step (b) may be performed by (b1) reacting the oligosaccharide composition with the phosphatidylethanolamine and (b2) treating the product obtained in step (b1) with a reducing agent. Without wishing to be bound by theory, when reacting the oligosaccharide composition with the phosphatidylethanolamine, an imine or iminium ion may be formed by reaction of the amine group of the phosphatidylethanolamine with an aldehyde moiety of the open-chain form of a saccharide unit of an oligosaccharide thus forming a carbon-nitrogen bond between the oligosaccharide and the phosphatidylethanolamine. This imine or iminium ion is then reduced to give an amine moiety. Any suitable reducing agent may be used. For example, the reducing agent may be a borohydride. In particular, the reducing agent may be selected from the group consisting of lithium borohydride (LiBH₄), sodium borohydride (NaBH₄), potassium borohydride (KBH₄), sodium triacetoxyborohydride (NaB(OAc)₃H), sodium cyanoborohydride (NaBH₃CN) and any combination thereof. Preferably the reducing agent is sodium cyanoborohydride, which provides good results in the reduction of an imine or an iminium ion. An exemplary non-limiting method for forming a neoglycolipid composition from an oligosaccharide composition and a phosphatidylethanolamine is found in "Pohlentz G, Egge H. Neoglycolipids of 1-Deoxy-1-phosphatidylethanolaminolactitol Type: Synthesis, Structure Analysis, and Use as Probes for Characterization of Glycosyltransferases. 1994 Methods Enzymol 242, 127-145", the complete disclosure of which is hereby incorporated by reference. During the formation of the neoglycolipid composition in step (b) side products of an Amadori rearrangement, so-called 1-amino-desoxyketoses, may be formed.

In any one of the methods of preparing a neoglycolipid composition described herein the phosphatidylethanolamine used for the formation of the neoglycolipid composition may have the structure:

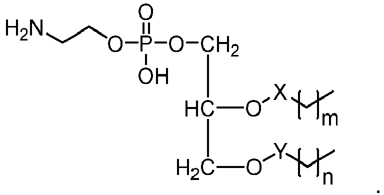

In any one of the methods for preparing a neoglycolipid composition described herein m and n is each, independently, an integer of from 8 to 20, wherein X and Y is each, independently, CH₂ or C=O. In an embodiment m and n is each, independently, an integer of from 10 to 18, preferably of from 12 to 16, and most preferably m and n are each 14. In an embodiment m and n are the same. In an embodiment X and Y are the same. In an embodiment X and Y are CH₂, m and n are the same and an integer of from 8 to 20, preferably of from 10 to 18, more preferably of from 12 to 16 and most preferably 14. In an embodiment X and Y are C=O, m and n are the same and an integer of from 8 to 20, preferably of from 10 to 18, more preferably of from 12 to 16 and most preferably 14. In a preferred embodiment of the method of preparing a neoglycolipid composition the phosphatidylethanolamine is dihexadecylglycerophosphoethanolamine. A suitable salt of the phosphatidylethanolamine may be used in any one of the methods of preparing a neoglycolipid composition as described herein. Suitable salts may include any pharmaceutically acceptable salt, such as, for example, a hydrochloride or a hydrobromide.

In embodiments the neoglycolipid composition obtained by any one of the methods for preparing a neoglycolipid composition disclosed herein may be purified. Suitable purification methods include but are not limited to chromatography such as, for example, chromatography using silica gel as stationary phase.

In order to bring the neoglycolipid composition into a transportable and storable form, the neoglycolipid composition may be dried. By such drying the neoglycolipid composition is in general obtained in a solid form. The drying method is not particularly limited and any suitable drying method known to a person skilled in the art may be employed. For example, the drying may be carried out using freeze-drying, spray drying, heat drying, vacuum drying, fluidized bed drying and/or spray granulation. Preferably, the drying may be performed using freeze-drying, which is also known as lyophilization. In this regard, lyophilization provides a gentle method for drying the neoglycolipid composition of the present invention.

The present invention further relates to a coupling product of an oligosaccharide composition obtainable or being obtained by the method of any one of the methods for preparing a coupling product of an oligosaccharide composition described herein. The coupling partner to which the oligosaccharides are coupled in the coupling product of an oligosaccharide composition may be, for example, selected from the group consisting of a lipid anchor, preferably a phosphatidylethanolamine, a Yariv reagent, a silica gel, a glucose functionalized with a spacer, an acrylamide polymer, an acrylamide copolymer, a carbosilane, a chitosan, a nanoparticle and any combination thereof as described herein above in the context of any one of the methods of preparing a coupling product of an oligosaccharide composition.

In a preferred embodiment of the present invention the coupling product of an oligosaccharide composition is a neoglycolipid composition. A preferred embodiment of the present invention relates to a neoglycolipid composition obtainable or being obtained by any one of the methods for preparing a neoglycolipid composition described herein. In particular, the neoglycolipid composition may be prepared by coupling the oligosaccharide composition with a lipid anchor as described herein. Preferably, the lipid anchor is a phosphatidylethanolamine. More preferably, the phosphatidylethanolamine is a phosphatidylethanolamine as described herein in the context of any one of the methods for preparing a neoglycolipid composition.

Accordingly, a preferred embodiment of the present invention is directed to a neoglycolipid composition obtainable or being obtained by a method for preparing a neoglycolipid composition, said method comprising the steps of:
(a) providing an oligosaccharide composition as described herein, in particular using any one of the methods for preparing an oligosaccharide composition described herein; and
(b) forming a neoglycolipid composition from the oligosaccharide composition and one or more of a phosphatidylethanolamine having a structure of: 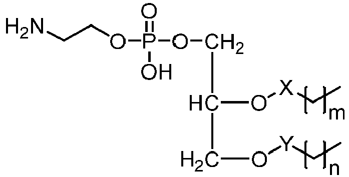 or any suitable salt thereof, wherein m and n is each, independently, an integer of from 8 to 20, wherein X and Y is each, independently, CH₂ or C=O; and wherein a carbon-nitrogen bond is formed between an oligosaccharide and a phosphatidylethanolamine.

Another preferred embodiment of the present invention relates to a neoglycolipid composition or pharmaceutically acceptable salt or tautomer obtainable or being obtained by a method for preparing a neoglycolipid composition, said method comprising the steps of:
(a1) providing a pectin;
(a2) cleavage of glycosidic bonds of the pectin under conditions suitable to give an oligosaccharide composition;
(b) forming a neoglycolipid composition from the oligosaccharide composition and one or more of a phosphatidylethanolamine having a structure of: 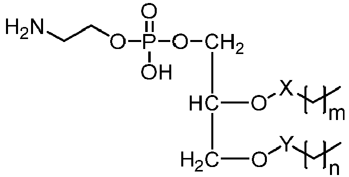 or any suitable salt thereof, wherein m and n is each, independently, an integer of from 8 to 20, wherein X and Y is each, independently, CH₂ or C=O; and wherein a carbon-nitrogen bond is formed between an oligosaccharide and a phosphatidylethanolamine; and
(c) conversion of carboxylic acid groups of the neoglycolipid composition of step (b) to the corresponding alcohols.

Any neoglycolipid composition described herein may be obtainable or being obtained by any one of the embodiments of the methods for preparing a neoglycolipid composition described herein.

The neoglycolipid composition obtainable or being obtained by any one of the methods described herein may comprise one or more of a neoglycolipid having the structure: 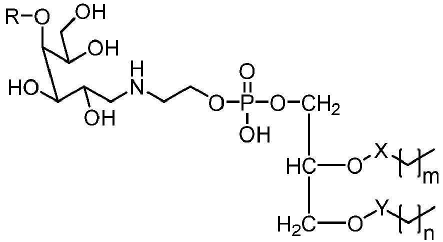 or any pharmaceutically acceptable salt thereof, wherein m and n is each, independently, an integer of from 8 to 20, wherein X and Y is each, independently, CH₂ or C=O; and wherein R is a residue comprising of from 1 to 19 saccharide units. In embodiments m, n, X and Y may be as described herein within the context of any one of the methods for preparing a neoglycolipid composition. Accordingly, in an embodiment m and n is each, independently, an integer of from 10 to 18, preferably of from 12 to 16, and most preferably m and n are each 14. In an embodiment m and n are the same. In an embodiment X and Y are the same. In an embodiment X and Y are CH₂, m and n are the same and an integer of from 8 to 20, preferably of from 10 to 18, more preferably of from 12 to 16 and most preferably 14. In an embodiment X and Y are C=O, m and n are the same and an integer of from 8 to 20, preferably of from 10 to 18, more preferably of from 12 to 16 and most preferably 14. In an embodiment R is a residue comprising 1 saccharide unit. In an embodiment R is a residue comprising 2 saccharide units. In an embodiment R is a residue comprising 3 saccharide units. In an embodiment R is a residue comprising 4 saccharide units. In an embodiment R is a residue comprising 5 saccharide units. In an embodiment R is a residue comprising 6 saccharide units. In an embodiment R is a residue comprising 7 saccharide units. In an embodiment R is a residue comprising 8 saccharide units. In an embodiment R is a residue comprising 9 saccharide units. In an embodiment R is a residue comprising 10 saccharide units. In an embodiment R is a residue comprising 11 saccharide units. In an embodiment R is a residue comprising 12 saccharide units. In an embodiment R is a residue comprising 13 saccharide units. In an embodiment R is a residue comprising 14 saccharide units. In an embodiment R is a residue comprising 15 saccharide units. In an embodiment R is a residue comprising 16 saccharide units. In an embodiment R is a residue comprising 17 saccharide units. In an embodiment R is a residue comprising 18 saccharide units. In an embodiment R is a residue comprising 19 saccharide units. R may be a residue consisting of from 1 to 19 saccharide units connected via glycosidic bonds. In an embodiment R is a residue consisting of 1 saccharide unit connected via a glycosidic bond. In an embodiment R is a residue consisting of 2 saccharide units connected via glycosidic bonds. In an embodiment R is a residue consisting of 3 saccharide units connected via glycosidic bonds. In an embodiment R is a residue consisting of 4 saccharide units connected via glycosidic bonds. In an embodiment R is a residue consisting of 5 saccharide units connected via glycosidic bonds. In an embodiment R is a residue consisting of 6 saccharide units connected via glycosidic bonds. In an embodiment R is a residue consisting of 7 saccharide units connected via glycosidic bonds. In an embodiment R is a residue consisting of 8 saccharide units connected via glycosidic bonds. In an embodiment R is a residue consisting of 9 saccharide units connected via glycosidic bonds. In an embodiment R is a residue consisting of 10 saccharide units connected via glycosidic bonds. In an embodiment R is a residue consisting of 11 saccharide units connected via glycosidic bonds. In an embodiment R is a residue consisting of 12 saccharide units connected via glycosidic bonds. In an embodiment R is a residue consisting of 13 saccharide units connected via glycosidic bonds. In an embodiment R is a residue consisting of 14 saccharide units connected via glycosidic bonds. In an embodiment R is a residue consisting of 15 saccharide units connected via glycosidic bonds. In an embodiment R is a residue consisting of 16 saccharide units connected via glycosidic bonds. In an embodiment R is a residue consisting of 17 saccharide units connected via glycosidic bonds. In an embodiment R is a residue consisting of 18 saccharide units connected via glycosidic bonds. In an embodiment R is a residue consisting of 19 saccharide units connected via glycosidic bonds. In particular, the residue R may comprise one or more of a saccharide unit selected from the group consisting of galactose, galacturonic acid, and galacturonic acid alkyl ester. The alkyl ester may be a C1-C6 alkyl ester, a C1-C5 alkyl ester, a C1-C4 alkyl ester, a C1-C3 alkyl ester, a C1-C2 alkyl ester or a C1 alkyl ester. Non-limiting examples for suitable alkyl esters include a methyl ester, an ethyl ester, an *n*-propyl ester, an *iso*-propyl ester, an *n*-butyl ester, a *sec*-butyl ester, an *iso*-butyl ester, a *tert*-butyl ester, a pentyl ester and a hexyl ester. Preferably, the alkyl ester is a methyl ester. The pharmaceutically acceptable salt is not particularly limited and may include, for example, a hydrochloride or a hydrobromide.

As indicated above, during step (b) of the methods for preparing a neoglycolipid composition a side product of an Amadori rearrangement may be formed. However, it has been found that such rearrangement in general does not influence the binding to a lectin, such as, for example, a Shiga toxin. Accordingly, such side product may be present in the neoglycolipid composition and may have the structure 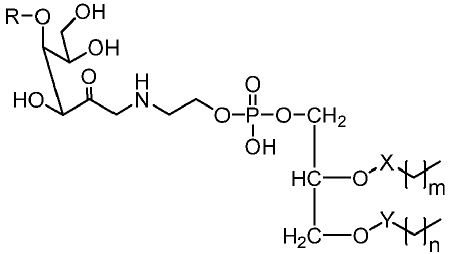 or any pharmaceutically acceptable salt or tautomer thereof, wherein m and n is each, independently, an integer of from 8 to 20, wherein X and Y is each, independently, CH₂ or C=O; and wherein R is a residue comprising of from 1 to 6 saccharide units. In embodiments m, n, X and Y may be as described herein within the context of any one of the methods for preparing a neoglycolipid composition. Accordingly, in an embodiment m and n is each, independently, an integer of from 10 to 18, preferably of from 12 to 16, and most preferably m and n are each 14. In an embodiment m and n are the same. In an embodiment X and Y are the same. In an embodiment X and Y are CH₂, m and n are the same and an integer of from 8 to 20, preferably of from 10 to 18, more preferably of from 12 to 16 and most preferably 14. In an embodiment X and Y are C=O, m and n are the same and an integer of from 8 to 20, preferably of from 10 to 18, more preferably of from 12 to 16 and most preferably 14. In an embodiment R is a residue comprising 1 saccharide unit. In an embodiment R is a residue comprising 2 saccharide units. In an embodiment R is a residue comprising 3 saccharide units. In an embodiment R is a residue comprising 4 saccharide units. In an embodiment R is a residue comprising 5 saccharide units. In an embodiment R is a residue comprising 6 saccharide units. In an embodiment R is a residue comprising 7 saccharide units. In an embodiment R is a residue comprising 8 saccharide units. In an embodiment R is a residue comprising 9 saccharide units. In an embodiment R is a residue comprising 10 saccharide units. In an embodiment R is a residue comprising 11 saccharide units. In an embodiment R is a residue comprising 12 saccharide units. In an embodiment R is a residue comprising 13 saccharide units. In an embodiment R is a residue comprising 14 saccharide units. In an embodiment R is a residue comprising 15 saccharide units. In an embodiment R is a residue comprising 16 saccharide units. In an embodiment R is a residue comprising 17 saccharide units. In an embodiment R is a residue comprising 18 saccharide units. In an embodiment R is a residue comprising 19 saccharide units. R may be a residue consisting of from 1 to 19 saccharide units connected via glycosidic bonds. In an embodiment R is a residue consisting of 1 saccharide unit connected via a glycosidic bond. In an embodiment R is a residue consisting of 2 saccharide units connected via glycosidic bonds. In an embodiment R is a residue consisting of 3 saccharide units connected via glycosidic bonds. In an embodiment R is a residue consisting of 4 saccharide units connected via glycosidic bonds. In an embodiment R is a residue consisting of 5 saccharide units connected via glycosidic bonds. In an embodiment R is a residue consisting of 6 saccharide units connected via glycosidic bonds. In an embodiment R is a residue consisting of 7 saccharide units connected via glycosidic bonds. In an embodiment R is a residue consisting of 8 saccharide units connected via glycosidic bonds. In an embodiment R is a residue consisting of 9 saccharide units connected via glycosidic bonds. In an embodiment R is a residue consisting of 10 saccharide units connected via glycosidic bonds. In an embodiment R is a residue consisting of 11 saccharide units connected via glycosidic bonds. In an embodiment R is a residue consisting of 12 saccharide units connected via glycosidic bonds. In an embodiment R is a residue consisting of 13 saccharide units connected via glycosidic bonds. In an embodiment R is a residue consisting of 14 saccharide units connected via glycosidic bonds. In an embodiment R is a residue consisting of 15 saccharide units connected via glycosidic bonds. In an embodiment R is a residue consisting of 16 saccharide units connected via glycosidic bonds. In an embodiment R is a residue consisting of 17 saccharide units connected via glycosidic bonds. In an embodiment R is a residue consisting of 18 saccharide units connected via glycosidic bonds. In an embodiment R is a residue consisting of 19 saccharide units connected via glycosidic bonds. In particular, the residue R may comprise one or more of a saccharide unit selected from the group consisting of galactose, galacturonic acid, and galacturonic acid alkyl ester. The alkyl ester may be a C1-C6 alkyl ester, a C1-C5 alkyl ester, a C1-C4 alkyl ester, a C1-C3 alkyl ester, a C1-C2 alkyl ester or a C1 alkyl ester. Non-limiting examples for suitable alkyl esters include a methyl ester, an ethyl ester, an *n*-propyl ester, an *iso*-propyl ester, an *n*-butyl ester, a *sec*-butyl ester, an *iso*-butyl ester, a *tert*-butyl ester, a pentyl ester and a hexyl ester. Preferably, the alkyl ester is a methyl ester. The pharmaceutically acceptable salt is not particularly limited and may include, for example, a hydrochloride or a hydrobromide.

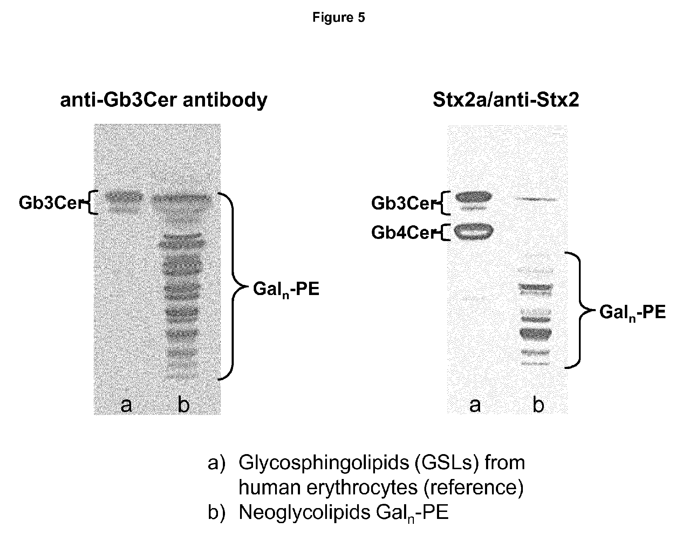

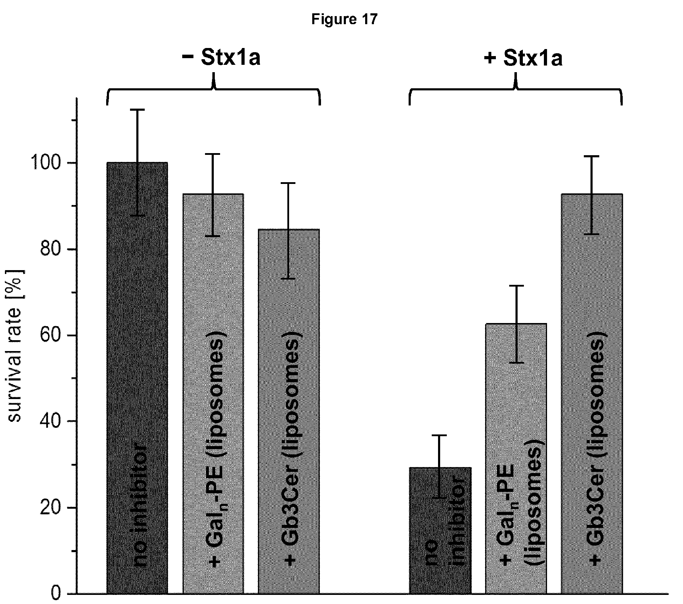

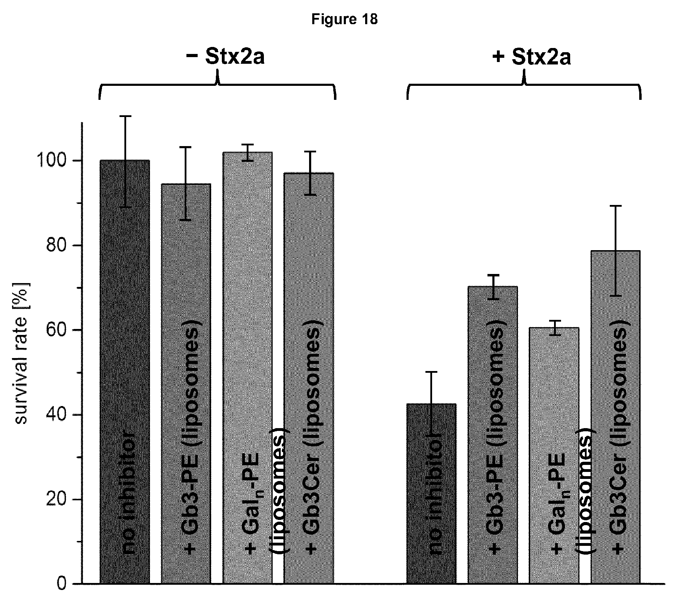

The neoglycolipids of the neoglycolipid composition described herein are suitable for binding to lectins. In this regard, Example 4 and Figures 5 to 12 show the binding of neoglycolipids to various subtypes of Shiga toxin. Furthermore, Example 7 in combination with Figures 17 and 18 shows that the survival rate of cells in presence of a Shiga toxin is significantly increased when the cells are treated with a neoglycolipid composition as described herein compared to the absence of such neoglycolipid composition.

The present invention also relates to a pharmaceutical composition comprising an oligosaccharide composition as described herein and a pharmaceutically acceptable carrier.

The term "pharmaceutical" as used herein refers to a chemical substance intended for use in the cure, treatment, or prevention of a disease. Details on techniques for formulation and administration of such compositions may be found in Remington, The Science and Practice of Pharmacy 21st Edition (Mack Publishing Co., Easton, PA) and Nielloud and Marti-Mestres, Pharmaceutical Emulsions and Suspensions: 2nd Edition (Marcel Dekker, Inc, New York).

The pharmaceutical compositions of the present invention comprise a pharmaceutical carrier. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well-known conventional methods. These pharmaceutical compositions can be administered to the subject at a suitable dose. The dosage regimen will be determined by the attending physician and clinical factors.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e. arresting its development; or (c) relieving the disease, i.e. causing regression of the disease. In the context of the present invention the term "subject" means an individual in need of a treatment of an affective disorder. Preferably, the subject is a mammalian, particularly preferred a human, a pig, a horse, a camel, a dog, a cat, a cow, a goat, a fowl, or any domestic or farm animal. A human is most preferred.

The term "administered" means administration of a therapeutically effective dose of the inhibitors and/or test-compounds as disclosed herein. By "therapeutically effective amount" is meant a dose that produces the effects for which it is administered. The exact dose will depend on the purpose of the treatment, and will be ascertainable by one skilled in the art using known techniques. Oral administration is particularly preferred. Any preparation suitable for oral administration known to a skilled person may be used. As non-limiting examples preparations for oral administration may include tablets, powders and/or granules.

In embodiments of the pharmaceutical composition the oligosaccharide composition is formulated in lipid vesicles. The lipid vesicles may be selected from the group consisting of liposomes, liosomes, micelles and transfersomes. Preferably, the lipid vesicles are liposomes. By "liposome" is meant a spherical lipid vesicle bounded by an ordered lipid bilayer and enclosing an aqueous phase. The lipid bilayer of liposomes is usually made of natural or synthetic phospholipids, but can also be made of non-phospholipids. The lipid bilayer of liposomes is an ordered bilayer, meaning that the molecular "head" and "tail" structures of the lipids are lined up next to one another. Liposomes utilized in the present invention can be multilamellar or more preferably are unilamellar (having one lipid bilayer). Liposomes that are "multilamellar" have multiple layers or membranes. This type of liposome has layers of lipid bilayers with an aqueous fluid spaced in between the lipid bilayers. Multilamellar liposomes have at least two layers of lipids. Those of ordinary skill in the art will recognize that various formulations of liposomes may be used. As non-limiting examples the liposomes may be prepared using phosphatidyl compounds, such as, for example, phosphatidylglycerol, phosphatidylcholine, phosphatidylserine, phosphatidylethanolamine, sphingolipids, cerebrosides, gangliosides and diacylphosphatidylglycerols and cholesterol. Preferably, in the pharmaceutical compositions disclosed herein a phosphatidylcholine is used for the formation of the liposomes. For example, a dioleoyl-phosphatidylcholine and cholesterol may be employed for the preparation of the liposomes.

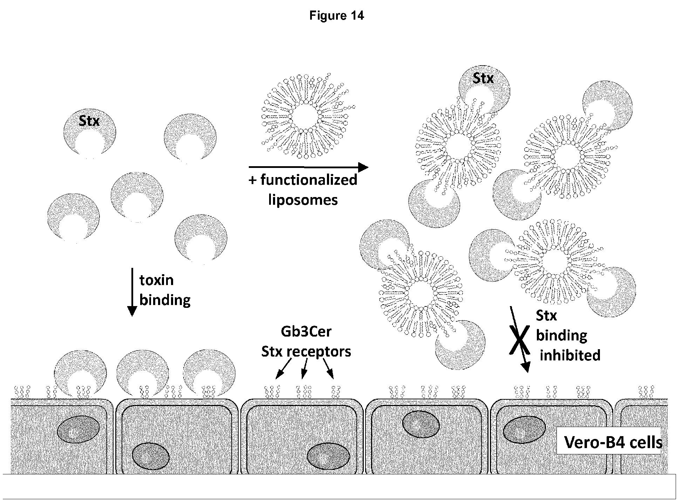

The present invention also relates to a pharmaceutical composition comprising a coupling product of an oligosaccharide composition as described herein and a pharmaceutically acceptable carrier. In embodiments the coupling product of an oligosaccharide composition is formulated in lipid vesicles. The lipid vesicles may be selected from the group consisting of liposomes, liosomes, micelles and transfersomes. Preferably, the lipid vesicles are liposomes. As non-limiting examples the liposomes may be prepared using phosphatidyl compounds, such as, for example, phosphatidylglycerol, phosphatidylcholine, phosphatidylserine, phosphatidylethanolamine, sphingolipids, cerebrosides, gangliosides and diacylphosphatidylglycerols and cholesterol. Preferably, in any one of the pharmaceutical compositions comprising a coupling product of an oligosaccharide composition described herein the coupling product of an oligosaccharide composition is a neoglycolipid composition as described herein. The neoglycolipid composition is preferably a neoglycolipid composition as described herein above in which the oligosaccharides are coupled to a phosphatidylethanolamine. Preferably, the phosphatidylethanolamine has a structure as disclosed herein above with regard to the neoglycolipid composition and the methods of preparing a neoglycolipid composition. Preferably, in case that the pharmaceutical composition comprises a neoglycolipid composition as disclosed herein a phosphatidylcholine is used for the formation of the liposomes. For example, a dioleoyl-phosphatidylcholine and cholesterol may be employed for the preparation of the liposomes. Example 6 illustrates the preparation of liposomes using a neoglycolipid composition as described herein. Figure 14 shows neoglycolipids of the present invention formulated into liposomes.

The present invention is also related to an oligosaccharide composition described herein or a pharmaceutical composition comprising an oligosaccharide composition described herein for use in treatment of a disease. In any one of the medical uses of an oligosaccharide composition or a pharmaceutical composition comprising an oligosaccharide composition disclosed herein the treatment of a disease may include a prophylactic and/or a therapeutic treatment. In particular, an oligosaccharide composition described herein or a pharmaceutical composition comprising an oligosaccharide composition described herein may be for use in treatment of a disease caused by a lectin or lectin producing pathogens. As non-limiting example an oligosaccharide composition described herein or a pharmaceutical composition comprising an oligosaccharide composition described herein may be for use in treatment of a disease caused by Shiga toxin or Shiga toxin producing pathogens. In this regard, the disease may be an infection by Shiga toxin producing pathogens or the disease may be caused by an infection by Shiga toxin producing pathogens. The pathogen may be a bacterial pathogen. As non-limiting examples, the bacterial pathogen may be *Shigella dysenteriae* or *Escherichia coli*. The pathogen may be a Shiga toxin producing *Escherichia coli* (STEC). The pathogen may be an enterohemorrhagic *Escherichia coli* (EHEC). The bacterial pathogen may be EHEC O157:H7 or EHEC O104:H4. Furthermore, the bacterial pathogen may be EHEC O111:H10, EHEC Ont:H-, EHEC O157:H7, EHEC O157:H-, EHEC O55:H7, EHEC O103:H2, EHEC O103:H-, EHEC OR:H2, EHEC O119:H2, EHEC O111:H8, EHEC O111:H-, EHEC O26:H11, EHEC O26:H-, EHEC OR:H11, EHEC Ont:Hnt, EHEC O145:H28, EHEC O145:H-, EHEC O112:H-, EHEC O73:H18, EHEC O55:Hnt, EHEC O113:H21, EHEC O163:H19, EHEC O128:H2, EHEC O70:H8, EHEC O98:H-, EHEC OR:H-, EHEC O136:Hnt, EHEC O145:H25, EHEC O91:H21, EHEC O121:H19, EHEC O145:H-, EHEC O104:H21, EHEC Ont:H21, EHEC O76:H19, EHEC O174:H21, EHEC O104:H4 or EHEC O165:H25 as disclosed in Mellmann A, Bielaszewska M, Köck R, Friedrich AW, Fruth A, Middendorf B, Harmsen D, Schmidt MA, Karch H. 2008 Emerg. Infect. Dis. 14(8), 1287-90 and http://campus.uni-muenster.de/hyg_klhus_husec.html, the whole disclosure of these documents incorporated herein by reference. The disease may be a gastrointestinal disorder. The gastrointestinal disorder may be diarrhea, hemorrhagic colitis, hemolytic uremic syndrome (HUS) or Shigellosis. The disease may be edema disease in pigs. In case that the disease is edema disease in pigs, the oligosaccharide composition or the pharmaceutical composition comprising an oligosaccharide composition may be administered together with the feed.

The present invention is also related to a method of treatment of a disease comprising administering to a subject an oligosaccharide composition described herein or a pharmaceutical composition comprising an oligosaccharide composition described herein. The method may be for prophylactic or therapeutic treatment of a disease. The administering may comprise to administer the oligosaccharide composition or the pharmaceutical composition comprising the oligosaccharide composition in an amount effective for a prophylactic or therapeutic treatment. In particular, the method of treatment may be for prophylactic or therapeutic treatment of a disease caused by a lectin or lectin producing pathogens. As non-limiting example the method of treatment may be for prophylactic or therapeutic treatment of a disease caused by Shiga toxin or Shiga toxin producing pathogens. In this regard, the disease may be an infection by Shiga toxin producing pathogens or the disease may be caused by an infection by Shiga toxin producing pathogens. The pathogen may be a bacterial pathogen. As non-limiting examples, the bacterial pathogen may be *Shigella dysenteriae* or *Escherichia coli.* The pathogen may be a Shiga toxin producing *Escherichia coli(STEC).* The pathogen may be an enterohemorrhagic *Escherichia coli* (EHEC)*.* The bacterial pathogen may be EHEC O157:H7 or EHEC O104:H4. Furthermore, the bacterial pathogen may be EHEC O111:H10, EHEC Ont:H-, EHEC O157:H7, EHEC O157:H-, EHEC O55:H7, EHEC O103:H2, EHEC O103:H-, EHEC OR:H2, EHEC O119:H2, EHEC O111:H8, EHEC O111:H-, EHEC O26:H11, EHEC O26:H-, EHEC OR:H11, EHEC Ont:Hnt, EHEC O145:H28, EHEC O145:H-, EHEC O112:H-, EHEC O73:H18, EHEC O55:Hnt, EHEC O113:H21, EHEC O163:H19, EHEC O128:H2, EHEC O70:H8, EHEC O98:H-, EHEC OR:H-, EHEC O136:Hnt, EHEC O145:H25, EHEC O91:H21, EHEC O121:H19, EHEC O145:H-, EHEC O104:H21, EHEC Ont:H21, EHEC O76:H19, EHEC O174:H21, EHEC O104:H4 or EHEC O165:H25 as disclosed in Mellmann A, Bielaszewska M, Köck R, Friedrich AW, Fruth A, Middendorf B, Harmsen D, Schmidt MA, Karch H. Emerg. Infect. Dis. 2008, 14(8), 1287-90 and http://campus.uni-muenster.de/hyg_klhus_husec.html, the whole disclosure of these documents being incorporated herein by reference. The disease may be a gastrointestinal disorder. The gastrointestinal disorder may be diarrhea, hemorrhagic colitis, hemolytic uremic syndrome (HUS) or Shigellosis. The disease may be edema disease in pigs. In case that the disease is edema disease in pigs, the oligosaccharide composition or the pharmaceutical composition comprising an oligosaccharide composition may be administered together with the feed.

The present invention is also related to a coupling product of an oligosaccharide composition described herein or a pharmaceutical composition comprising a coupling product of an oligosaccharide composition described herein for use in treatment of a disease. In any one of the medical uses of a coupling product of an oligosaccharide composition or a pharmaceutical composition comprising a coupling product of an oligosaccharide composition disclosed herein the treatment of a disease may include a prophylactic and/or a therapeutic treatment. In particular, a coupling product of an oligosaccharide composition described herein or a pharmaceutical composition comprising a coupling product of an oligosaccharide composition described herein may be for use in treatment of a disease caused by a lectin or lectin producing pathogens. As non-limiting example a coupling product of an oligosaccharide composition described herein or a pharmaceutical composition comprising a coupling product of an oligosaccharide composition described herein may be for use in treatment of a disease caused by Shiga toxin or Shiga toxin producing pathogens. In this regard, the disease may be an infection by Shiga toxin producing pathogens or the disease may be caused by an infection by Shiga toxin producing pathogens. The pathogen may be a bacterial pathogen. As non-limiting examples, the bacterial pathogen may be *Shigella dysenteriae* or *Escherichia coli.* The pathogen may be a Shiga toxin producing *Escherichia coli* (STEC). The pathogen may be an enterohemorrhagic *Escherichia coli* (EHEC). The bacterial pathogen may be EHEC O157:H7 or EHEC O104:H4. Furthermore, the bacterial pathogen may be EHEC O111:H10, EHEC Ont:H-, EHEC O157:H7, EHEC O157:H-, EHEC O55:H7, EHEC O103:H2, EHEC O103:H-, EHEC OR:H2, EHEC O119:H2, EHEC O111:H8, EHEC O111:H-, EHEC O26:H11, EHEC O26:H-, EHEC OR:H11, EHEC Ont:Hnt, EHEC O145:H28, EHEC O145:H-, EHEC O112:H-, EHEC O73:H18, EHEC O55:Hnt, EHEC O113:H21, EHEC O163:H19, EHEC O128:H2, EHEC O70:H8, EHEC O98:H-, EHEC OR:H-, EHEC O136:Hnt, EHEC O145:H25, EHEC O91:H21, EHEC O121:H19, EHEC O145:H-, EHEC O104:H21, EHEC Ont:H21, EHEC O76:H19, EHEC O174:H21, EHEC O104:H4 or EHEC O165:H25 as disclosed in Mellmann A, Bielaszewska M, Köck R, Friedrich AW, Fruth A, Middendorf B, Harmsen D, Schmidt MA, Karch H. 2008 Emerg. Infect. Dis. 14(8), 1287-90 and http://campus.uni-muenster.de/hyg_klhus_husec.html, the whole disclosure of these documents being incorporated herein by reference. The disease may be a gastrointestinal disorder. The gastrointestinal disorder may be diarrhea, hemorrhagic colitis, hemolytic uremic syndrome (HUS) or Shigellosis. The disease may be edema disease in pigs. In case that the disease is edema disease in pigs, the oligosaccharide composition or the pharmaceutical composition comprising an oligosaccharide composition may be administered together with the feed. In any one of the uses for treatment of a disease described herein the coupling product of an oligosaccharide composition is preferably a neoglycolipid composition as described herein. Preferably, the neoglycolipid composition is a neoglycolipid composition as described herein above in which the oligosaccharides are coupled to a phosphatidylethanolamine as also disclosed herein above. Preferably, the phosphatidylethanolamine has a structure as disclosed herein above with regard to the neoglycolipid composition and the methods of preparing a neoglycolipid composition.

The present invention is also related to a method of treatment of a disease comprising administering to a subject a coupling product of an oligosaccharide composition described herein or a pharmaceutical composition comprising a coupling product of an oligosaccharide composition described herein. The method may be for therapeutic or prophylactic treatment of a disease. The administering may comprise to administer the coupling product of an oligosaccharide composition or the pharmaceutical composition comprising a coupling product of an oligosaccharide composition in an amount effective for a prophylactic or therapeutic treatment. In particular, the method of treatment may be for prophylactic or therapeutic treatment of a disease caused by a lectin or lectin producing pathogens. As non-limiting example the method of treatment may be for prophylactic or therapeutic treatment of a disease caused by Shiga toxin or Shiga toxin producing pathogens. In this regard, the disease may be an infection by Shiga toxin producing pathogens or the disease may be caused by an infection by Shiga toxin producing pathogens. The pathogen may be a bacterial pathogen. As non-limiting examples, the bacterial pathogen may be *Shigella dysenteriae* or *Escherichia coli.* The pathogen may be a Shiga toxin producing *Escherichia coli(STEC).* The pathogen may be an enterohemorrhagic *Escherichia coli*(EHEC). The bacterial pathogen may be EHEC O157:H7 or EHEC O104:H4. Furthermore, the bacterial pathogen may be EHEC O111:H10, EHEC Ont:H-, EHEC O157:H7, EHEC O157:H-, EHEC O55:H7, EHEC O103:H2, EHEC O103:H-, EHEC OR:H2, EHEC O119:H2, EHEC O111:H8, EHEC O111:H-, EHEC O26:H11, EHEC O26:H-, EHEC OR:H11, EHEC Ont:Hnt, EHEC O145:H28, EHEC O145:H-, EHEC O112:H-, EHEC O73:H18, EHEC O55:Hnt, EHEC O113:H21, EHEC O163:H19, EHEC O128:H2, EHEC O70:H8, EHEC O98:H-, EHEC OR:H-, EHEC O136:Hnt, EHEC O145:H25, EHEC O91:H21, EHEC O121:H19, EHEC O145:H-, EHEC O104:H21, EHEC Ont:H21, EHEC O76:H19, EHEC O174:H21, EHEC O104:H4 or EHEC O165:H25 as disclosed in Mellmann A, Bielaszewska M, Köck R, Friedrich AW, Fruth A, Middendorf B, Harmsen D, Schmidt MA, Karch H. 2008 Emerg. Infect. Dis. 14(8), 1287-90 and http://campus.uni-muenster.de/hyg_klhus_husec.html, the whole disclosure of these documents being incorporated herein by reference. The disease may be a gastrointestinal disorder. The gastrointestinal disorder may be diarrhea, hemorrhagic colitis, hemolytic uremic syndrome (HUS) or Shigellosis. The disease may be edema disease in pigs. In case that the disease is edema disease in pigs, the oligosaccharide composition or the pharmaceutical composition comprising an oligosaccharide composition may be administered together with the feed. In any one of the methods of treatment of a disease described herein the coupling product of an oligosaccharide composition is preferably a neoglycolipid composition as described herein. Preferably, the neoglycolipid composition is a neoglycolipid composition as described herein above in which the oligosaccharides are coupled to a phosphatidylethanolamine as also disclosed herein above. Preferably, the phosphatidylethanolamine has a structure as disclosed herein above with regard to the neoglycolipid composition and the methods of preparing a neoglycolipid composition.

The oligosaccharide composition described herein is also suitable for the treatment of a disease which is caused by uropathogenic *Escherichia coli* (UPEC). Accordingly, the present invention is also related to an oligosaccharide composition described herein or a pharmaceutical composition comprising an oligosaccharide composition described herein for use in treatment of a disease, wherein the disease is an infection by uropathogenic *Escherichia coli*(UPEC) or wherein the disease is caused by an infection with uropathogenic *Escherichia coli* (UPEC). In particular, the disease is an infection of the urinary tract. The treatment of a disease may include a prophylactic and/or a therapeutic treatment.

The present invention is also related to a method of treatment of a disease comprising administering to a subject an oligosaccharide composition described herein or a pharmaceutical composition comprising an oligosaccharide composition described herein, wherein the disease is an infection by uropathogenic *Escherichia coli(UPEC)* or wherein the disease is caused by an infection with uropathogenic *Escherichia coli*(UPEC). The method may be for prophylactic or therapeutic treatment of a disease. The administering may comprise to administer the oligosaccharide composition or the pharmaceutical composition comprising the oligosaccharide composition in an amount effective for a prophylactic or therapeutic treatment. In particular, the disease is an infection of the urinary tract.

The coupling product of an oligosaccharide composition described herein is also suitable for the treatment of a disease which is caused by uropathogenic *Escherichia coli* (UPEC). Accordingly, the present invention is also related to a coupling product of an oligosaccharide composition described herein or a pharmaceutical composition comprising a coupling product of an oligosaccharide composition described herein for use in treatment of a disease, wherein the disease is an infection by uropathogenic *Escherichia coli* (UPEC) or wherein the disease is caused by an infection with uropathogenic *Escherichia coli*(UPEC). The treatment of such disease may include a prophylactic and/or a therapeutic treatment. In particular, the inventors have found that a neoglycolipid composition described herein is suitable for the treatment of a disease which is caused by uropathogenic *Escherichia coli* (UPEC). Accordingly, in any one of the uses in treatment of a disease, wherein the disease is an infection by uropathogenic *Escherichia coli*(UPEC) or wherein the disease is caused by an infection with uropathogenic *Escherichia coli* (UPEC), described herein the coupling product of an oligosaccharide composition is preferably a neoglycolipid composition as described herein. In any one of the uses in treatment of a disease, wherein the disease is an infection by uropathogenic *Escherichia coli*(UPEC) or wherein the disease is caused by an infection with uropathogenic *Escherichia coli* (UPEC), described herein the pharmaceutical composition comprising a coupling product of an oligosaccharide composition is preferably a pharmaceutical composition comprising a neoglycolipid composition as described herein. Preferably, the neoglycolipid composition is a neoglycolipid composition as described herein above in which the oligosaccharides are coupled to a phosphatidylethanolamine as also disclosed herein above. Preferably, the phosphatidylethanolamine has a structure as disclosed herein above with regard to the neoglycolipid composition and the methods of preparing a neoglycolipid composition. In particular, the disease is an infection of the urinary tract.

The present invention is also related to a method of treatment of a disease comprising administering to a subject a coupling product of an oligosaccharide composition described herein or a pharmaceutical composition comprising a coupling product of an oligosaccharide composition described herein, wherein the disease is an infection by uropathogenic *Escherichia coli*(UPEC) or wherein the disease is caused by an infection with uropathogenic *Escherichia coli* (UPEC). The method may be for prophylactic or therapeutic treatment of a disease. The administering may comprise to administer the coupling product of an oligosaccharide composition or the pharmaceutical composition comprising a coupling product of an oligosaccharide composition in an amount effective for a prophylactic or therapeutic treatment. In particular, a neoglycolipid composition as described herein is suitable for the treatment of a disease which is caused by uropathogenic *Escherichia coli* (UPEC). Accordingly, in any one of the methods of treatment of a disease, wherein the disease is an infection by uropathogenic *Escherichia coli* (UPEC) or wherein the disease is caused by an infection with uropathogenic *Escherichia coli* (UPEC), described herein the coupling product of an oligosaccharide composition is preferably a neoglycolipid composition as described herein. In any one of the methods of treatment of a disease, wherein the disease is an infection by uropathogenic *Escherichia coli*(UPEC) or wherein the disease is caused by an infection with uropathogenic *Escherichia coli* (UPEC), described herein the pharmaceutical composition comprising a coupling product of an oligosaccharide composition is preferably a pharmaceutical composition comprising a neoglycolipid composition as described herein. Preferably, the neoglycolipid composition is a neoglycolipid composition as described herein above in which the oligosaccharides are coupled to a phosphatidylethanolamine as also disclosed herein above. Preferably, the phosphatidylethanolamine has a structure as disclosed herein above with regard to the neoglycolipid composition and the methods of preparing a neoglycolipid composition. In particular, the disease is an infection of the urinary tract.

Without wishing to be bound by theory it is assumed that the oligosaccharides of the oligosaccharide composition or the coupling product of an oligosaccharide composition, in particular the neoglycolipidis of the neoglycolipid composition, bind to the PapG adhesin carried by the P-fimbriae of uropathogenic *Escherichia coli* (UPEC), thereby preventing adhesion of uropathogenic *Escherichia coli*(UPEC) to uroepithelial cells.

The oligosaccharide composition described herein is also suitable for the treatment of a disease which is caused by *Streptococcus suis.* Accordingly, the present invention is also related to an oligosaccharide composition described herein or a pharmaceutical composition comprising an oligosaccharide composition described herein for use in treatment of a disease, wherein the disease is an infection by *Streptococcus suis* or wherein the disease is caused by an infection with *Streptococcus suis.* The disease may be meningitis, sepsis, pneumonia or endocarditis. In particular, the disease is meningitis, sepsis or pneumonia in pigs or meningitis in humans. The treatment of a disease may include a prophylactic and/or a therapeutic treatment.

The present invention is also related to a method of treatment of a disease comprising administering to a subject an oligosaccharide composition described herein or a pharmaceutical composition comprising an oligosaccharide composition described herein, wherein the disease is an infection by *Streptococcus suis* or wherein the disease is caused by an infection with *Streptococcus suis.* The method may be for prophylactic or therapeutic treatment of a disease. The administering may comprise to administer the oligosaccharide composition or the pharmaceutical composition comprising the oligosaccharide composition in an amount effective for a prophylactic or therapeutic treatment. The disease may be meningitis, sepsis, pneumonia or endocarditis. In particular, the disease is meningitis, sepsis or pneumonia in pigs or meningitis in humans.

The coupling product of an oligosaccharide composition described herein is also suitable for the treatment of a disease which is caused by *Streptococcus suis.* Accordingly, the present invention is also related to a coupling product of an oligosaccharide composition described herein or a pharmaceutical composition comprising a coupling product of an oligosaccharide composition described herein for use in treatment of a disease, wherein the disease is an infection by *Streptococcus suis* or wherein the disease is caused by an infection with *Streptococcus suis.* The treatment of such disease may include a prophylactic and/or a therapeutic treatment. In particular, a neoglycolipid composition described herein is suitable for the treatment of a disease which is caused by *Streptococcus suis.* Accordingly, in any one of the uses for treatment of a disease, wherein the disease is an infection by *Streptococcus suis* or wherein the disease is caused by an infection with *Streptococcus suis,* described herein the coupling product of an oligosaccharide composition is preferably a neoglycolipid composition as described herein. In any one of the uses in treatment of a disease, wherein the disease is an infection by uropathogenic *Streptococcus suis* or wherein the disease is caused by an infection with *Streptococcus suis,* described herein the pharmaceutical composition comprising a coupling product of an oligosaccharide composition is preferably a pharmaceutical composition comprising a neoglycolipid composition as described herein. Preferably, the neoglycolipid composition is a neoglycolipid composition as described herein above in which the oligosaccharides are coupled to a phosphatidylethanolamine as also disclosed herein above. Preferably, the phosphatidylethanolamine has a structure as disclosed herein above with regard to the neoglycolipid composition and the methods of preparing a neoglycolipid composition. The disease may be meningitis, sepsis, pneumonia or endocarditis. In particular, the disease is meningitis, sepsis or pneumonia in pigs or meningitis in humans.

The present invention is also related to a method of treatment of a disease comprising administering to a subject a coupling product of an oligosaccharide composition described herein or a pharmaceutical composition comprising a coupling product of an oligosaccharide composition described herein, wherein the disease is an infection by *Streptococcus suis* or wherein the disease is caused by an infection with *Streptococcus suis.* The method may be for prophylactic or therapeutic treatment of a disease. The administering may comprise to administer the coupling product of an oligosaccharide composition or the pharmaceutical composition comprising a coupling product of an oligosaccharide composition in an amount effective for a prophylactic or therapeutic treatment. In particular, a neoglycolipid composition described herein described herein is suitable for the treatment of a disease which is caused by *Streptococcus suis.* Accordingly, in any one of the methods of treatment of a disease, wherein the disease is an infection by *Streptococcus suis* or wherein the disease is caused by an infection with *Streptococcus suis,* described herein the coupling product of an oligosaccharide composition is preferably a neoglycolipid composition as described herein. In any one of the methods of treatment of a disease, wherein the disease is an infection by *Streptococcus suis* or wherein the disease is caused by an infection with *Streptococcus suis,* described herein the pharmaceutical composition comprising a coupling product of an oligosaccharide composition is preferably a pharmaceutical composition comprising a neoglycolipid composition as described herein. Preferably, the neoglycolipid composition is a neoglycolipid composition as described herein above in which the oligosaccharides are coupled to a phosphatidylethanolamine as also disclosed herein above. Preferably, the phosphatidylethanolamine has a structure as disclosed herein above with regard to the neoglycolipid composition and the methods of preparing a neoglycolipid composition. The disease may be meningitis, sepsis, pneumonia or endocarditis. In particular, the disease is meningitis, sepsis or pneumonia in pigs or meningitis in humans.

Without wishing to be bound by theory it is assumed that the oligosaccharides of the oligosaccharide composition or the coupling product of an oligosaccharide composition, in particular the neoglycolipidis of the neoglycolipid composition, bind to the SadP adhesin of *Streptococcus suis,* thereby preventing adhesion of *Streptococcus suis* to host receptors.

The present inventors have further found that the neoglycolipid composition described herein can be used for diagnostic applications. Accordingly, the present invention also relates to a method of determining a lectin in a sample comprising contacting a sample suspected to contain a lectin with a neoglycolipid composition as defined herein. The method of determining a lectin may comprise contacting the sample with the neoglycolipid composition over a suitable period of time to enable complex formation of the lectin with the neoglycolipids. The determining may comprise determining of the absence or presence, the amount or the subtype of the lectin in the sample. The method may comprise, in particular in case that the method is for determining a subtype of a Shiga toxin, the steps of:
(a) separating a neoglycolipid composition of any one of claims 55 to 59 on a thin layer chromatography plate;
(b) detecting the neoglycolipids using the lectin which is to be determined thereby obtaining a binding pattern; and
(c) comparing the binding pattern with a reference binding pattern of the lectin. Step (a) of this method may be performed on a silica gel thin layer chromatography plate. A suitable silica gel is, for example, silica gel 60. Step (b) of this method may comprise the following steps:
   (b1) contacting the thin layer chromatography plate with the lectin to be determined;
   (b2) contacting the thin layer chromatography plate with a primary antibody binding to the lectin;
   (b3) contacting the thin layer chromatography plate with a secondary antibody binding to the primary antibody; and
   (b4) detecting a construct of a neoglycolipid, the lectin, the primary antibody and the secondary antibody.

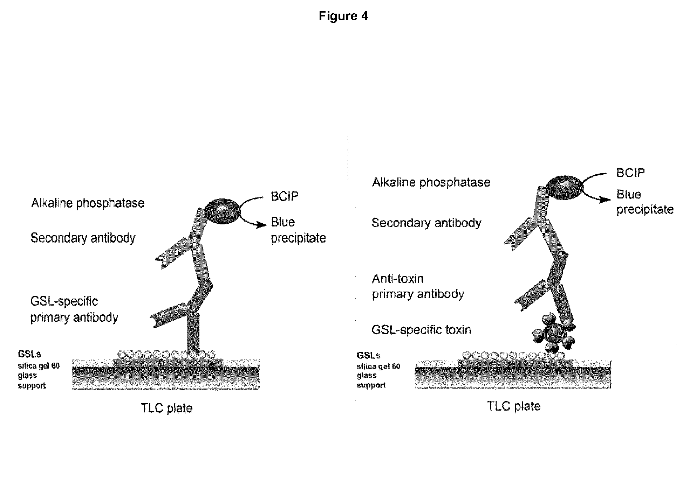

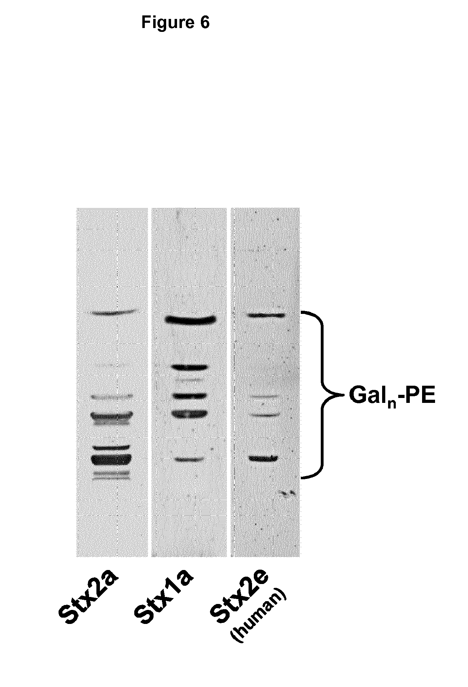

This method is similar to an enzyme-linked immunosorbent assay (ELISA) (Müthing J, Distler U. Advances on the compositional analysis of glycosphingolipids combining thin-layer chromatography with mass spectrometry. 2010 Mass Spectrom Rev 29, 425-479; Meisen I, Mormann M, Müthing J. Thin-layer chromatography, overlay technique and mass spectrometry: a versatile triad advancing glyocsphingolipidomics. 2011 Biochim. Biophys. Acta 1811, 875-896; Müsken A, Souady J, Dreisewerd K, Zhang W, Distler U, Peter-Katalinic J, Miller-Podraza H, Karch H, Müthing J. Application of thin-layer chromatography/infrared matrix-assisted laser desorption/ionization orthogonal time-of-flight mass spectrometry to structural analysis of bacteria-binding glycosphingolipids selected by affinity detection. 2010 Rapid Commun Mass Spectrom 24, 1032-1038). Below Example 4 and Figures 4 to 12 show a thin layer chromatography overlay assay which is in accordance with the present invention). In step (b3) a secondary antibody which is coupled to an alkaline phosphatase may be used and in step (b4) detection may be performed by contacting the thin layer chromatography plate with 5-bromo-4-chloro-3-indolylphosphate. The alkaline phosphatase cleaves the phosphate group of the 5-bromo-4-chloro-3-indolylphosphate which leads to formation of a blue precipitate which visualizes the construct of the neoglycolipid, the lectin, the primary antibody and the secondary antibody. Alternatively, detection may be performed using a secondary antibody coupled to horseradish peroxidase and an appropriate substrate for horseradish peroxidase, or detection may be performed using a fluorochrome-labeled secondary antibody. The thin layer chromatography bands may be further analyzed using mass spectrometry which allows to determine the number of saccharide units of the neoglycolipid to which the lectin is bound. The lectin which is to be determined by any one of the methods of determining a lectin described herein is preferably a Shiga toxin. Accordingly, the present invention also relates to a method of determining a Shiga toxin in a sample comprising contacting a sample suspected to contain a Shiga toxin with a neoglycolipid composition as defined herein. The method of determining a Shiga toxin may be carried out using any one of the methods and steps described herein in the context of determining a lectin. The Shiga toxin may be a Shiga toxin 1 or a Shiga toxin 2 The Shiga toxin 1 may be selected from the group consisting of Stx1a, Stx1c and Stx1d. Preferably, the Shiga toxin 1 is Stx1 a. The Shiga toxin 2 may be selected from the group consisting of Stx2a, Stx2b, Stx2c, Stx2d, Stx2e, Stx2f and Stx2g. Preferably, the Shiga toxin 2 is Stx2a or Stx2e. The Shiga toxin, in particular, the Shiga toxin 1 or the Shiga toxin 2, may be from a Shiga toxin producing *Escherichia coli*(STEC). The methods of determining a lectin described herein are particularly suitable for determining the subtype of a Shiga toxin as illustrated by Example 4 in combination with Figures 4 to 12. In this regard, Example 4 describes performing a thin layer chromatography overlay assay, and Figures 6 to 12 show that specific binding patterns are obtained for different subtypes of Shiga toxin by a thin layer chromatography overlay assay. In the methods of determining a lectin described herein the lectin may be an adhesin. For example, the adhesin may be a PapG adhesin from uropathogenic *Escherichia coli* (UPEC). More specifically, the adhesin may be a PapG-I, PapG-II or PapG-III adhesin from uropathogenic *Escherichia coli* (UPEC). Accordingly, the present invention also relates to a method of determining a uropathogenic *Escherichia coli* in a sample comprising contacting a sample suspected to contain a uropathogenic *Escherichia coli* with a neoglycolipid composition as defined herein. The method of determining a uropathogenic *Escherichia coli* may be carried out using any one of the methods and steps described herein in the context of determining a lectin. For example, the adhesin may be SadP from *Streptococcus suis.* Accordingly, the present invention also relates to a method of determining *Streptococcus suis* in a sample comprising contacting a sample suspected to contain *Streptococcus suis* with a neoglycolipid composition as defined herein. The method of determining *Streptococcus suis* may be carried out using any one of the methods and steps described herein in the context of determining a lectin. In any one of the methods disclosed herein of determining a lectin, in particular a Shiga toxin, a uropathogenic *Escherichia coli* or *Streptococcus suis,* the neoglycolipid composition is preferably a neoglycolipid composition as described herein above in which the oligosaccharides are coupled to a phosphatidylethanolamine as also disclosed herein above. Preferably, the phosphatidylethanolamine has a structure as disclosed herein above with regard to the neoglycolipid composition and the methods of preparing a neoglycolipid composition. Such neoglycolipid composition in which the oligosaccharides are coupled to a phosphatidylethanolamine having a structure as described herein above are suitable for determining a Shiga toxin, in particular for determining the subtype of a Shiga toxin. As a purely illustrative example, Example 5 in connection with Figures 4 to 12 shows determination of Shiga toxin subtypes via specific binding patterns using thin layer chromatography overlay assays.

The present invention further relates to a kit comprising a neoglycolipid composition as described herein and at least one lectin. Such kit may, for example, be used for determining a lectin such as, for example, in any one of the methods of determining a lectin in a sample as described herein above. In this regard, the lectin comprised in the kit may be used as reference. The lectin may be a Shiga toxin In particular, the Shiga toxin may be a Shiga toxin 1 or Shiga toxin 2. The Shiga toxin 1 may be selected from the group consisting of Stx1 a, Stx1 c and Stx1 d. Preferably, the Shiga toxin 1 is Stx1 a. The Shiga toxin 2 may be selected from the group consisting of Stx2a, Stx2b, Stx2c, Stx2d, Stx2e, Stx2f and Stx2g. Preferably, the Shiga toxin 2 is Stx2a or Stx2e. In any one of the kits disclosed herein the neoglycolipid composition is preferably a neoglycolipid composition as described herein above in which the oligosaccharides are coupled to a phosphatidylethanolamine as also disclosed herein above. Preferably, the phosphatidylethanolamine has a structure as disclosed herein above with regard to the neoglycolipids and the methods of preparing a neoglycolipid composition. Such neoglycolipid composition in which the oligosaccharides are coupled to a phosphatidylethanolamine having a structure as described herein above are suitable for determining a Shiga toxin, in particular for determining the subtype of a Shiga toxin. Hence, the kit disclosed herein is suitable for determining a Shiga toxin, in particular for determining the subtype of a Shiga toxin.

The present invention is further characterized by the following list of items:
Item 1:
   A method of preparing an oligosaccharide composition, said method comprising the steps of:
      (a) providing a pectin; and
      (b) performing, in any order, conversion of carboxylic acid groups to the corresponding alcohols and cleavage of glycosidic bonds under conditions suitable to give an oligosaccharide composition.
Item 2:
   The method of preparing an oligosaccharide composition of item 1, said method comprising the steps of:
      (a) providing a pectin;
      (b1) conversion of carboxylic acid groups of the pectin to the corresponding alcohols; and
      (b2) cleavage of glycosidic bonds of the conversion product of step (b1) under conditions suitable to give an oligosaccharide composition.
Item 3:
   The method of preparing an oligosaccharide composition of item 1, said method comprising the steps of:
      (a) providing a pectin;
      (b1) cleavage of glycosidic bonds of the pectin under conditions suitable to give an oligomeric pectin cleavage product; and
      (b2) conversion of carboxylic acid groups of the cleavage product of step (b1) to the corresponding alcohols to give an oligosaccharide composition.
Item 4:
   The method of any one of the preceding items, wherein the method further comprises at least partial separation of oligosaccharides having non-reduced carboxylic acid groups.
Item 5:
   The method of item 4, wherein the separation is performed using anion exchange chromatography or extraction.
Item 6:
   The method of item 5, wherein the anion exchange chromatography is performed using a cross-linked agarose modified with diethylaminoethyl groups, a cross-linked agarose modified with dimethylaminoethyl groups, a cross-linked agarose modified with trimethylaminoethyl groups, a cross-linked cellulose modified with diethylaminoethyl groups, a cross-linked cellulose modified with dimethylaminoethyl groups, or a cross-linked cellulose modified with trimethylaminoethyl groups as stationary phase.
Item 7:
   The method of any one of the preceding items, wherein the conversion of carboxylic acid groups to the corresponding alcohols comprises activating the carboxylic acid groups with a carbodiimide and reducing the obtained conjugate of the carboxylic acid groups and the carbodiimide using a borohydride.
Item 8:
   The method of item 7, wherein the borohydride is selected from the group consisting of lithium borohydride, sodium borohydride, potassium borohydride and any combinations thereof.
Item 9:
   The method of item 8, wherein the borohydride is sodium borohydride.
Item 10:
   The method of any one of items 7 to 9, wherein the carbodiimide is *N-*cyclohexyl-*N'*-(2-morpholinoethyl)carbodiimide metho-*p*-toluenesulfonate and/or 1-ethyl-3-(3-dimethylaminopropyl)carbodiimide.
Item 11:
   The method of any one of the preceding items, wherein the composition obtained by the conversion of the carboxylic acid groups to the corresponding alcohols is purified.
Item 12:
   The method of item 11, wherein the composition is purified using a method selected from the group consisting of dialysis, ultrafiltration and size exclusion chromatography.
Item 13:
   The method of item 12, wherein the composition is purified using dialysis.
Item 14:
   The method of any one of the preceding items, wherein the cleavage of glycosidic bonds is performed using hydrolysis of glycosidic bonds.
Item 15:
   The method of item 14, wherein the hydrolysis of glycosidic bonds is performed in presence of an acid.
Item 16:
   The method of item 15, wherein the acid is trifluoroacetic acid.
Item 17:
   The method of item 15 or 16, wherein the hydrolysis is performed at a temperature of from 70 to 120 °C for 5 minutes to 3 hours, preferably at a temperature of from 80 to 110 °C for 10 minutes to 60 minutes, more preferably at a temperature of from 90 to 95 °C for 30 to 40 minutes, most preferably at a temperature of from 100 to 105 °C for 15 to 20 minutes.
Item 18:
   The method of any one of items 15 to 17, wherein the pH of the reaction mixture is between 2 and 5, preferably between 2.1 and 4, more preferably between 2.2 and 3.5, most preferably between 2.3 and 3.0.
Item 19:
   The method of item 14, wherein the hydrolysis of glycosidic bonds is performed using an enzyme, preferably using a glycosidase.
Item 20:
   The method of any one of the preceding items, wherein the composition after cleavage of glycosidic bonds is analyzed using mass spectrometry, HPLC, capillary gel electrophoresis and/or hydrophilic interaction chromatography.
Item 21:
   The method of item 20, wherein the mass spectrometry is performed using atmospheric pressure chemical ionization (APCI), direct chemical ionization (DCI), electrospray ionization (ESI), fast atom bombardment (FAB), and/or matrix-assisted laser desorption ionization (MALDI).
Item 22:
   The method of item 20, wherein the HPLC is performed using high-performance anion-exchange chromatography with pulsed amperometric detection (HPAEC-PAD).
Item 23:
   The method of any one of the preceding items, wherein the cleavage of glycosidic bonds is terminated at a time at which one or more of an oligosaccharide selected from the group consisting of an oligosaccharide having 20 saccharide units, an oligosaccharide having 19 saccharide units, an oligosaccharide having 18 saccharide units, an oligosaccharide having 17 saccharide units, an oligosaccharide having 16 saccharide units, an oligosaccharide having 15 saccharide units, an oligosaccharide having 14 saccharide units, an oligosaccharide having 13 saccharide units, an oligosaccharide having 12 saccharide units, an oligosaccharide having 11 saccharide units, an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units is present in the reaction mixture.
Item 24:
   The method of item 23, wherein the cleavage of glycosidic bonds is terminated at a time at which one or more of an oligosaccharide selected from the group consisting of 15 saccharide units, an oligosaccharide having 14 saccharide units, an oligosaccharide having 13 saccharide units, an oligosaccharide having 12 saccharide units, an oligosaccharide having 11 saccharide units, an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units is present in the reaction mixture.
Item 25:
   The method of item 24, wherein the cleavage of glycosidic bonds is terminated at a time at which one or more of an oligosaccharide selected from the group consisting of an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units is present in the reaction mixture.
Item 26:
   The method of item 25, wherein the cleavage of glycosidic bonds is terminated at a time at which one or more of an oligosaccharide selected from the group consisting of an oligosaccharide having 7 saccharide units, an oligosaccharide having 6 saccharide units, an oligosaccharide having 5 saccharide units, an oligosaccharide having 4 saccharide units, and an oligosaccharide having 3 saccharide units is present in the reaction mixture.
Item 27:
   The method of item 26, wherein the cleavage of glycosidic bonds is terminated at a time at which one or more of an oligosaccharide selected from the group consisting of an oligosaccharide having 6 saccharide units, an oligosaccharide having 5 saccharide units, an oligosaccharide having 4 saccharide units, and an oligosaccharide having 3 saccharide units is present in the reaction mixture.
Item 28:
   The method of item 27, wherein the cleavage of glycosidic bonds is terminated at a time at which one or more of an oligosaccharide selected from the group consisting of an oligosaccharide having 5 saccharide units, and an oligosaccharide having 4 saccharide units is present in the reaction mixture.
Item 29:
   The method of any one of items 23 to 28, wherein the oligosaccharides comprise one or more of a saccharide unit selected from the group consisting of galactose, galacturonic acid, and galacturonic acid alkyl ester.
Item 30:
   The method of any one of the preceding items, wherein the pectin is selected from the group consisting of pectin from apples, pectin from citrus fruits, pectin from vegetables, pectin from carrots, pectin from sugar beets, pectin from fodder beets, pectin from mangold, pectin from sunflower heads and any combinations thereof.
Item 31:
   An oligosaccharide composition obtainable or being obtained by the method of any one of items 1 to 30.
Item 32:
   The oligosaccharide composition of item 31, wherein the oligosaccharide composition comprises one or more of an oligosaccharide selected from the group consisting of an oligosaccharide having 20 saccharide units, and oligosaccharide having 19 saccharide units, an oligosaccharide having 18 saccharide units, an oligosaccharide having 17 saccharide units, an oligosaccharide having 16 saccharide units, an oligosaccharide having 15 saccharide units, an oligosaccharide having 14 saccharide units, an oligosaccharide having 13 saccharide units, an oligosaccharide having 12 saccharide units, an oligosaccharide having 11 saccharide units, an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units.
Item 33:
   The oligosaccharide composition of item 32, wherein the oligosaccharide composition comprises one or more of an oligosaccharide selected from the group consisting of an oligosaccharide having 15 saccharide units, an oligosaccharide having 14 saccharide units, an oligosaccharide having 13 saccharide units, an oligosaccharide having 12 saccharide units, an oligosaccharide having 11 saccharide units, an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units.
Item 34:
   The oligosaccharide composition of item 33, wherein the oligosaccharide composition comprises one or more of an oligosaccharide selected from the group consisting of an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units.
Item 35:
   The oligosaccharide composition of item 34, wherein the oligosaccharide composition comprises one or more of an oligosaccharide selected from the group consisting of an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units.
Item 36:
   The oligosaccharide composition of item 35, wherein the oligosaccharide composition comprises one or more of an oligosaccharide selected from the group consisting of an oligosaccharide having 7 saccharide units, an oligosaccharide having 6 saccharide units, an oligosaccharide having 5 saccharide units, an oligosaccharide having 4 saccharide units, and an oligosaccharide having 3 saccharide units.
Item 37:
   The oligosaccharide composition of item 36, wherein the oligosaccharide composition comprises one or more of an oligosaccharide selected from the group consisting of an oligosaccharide having 6 saccharide units, an oligosaccharide having 5 saccharide units, an oligosaccharide having 4 saccharide units, and an oligosaccharide having 3 saccharide units.
Item 38:
   The oligosaccharide composition of item 37, wherein the oligosaccharide composition comprises one or more of an oligosaccharide selected from the group consisting of an oligosaccharide having 5 saccharide units, and an oligosaccharide having 4 saccharide units.
Item 39:
   The oligosaccharide composition of any one of items 32 to 38, wherein the oligosaccharides comprise one or more of a saccharide unit selected from the group consisting of galactose, galacturonic acid, and galacturonic acid alkyl ester.
Item 40:
   The oligosaccharide composition of any one of items 31 to 39, wherein the composition is solid.
Item 41:
   A method of preparing a coupling product of an oligosaccharide composition, said method comprising the steps of:
      (a) providing an oligosaccharide composition of any one of items 31 to 40, in particular an oligosaccharide composition obtained by any one of the methods of any one of items 1 to 30; and
      (b) forming a coupling product of an oligosaccharide composition from the oligosaccharide composition and one or more of a coupling partner.
Item 42:
   A method of preparing a coupling product of an oligosaccharide composition, said method comprising the steps of:
      (a1) providing a pectin;
      (a2) cleavage of glycosidic bonds of the pectin under conditions suitable to give an oligosaccharide composition;
      (b) forming a coupling product of an oligosaccharide composition from the oligosaccharide composition and one or more of a coupling partner; and
      (c) conversion of carboxylic acid groups of the coupling product of step (b) to the corresponding alcohols.
Item 43:
   The method of preparing a coupling product of an oligosaccharide composition of item 41 or 42, wherein the coupling partner is selected from the group consisting of a lipid anchor, preferably a phosphatidylethanolamine, a Yariv reagent, a silica gel, a glucose functionalized with a spacer, an acrylamide polymer, an acrylamide copolymer, a carbosilane, a chitosan, a nanoparticle and any combination thereof.
Item 44:
   The method of item 43, wherein the coupling partner is a phosphatidylethanolamine, said method comprising the steps of:
      (a) providing an oligosaccharide composition of any one of items 31 to 40, in particular an oligosaccharide composition obtained by any one of the methods of any one of items 1 to 30; and
      (b) forming a neoglycolipid composition from the oligosaccharide composition and one or more of a phosphatidylethanolamine having a structure of: 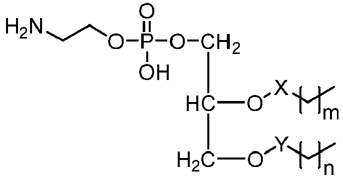 or any suitable salt thereof, wherein m and n is each, independently, an integer of from 8 to 20, wherein X and Y is each, independently, CH₂ or C=O; and wherein a carbon-nitrogen bond is formed between an oligosaccharide and a phosphatidylethanolamine.
Item 45:
   The method of item 43, wherein the coupling partner is a phosphatidylethanolamine, said method comprising the steps of:
      (a1) providing a pectin;
      (a2) cleavage of glycosidic bonds of the pectin under conditions suitable to give an oligosaccharide composition;
      (b) forming a neoglycolipid composition from the oligosaccharide composition and one or more of a phosphatidylethanolamine having a structure of: 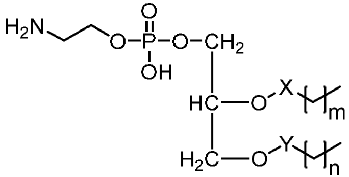 or any suitable salt thereof, wherein m and n is each, independently, an integer of from 8 to 20, wherein X and Y is each, independently, CH₂ or C=O; and wherein a carbon-nitrogen bond is formed between an oligosaccharide and a phosphatidylethanolamine; and
      (c) conversion of carboxylic acid groups of the neoglycolipid composition of step (b) to the corresponding alcohols.
Item 46:
   The method of item 44 or 45, wherein step (b) is performed by (b1) reacting the oligosacchararide composition with the phosphatidylethanolamine and (b2) treating the product obtained in step (b1) with a reducing agent.
Item 47:
   The method of item 46, wherein the reducing agent is selected from the group consisting of lithium borohydride, sodium borohydride, potassium borohydride, sodium triacetoxyborohydride, sodium cyanoborohydride and any combinations thereof.
Item 48:
   The method of item 47, wherein the reducing agent is sodium cyanoborohydride.
Item 49:
   The method of any one of items 44 to 49, wherein m and n is each, independently, an integer of from 10 to 18, preferably of from 12 to 16, and most preferably m and n are each 14.
Item 50:
   The method of any one of items 44 to 48, wherein m and n are the same.
Item 51:
   The method of any one of items 44 to 50, wherein X and Y are the same.
Item 52:
   The method of any one of items 44 to 51, wherein X and Y are CH₂, wherein m and n are the same and an integer of from 8 to 20, preferably of from 10 to 18, more preferably of from 12 to 16 and most preferably 14.
Item 53:
   The method of any one of items 44 to 51, wherein X and Y are C=O, wherein m and n are the same and an integer of from 8 to 20, preferably of from 10 to 18, more preferably of from 12 to 16 and most preferably 14.
Item 54:
   A coupling product of an oligosaccharide composition obtainable or being obtained by the method of any one of items 41 to 53.
Item 55:
   The coupling product of an oligosaccharide composition of item 54, wherein the coupling product is a neoglycolipid composition obtainable or being obtained by the method of any one of items 44 to 53.
Item 56:
   The neoglycolipid composition of item 55, comprising one or more of a neoglycolipid having the structure: 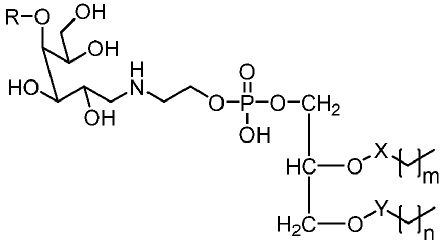 or any pharmaceutically acceptable salt thereof, wherein m and n is each, independently, an integer of from 8 to 20, wherein X and Y is each, independently, CH₂ or C=O; and wherein R is a residue comprising of from 1 to 19 saccharide units.
Item 57:
   The neoglycolipid composition of item 56, further comprising one or more of a compound having the structure: 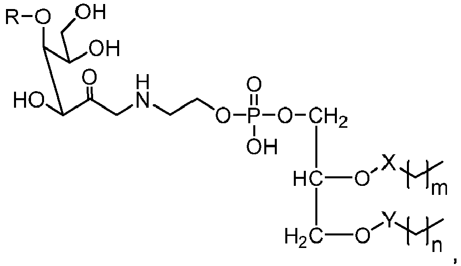 or any pharmaceutically acceptable salt or tautomer thereof, wherein m and n is each, independently, an integer of from 8 to 20, wherein X and Y is each, independently, CH₂ or C=O; and wherein R is a residue comprising of from 1 to 19 saccharide units.
Item 58:
   The neoglycolipid composition of item 56 or 57, wherein R is a residue consisting of from 1 to 19 saccharide units connected *via* glycosidic bonds.
Item 59:
   The neoglycolipid composition of any one of items 56 to 58, wherein the residue R comprises one or more of a saccharide unit selected from the group consisting of galactose, galacturonic acid, and galacturonic acid alkyl ester.
Item 60:
   A pharmaceutical composition comprising an oligosaccharide composition of any one of items 31 to 40 and a pharmaceutically acceptable carrier.
Item 61:
   The pharmaceutical composition of item 60, wherein the oligosaccharide composition is formulated in lipid versicles.
Item 62:
   The pharmaceutical composition of item 61, wherein the lipid vesicles are selected from the group consisting of liposomes, liosomes, micelles and transfersomes.
Item 63:
   The pharmaceutical composition of item 62, wherein the lipid vesicles are liposomes.
Item 64:
   A pharmaceutical composition comprising a coupling product of an oligosaccharide composition of any one of items 54 to 59 and a pharmaceutically acceptable carrier.
Item 65:
   The pharmaceutical composition of item 64, wherein the coupling product of an oligosaccharide composition is formulated in lipid vesicles.
Item 66:
   The pharmaceutical composition of item 65, wherein the lipid vesicles are selected from the group consisting of liposomes, liosomes, micelles and transfersomes.
Item 67:
   The pharmaceutical composition of item 66, wherein the lipid vesicles are liposomes.
Item 68:
   The pharmaceutical composition of item 64 to 67, wherein the coupling product of an oligosaccharide composition is a neoglycolipid composition of any one of items 56 to 59.
Item 69:
   The oligosaccharide composition of any one of items 31 to 40 or the pharmaceutical composition of any one of items 60 to 63 for use in treatment of a disease.
Item 70:
   The coupling product of an oligosaccharide composition of any one of items 54 to 59 or the pharmaceutical composition of any one of items 64 to 68 for use in treatment of a disease.
Item 71:
   The oligosaccharide composition of any one of items 31 to 40 or the pharmaceutical composition of any one of items 60 to 63 for use in treatment of a disease caused by a lectin or lectin producing pathogens.
Item 72:
   The coupling product of an oligosaccharide composition of any one of items 54 to 59 or the pharmaceutical composition of any one of items 64 to 68 for use in treatment of a disease caused by a lectin or lectin producing pathogens.
Item 73:
   The composition for use of item 71 or 72, wherein the disease is caused by Shiga toxin or Shiga toxin producing pathogens.
Item 74:
   The composition for use of item 73, wherein the disease is an infection by Shiga toxin producing pathogens or wherein the disease is caused by an infection by Shiga toxin producing pathogens.
Item 75:
   The composition for use of claim 74, wherein the pathogen is a bacterial pathogen.
Item 76:
   The composition for use of item 75, wherein the bacterial pathogen is *Shigella dysenteriae* or *Escherichia coli.*
Item 77:
   The composition for use of item 76, wherein the bacterial pathogen is a Shiga-toxin producing *Escherichia coli*(STEC).
Item 78:
   The composition for use of item 77, wherein the bacterial pathogen is an enterohemorrhagic *Escherichia coli*(EHEC).
Item 79:
   The composition for use of item 78, wherein the bacterial pathogen is EHEC O157:H7 or EHEC O104:H4.
Item 80:
   The composition for use of any one of items 71 to 79, wherein the disease is a gastrointestinal disorder.
Item 81:
   The composition for use of item 80, wherein the gastrointestinal disorder is diarrhea, hemorrhagic colitis, hemolytic uremic syndrome (HUS) or Shigellosis.
Item 82:
   The composition for use of any one of items 71 to 79, wherein the disease is edema disease in pigs.
Item 83:
   The oligosaccharide composition of any one of items 31 to 40 or the pharmaceutical composition of any one of items 60 to 63 for use in treatment of a disease, wherein the disease is an infection by uropathogenic *Escherichia coli* (UPEC) or wherein the disease is caused by an infection with uropathogenic *Escherichia coli*(UPEC).
Item 84:
   The coupling product of an oligosaccharide composition of any one of items 54 to 59 or the pharmaceutical composition of any one of items 64 to 68 for use in treatment of a disease, wherein the disease is an infection by uropathogenic *Escherichia coli*(UPEC) or wherein the disease is caused by an infection with uropathogenic *Escherichia coli*(UPEC).
Item 85:
   The composition for use of items 83 or 84, wherein the disease is an infection of the urinary tract.
Item 86:
   The oligosaccharide composition of any one of items 31 to 40 or the pharmaceutical composition of any one of items 60 to 63 for use in treatment of a disease, wherein the disease is an infection by *Streptococcus suis* or wherein the disease is caused by an infection with *Streptococcus suis.*
Item 87:
   The coupling product of an oligosaccharide composition of any one of items 54 to 59 or the pharmaceutical composition of any one of items 64 to 68 for use in treatment of a disease, wherein the disease is an infection by *Streptococcus suis* or wherein the disease is caused by an infection with *Streptococcus suis.*
Item 88:
   The composition for use of item 86 or 87, wherein the disease is meningitis, sepsis, pneumonia or endocarditis.
Item 89:
   A method of determining a lectin in a sample comprising contacting a sample suspected to contain a lectin with a neoglycolipid composition as defined in any one of items 55 to 59.
Item 90:
   The method of item 89 comprising contacting the sample with the neoglycolipid composition over a suitable period of time to enable complex formation of the lectin with the neoglycolipids.
Item 91:
   The method of item 89 or 90, wherein the determining comprises determining of the absence or presence, the amount or the subtype of the lectin in the sample.
Item 92:
   The method of any one of item 89 to 91, wherein the method comprising the steps of:
      (a) separating a neoglycolipid composition of any one of items 55 to 59 on a thin layer chromatography plate;
      (b) detecting the neoglycolipids using the lectin which is to be determined thereby obtaining a binding pattern; and
      (c) comparing the binding pattern with a reference binding pattern of the lectin.
Item 93:
   The method of item 92, wherein step (a) is performed on a silica gel thin layer chromatography plate.
Item 94:
   The method of item 92 or 93, wherein step (b) comprises the following steps:
      (b1) contacting the thin layer chromatography plate with the lectin to be determined;
      (b2) contacting the thin layer chromatography plate with a primary antibody binding to the lectin;
      (b3) contacting the thin layer chromatography plate with a secondary antibody binding to the primary antibody; and
      (b4) detecting a construct of a neoglycolipid, the lectin, the primary antibody and the secondary antibody.
Item 95:
   The method of item 94, wherein in step (b3) a secondary antibody which is coupled to an alkaline phosphatase is used and wherein in step (b4) detection is performed by contacting the thin layer chromatography plate with 5-bromo-4-chloro-3-indolylphosphate.
Item 96:
   The method of any one of items 89 to 95, wherein the lectin which is to be determined is a Shiga toxin.
Item 97:
   The method of item 96, wherein the Shiga toxin is a Shiga toxin 1 and/or a Shiga toxin 2.
Item 98:
   The method of item 97, wherein the Shiga toxin 1 is selected from the group consisting of Stx1a, Stx1c and Stx1d.
Item 99:
   The method of item 98, wherein the Shiga toxin 1 is Stx1 a.
Item 100:
   The method of item 97, wherein the Shiga toxin 2 is selected from the group consisting of Stx2a, Stx2b, Stx2c, Stx2d, Stx2e, Stx2f and Stx2g.
Item 101:
   The method of item 100, wherein the Shiga toxin 2 is Stx2a and/or Stx2e.
Item 102:
   The method of any one of items 96 to 101, wherein the Shiga toxin is from a Shiga-toxin producing *Escherichia coli*(STEC)*.*
Item 103:
   The method of any one of items 89 to 95, wherein the lectin is an adhesin.
Item 104:
   The method of item 103, wherein the adhesin is a PapG adhesin from uropathogenic *Escherichia coli*(UPEC).
Item 105:
   The method of item 103, wherein the adhesin is SadP from *Streptococcus suis.*
Item 106:
   A kit comprising a neoglycolipid composition of any one of items 55 to 59 and at least one lectin.
Item 107:
   The kit of item 106, wherein the lectin is a Shiga toxin.
Item 108:
   The kit of item 106, wherein the Shiga toxin is a Shiga toxin 1 and/or a Shiga toxin 2.
Item 109:
   The kit of item 108, wherein the Shiga toxin 1 is selected from the group consisting of Stx1 a, Stx1c and Stx1 d.
Item 110:
   The kit of item 109, wherein the Shiga toxin is Stx1 a.
Item 111:
   The kit of item 108, wherein the Shiga toxin 2 is selected from the group consisting of Stx2a, Stx2b, Stx2c, Stx2d, Stx2e, Stx2f and Stx2g.
Item 112:
   The kit of item 111, wherein the Shiga toxin 2 is Stx2a and/or Stx2e.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of' does not exclude materials or steps that do not materially affect the basic and novel characteristics of the claim. In each instance herein any of the terms "comprising", "consisting essentially of" and "consisting of" may be replaced with either of the other two terms.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

### EXAMPLES

The following Examples further illustrate the invention. These examples should however not be construed as to limit the scope of this invention. The examples are included for purposes of illustration and the present invention is limited only by the claims.

### Example 1

### Preparation of Oligosaccharide Composition

3 g of *N*-cyclohexyl-*N'*(2-morpholinoethyl)carbodiimide metho-*p*-toluenesulfonate was dissolved in 200 ml of deionized water in a 1 I beaker. 100 mg of apple (or citrus) low methylated pectin (degree of esterification < 5%) in 15 to 20 ml of deionized water was added. The pH was adjusted to and kept at 4.75 by use of 0.1 M HCl. After roughly 2 h the pH shift stopped i.e. the reaction was completed. 20 g of NaBH₄ was dissolved in 100 ml of deionized water. The NaBH₄ solution was added dropwise over a time period of 2 to 3 h while keeping the pH at 7.0 by addition of 6 M HCl and avoiding excessive foaming as well as maintaining the temperature between 25 and 35 °C. After additional stirring for 1 h at ambient temperature the mixture was dialyzed against 5 l deionized water at 21 °C for at least 48 h with a fivefold water exchange. For hydrolysis of the glycosidic bonds the retenate was adjusted to 3.5 mM trifluoroacetic acid and boiled under reflux for 15 to 20 min. After cooling to 4 °C an aliquot of 200 µl was taken for MS analysis (*vide infra* Example 2) and evaporated *in vacuo.* The remaining hydrolysate was lyophilized. Galacturonate-containing oligosaccharides were removed by anion exchange chromatography by use of DEAE-Sepharose CL6B in its acetate form with water as eluent. Eluate fractions were scrutinized for their oligosaccharide content by MS and fractions containing the target compounds were combined and lyophilized, yielding 19 to 35 mg of the desired oligosaccharide composition.

Figure 1 schematically illustrates the preparation of an oligosaccharide composition in accordance with Example 1. Accordingly, in a first step carboxylic acid groups of the pectin are reduced to the corresponding alcohols. In a second step glycosidic bonds between the saccharide units are cleaved to yield the oligosaccharide composition. In a further step oligosaccharides comprising carboxylic acid groups which have not been reduced during the first step are removed by anion exchange chromatography. Figure 1 further shows a mass spectrum of the oligosaccharide composition in which oligosaccharides having two saccharide units (Gal₂), oligosaccharides having three saccharide units (Gal₃), oligosaccharides having four saccharide units (Gal₄),. oligosaccharides having five saccharide units (Gal₅) and oligosaccharides having six saccharide units (GaI₆) have been identified. "Gal" denotes galactose.

### Example 2

### Mass Spectrometry (MS)

Oligosaccharides and neoglycolipids were analyzed by nano electrospray ionization mass spectrometry (nanoESI MS). Oligosaccharides were dissolved in 50 % methanol and neoglycolipids in chloroform/methanol 2:8 (v/v). MS experiments were carried out by use of a SYNAPT G2-S mass spectrometer (Waters, Manchester, UK) equipped with a Z-spray source in the positive ion sensitivity mode. Typical source parameters were: source temperature: 80 °C, capillary voltage: 0.8 kV, sampling cone voltage: 20 V, and source offset voltage: 50 V. For low energy collision induced dissociation (CID) experiments, the corresponding precursor ions were selected in the quadrupole analyzer and fragmented in the trap cell using a collision gas (Ar) flow rate of 2.0 ml/min and collision energies up to 50 eV (Elab).

Figure 1 shows a mass spectrum of an oligosaccharide composition in accordance with the present invention as, for example, prepared by the method of Example 1.

Figure 3 shows a collision-induced dissociation (CID) mass spectrum and fragmentation pattern of a neoglycolipid having an oligosaccharide of four galactose units coupled to a phosphatidylethanolamine (Gal₄-PE).

### Example 3

### Preparation of Neoglycolipid Composition

10 mg of the oligosaccharide composition obtained in Example 1 was suspended in 150 µl of dimethylsulfoxide. Then 300 µl of methanol was added, whereupon the solids dissolved. 25 mg of dihexadecylglycerophosphoethanolamine (PE) was dissolved in 2.5 ml of chloroform/methanol (2:1 (v/v)). Both solutions were combined, and the obtained mixture was shaken at 60 °C for 2 h. Then a freshly prepared solution of sodium cyanoborohydride (10 mg/ml in chloroform/methanol (1:1 (v/v) containing 1 % acetic acid) was added in 8 portions of 0.1 ml over a period of 16 h and the mixture was kept at 60 °C. The progress of the reaction was monitored using thin layer chromatography (solvent chloroform/methanol/water, 60:35:8 (v/v), visualized with orcinol as staining reagent). When no residual oligosaccharide was detectable, the solvent was evaporated under a stream of nitrogen. The residue was suspended in 5 to 10 ml water, dialyzed (for 48 h against 2 I of water with 4 times water exchange), and lyophilized to yield 16.9 mg of a neoglycolipid/PE mixture.

Figure 2 schematically depicts formation of a neoglycolipid composition *via* reaction of an oligosaccharide composition as, for example, prepared according to Example 1 with a phosphatidylethanolamine. The progress of the reaction is monitored by thin layer chromatography using orcinol as staining reagent.

Figure 3 depicts a CID mass spectrum and fragmentation pattern of a neoglycolipid having an oligosaccharide of four galactose units coupled to a phosphatidylethanolamine (Gal₄-PE).

### Example 4

### Thin Layer Chromatography Overlay Assay For Determining Shiga Toxin

This method is similar to an enzyme-linked immunosorbent assay (ELISA) (Müthing J, Distler U. Advances on the compositional analysis of glycosphingolipids combining thin-layer chromatography with mass spectrometry. 2010 Mass Spectrom Rev 29, 425-479; Meisen I, Mormann M, Müthing J. Thin-layer chromatography, overlay technique and mass spectrometry: a versatile triad advancing glyocsphingolipidomics. 2011 Biochim. Biophys. Acta 1811, 875-896; Musken A, Souady J, Dreisewerd K, Zhang W, Distler U, Peter-Katalinic J, Miller-Podraza H, Karch H, Müthing J. Application of thin-layer chromatography/infrared matrix-assisted laser desorption/ionization orthogonal time-of-flight mass spectrometry to structural analysis of bacteria-binding glycosphingolipids selected by affinity detection. 2010 Rapid Commun Mass Spectrom 24, 1032-1038).

Three aliquots of a neoglycolipid composition obtained in Example 3 were separated in parallel on a thin layer chromatography plate. Application of the neoglycolipid composition to silica gel 60 precoated glass plates (HPTLC plates, size 10 cm x 10 cm, thickness 0.2 mm, no. 1.05633.0001; Merck, Darmstadt, Germany) was accomplished with an automatic sample applicator (Linomat IV; CAMAG, Muttenz, Switzerland). The neoglycolipid compositions were separated in a mixture of chloroform/methanol/water (60:35:8 (v/v/v)), supplemented with 2 mM CaCl₂ in a saturated chamber for 20 minutes. The thin layer chromatography plate was successively cut into two strips. Strip 1 (lane 1) was stained with orcinol. The strip was dipped for 15 seconds into 0.3% (w/v) orcinol in 3 M H₂SO₄, transferred onto a preheated heating plate (110 °C) and kept on the plate until pinkish-violet spots appeared. Strip 2 (lanes 2 and 3) was prepared for Shiga toxin and control antibody thin layer chromatography overlay assay. The silica gel was fixed with Plexigum P28 (Röhm, Darmstadt, Germany) to avoid detachment of silica gel from the glass plates. The strip was then cut into two halves (lanes 2 and 3, respectively). One half (lane 2) was subjected to the Shiga toxin thin layer chromatography assay (scheduled for subsequent mass spectrometry analysis, *vide infra*) and one half (lane 3) to the control assay. For the Shiga toxin assay the thin layer chromatography strip was saturated with 2% (w/v) bovine serum albumin (BSA) in phosphate buffered saline (PBS) for 2 h and then incubated with an Stx2a-containing bacterial supernatant for 2 h with slow agitation. Binding of Shiga toxin was visualized with an anti-Shiga toxin primary antibody and the appropriate alkaline phosphatase conjugated secondary antibody. Both antibodies were diluted 1:2000 with 1% (w/v) BSA in PBS and incubated for 1 h. Bound secondary antibodies were detected with 0.05% (w/v) 5-bromo-4-chloro-3-indolylphosphate *p*-toluidine salt (BCIP, no. 6368.3; Roth, Karlsruhe, Germany) in glycine buffer (0.1 M glycine), 1 mM ZnCl₂, 1 mM MgCl₂, pH 10.4). Between each incubation step the thin layer chromatography strip was washed three times with PBS and once before and after the substrate incubation with glycine buffer, respectively. The other half of the strip (lane 3) was used for an antibody thin layer chromatography overlay assay for detection of glycosphingolipids from erythrocytes as described in Meisen I, Friedrich AW, Karch H, Witting U, Peter-Katalinic J, Müthing J. 2005 Rapid Comm Mass Spectrom 19, 3659). The BCIP-stained chromatograms were stored at -20 °C until mass spectrometry analysis.

Figure 4 schematically depicts the thin layer chromatography overlay assay. In the left chart glycosphingolipids (GSLs) were separated and then detected in a reference method using a GSL-specific primary antibody and secondary antibody coupled to alkaline phosphatase. In the right chart GSLs were separated and then incubated with a Shiga toxin. The Shiga toxin is then detected using an anti-Shiga toxin primary antibody and an appropriate secondary antibody coupled to alkaline phosphatase. The blue bands result from staining with 5-bromo-4-chloro-3-indolylphosphate. For overlay assays on neoglycolipids GSLs may be replaced by neoglycolipids from a neoglycolipid composition according to the present invention. "TLC" denotes thin layer chromatography (taken from Müthing J, Distler U. 2010 Mass Spectrom Rev 29, 425-479, modified).

Figure 5 depicts thin layer chromatography overlay assays using a reference mixture of neutral glycosphingolipids from human erythrocytes comprising Gb3Cer and Gb4Cer as major compounds and a neoglycolipid composition comprising neoglycolipids Galₙ-PE of the present invention. In the left picture Gb3Cer (lane a) and the neoglycolipids (lane b) are detected using an anti-Gb3Cer antibody. In the right picture Gb3Cer and Gb4Cer (lane a) and the neoglycolipids (lane b) are detected using Shiga toxin of subtype Stx2a and an anti-Stx2 antibody. "Gb3Cer" denotes globotriaosylceramide. "Gb4Cer" denotes globotetraosylceramide. "Galₙ-PE" denotes neoglycolipids in which an oligosaccharide having n galactose units is coupled with a phosphatidyl ethanolamine "PE", wherein "PE" denotes dihexadecylglycerophosphoethanolamine.

Figure 6 depicts specific binding patterns of different Shiga toxin subtypes detected by thin layer chromatography overlay assays using a neoglycolipid composition comprising neoglycolipids Galₙ-PE according to the present invention. The chromatograms show that different subtypes of Shiga toxin show different binding specificity to neoglycolipids of a neoglycolipid composition according to the present invention. As a consequence, different specific binding patterns result for each subtype of Shiga toxin. These different binding patterns allow for identifying the subtype of the Shiga toxin used in the thin layer chromatography overlay assay. Consequently, the neoglycolipid composition of the present invention represents an analytic reagent for determining the subtype of a Shiga toxin. Therefore, disclosed herein is a method of determining a subtype of a Shiga toxin using the neoglycolipid composition of the present invention. In Figure 6 Shiga toxin subtypes Stx2a, Stx1 a and Stx2e (human) have been tested.

The bands of the chromatograms have been further analyzed using mass spectrometry.

### Mass spectrometry of neoglycolipids

The overlay strip was dipped three times in chloroform to remove Plexigum. The silica gel of an Stx-positive band was scraped from the TLC plate and the silica gel was extracted by shaking 3 times with 300 µl of chloroform/methanol (1:1, v/v) followed by centrifugation at 15,000 x g for 10 min, and combining the supernatants. The extraction solvent was evaporated *in vacuo* and the residue was redissolved in 20 µl of chloroform/methanol (2:8, v/v) for mass spectrometric analysis. The extracted neoglycolipids were analysed by nano electrospray ionization (nanoESI) MS. NanoESI MS experiments were carried out by use of a SYNAPT G2-S mass spectrometer (Waters, Manchester, UK) equipped with a Z-spray source in the positive ion sensitivity mode. Typical source parameters were: temperature: 80°C, capillary voltage: 1.0 kV, sampling cone voltage: 20 V, and offset voltage: 50 V.

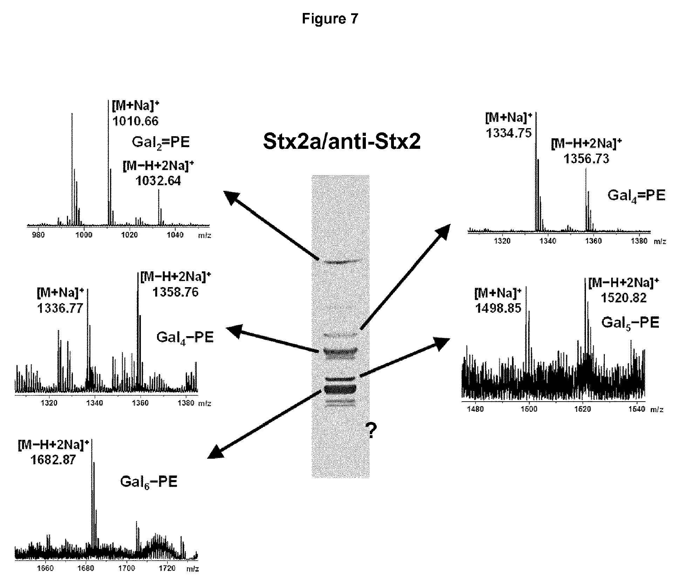

Figure 7 depicts the mass spectrometric analysis of thin layer chromatography bands obtained by a thin layer chromatography overlay assay carried out using a neoglycolipid composition of the present invention and Shiga toxin of subtype Stx2a. Galₙ=PE denotes the corresponding Amadori products to Galₙ-PE.

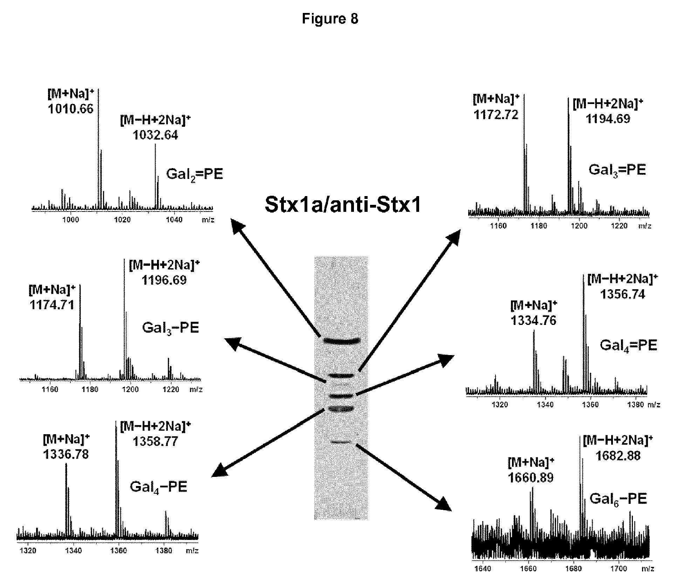

Figure 8 depicts the mass spectrometric analysis of thin layer chromatography bands obtained by a thin layer chromatography overlay assay carried out using a neoglycolipid composition of the present invention and Shiga toxin of subtype Stx1 a. Galₙ=PE denotes the corresponding Amadori products to Galₙ-PE.

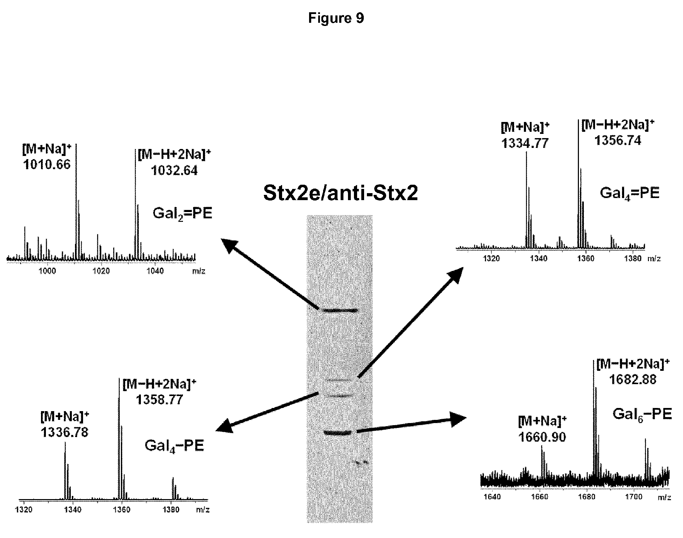

Figure 9 depicts the mass spectrometric analysis of thin layer chromatography bands obtained by a thin layer chromatography overlay assay carried out using a neoglycolipid composition of the present invention and Shiga toxin subtype Stx2e (human). Galₙ=PE denotes the corresponding Amadori products to Galₙ-PE.

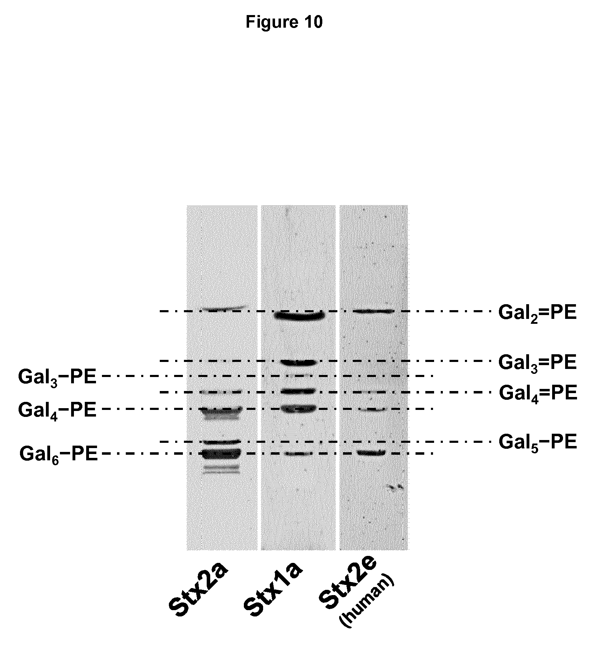

Figure 10 depicts binding patterns of Shiga toxin subtypes Stx2a, Stx1a and Stx2e (human) obtained by a thin layer chromatography overlay assay using a neoglycolipid composition according to the present invention. Figure 10 further shows the results of the mass spectrometric analysis and provides an assignment of the thin layer chromatography bands to neoglycolipids having a specific number of galactose units. Galₙ=PE denotes the corresponding Amadori products to Galₙ-PE.

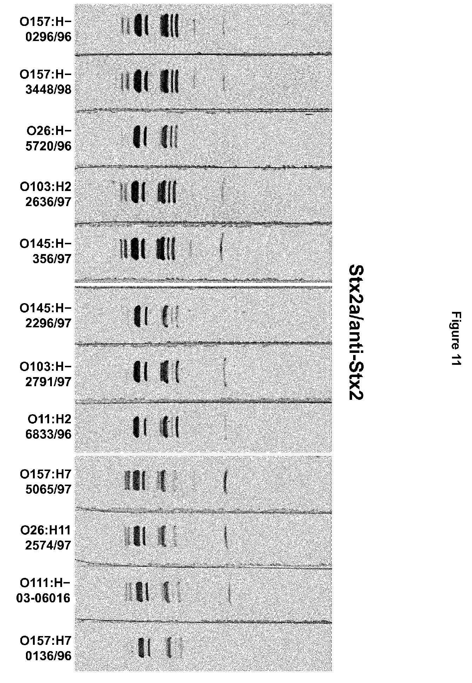

Figure 11 depicts binding patterns of Shiga toxin subtype Stx2a from different isolates of Shiga toxin producing *Escherichia coli* (STEC) obtained by a thin layer chromatography overlay assay using a neoglycolipid composition according to the present invention. All bands have been detected using Shiga toxin of subtype Stx2a. Stx2a-containing bacterial supernatants have been isolated from different strains of *Escherichia coli* and were used as indicated below the respective thin layer chromatography lanes. The results show that the method is suitable for determination of Shiga toxin subtypes which derive from different strains of Shiga toxin producing *Escherichia coli*(STEC).

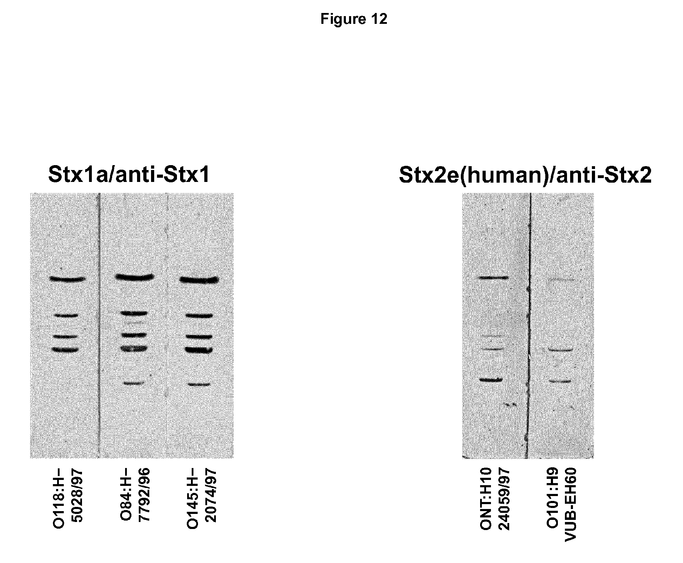

Figure 12 depicts binding patterns of Shiga toxin subtypes Stx1 a and Stx2e (human) from different isolates of Shiga toxin producing *Escherichia coli* (STEC) obtained by a thin layer chromatography overlay assay using a neoglycolipid composition according to the present invention. Also Figure 12 shows data for Shiga toxin subtype Stx1 a and Stx2e (human) isolated from different strains of *Escherichia coli* indicated below the respective thin layer chromatography plate.

### Example 5

### Cytotoxicity Assay

A reliable and robust Vero-B4 cell-based cytotoxicity assay was developed to determine the cytotoxic capacity of Shiga toxin (Stx)-containing bacterial supernatants produced with Shiga toxin producing *Escherichia coli*(STEC) of various serotypes.

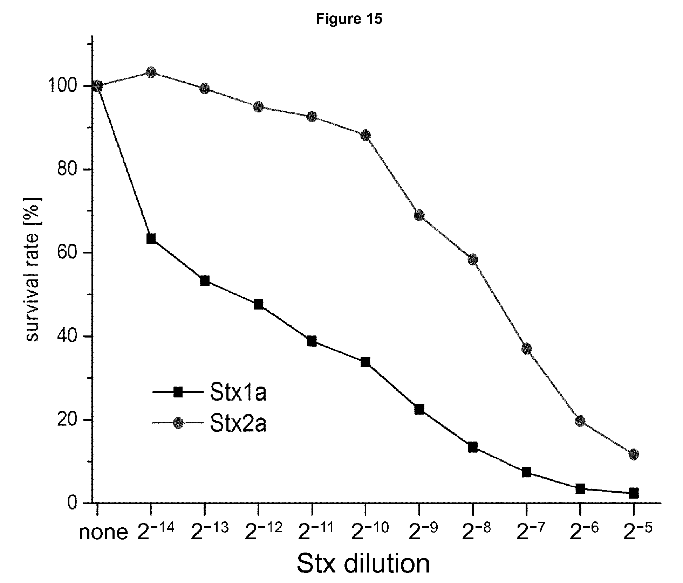

Figure 15 exemplarily shows the concentration-dependent damage of Stx1 a and Stx2a towards Vero-B4 cells. The data shows that the survival rate of the cells decreases when the concentration of the Shiga toxin increases with less dilution.

Vero-B4 cells were grown in serum-free medium (Gibco OptiPRO™ SFM, below called medium) to 70 to 75 % confluence. Cells were harvested by mild trypsin treatment, transferred to a 96-well microtiter plate (4 x 10³ cells in 100 µl medium per well), and incubated for 24 h at 37 °C and 5 % CO₂ in a humidified atmosphere. Stx-containing bacterial supernatants were serial 1:2 diluted in medium. 100 µl of Stx dilutions were applied to the appropriate wells while 100 µl medium was added to control cells. The plate was incubated for 1 h at 37 °C and 5 % CO₂, the supernatants were discarded, and 200 µl of fresh medium were added to each well. Subsequently the plate was incubated for 48 h at 37 °C and 5 % CO₂. For blank values the medium was removed from the corresponding wells and 200 µl of a 1 % Triton X-100 solution in medium were added. After incubation for 1 h at 37 °C and 5 % CO₂ all supernatants were discarded and cells were fixed by treatment with 100 µl of 2 % formaldehyde in PBS for 2 min. The formaldehyde solution was removed and the cells were stained with a 6.25 x 10⁻² % crystal violet solution in PBS containing 5 % ethanol and 1.85 % formaldehyde for 1 h at ambient temperature. The crystal violet solution was discarded and the wells were rinsed 3 times with water. After drying over night at 37 °C 100 µl of 50 % ethanol was applied to each well and the plate was shaken for 1 h at ambient temperature. Finally the extinction was determined at 570 nm by use of an ELISA reader. Survival rates were calculated relative to control values (100 %) after subtraction of the appropriate blank values.

### Example 6

### Fabrication of Neoglycolipid-Containing Liposomes

Liposomes were prepared by mixing the lipid constituents in organic solvents, followed by evaporation of the solvent, and hydration in phosphate-buffered saline (PBS) by ultrasonication to yield multilamellar vesicles. Unilamellar liposomes were finally obtained by extrusion through polycarbonate membranes with pore sizes of 50 to 100 nm. Size and quality of liposomes are monitored by dynamic light scattering measurements.

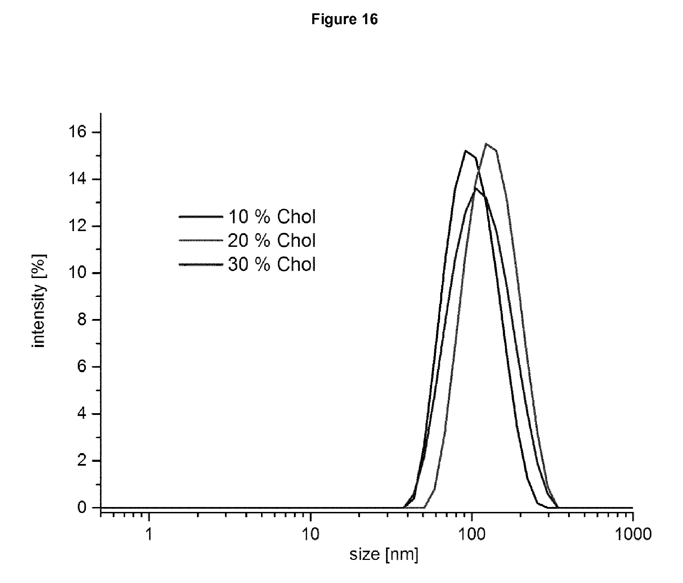

A representative size distribution of Galₙ-PE-containing liposomes is depicted in Figure 16 and demonstrates the production of uniformly sized vesicles of approximately 100 nm. "Chol" denotes cholesterol.

A mixture of 1 mg of total lipid consisting of dioleyl-phosphatidylcholine, the neoglycolipid composition obtained in Example 3 (up to 50 %, by weight) and cholesterol (up to 30 %, by weight) was prepared from chloroform/methanol solutions. After thorough mixing the solvent was evaporated *in vacuo.* For hydration 1 ml of PBS buffer was added and the mixture was sonicated in a bath-type sonicater at 45 °C for 20 min. The resulting suspension was extruded 11 times through polycarbonate membranes with a pore size of 50 to 100 nm at 45 °C. Small unilamellar vesicles with a mean diameter of 100 to 200 nm are obtained. Size and quality of the resulting liposomes were controlled by dynamic light scattering measurements. For a literature reference see Hepbildikler ST, Sandhoff R, Kölzer M, Proia R L, Sandhoff K. Physiological substrates for human lysosomal β-hexosaminidase S. 2002 J Biol Chem 277, 2562-2572.

### Example 7

### Inhibition of Stx-Mediated Cellular Damage by Galₙ-PE-Containing Liposomes

Figure 13 depicts schematically the binding of Shiga toxin (Stx) to globotriaosylceramide (Gb3Cer) receptors for Shiga toxin on the cell surface. Furthermore, Figure 13 shows the binding of Shiga toxin to oligosaccharides of an oligosaccharide composition of the present invention. By such binding to the oligosaccharides of the oligosaccharide composition of the present invention the binding of the Shiga toxin to the globotriaosylceramide receptors of the cells is prevented. Therefore, the survival rate of the cells in presence of a Shiga toxin is enhanced.

Similarly, also Figure 14 depicts schematically the binding of Shiga toxin (Stx) to globotriaosylceramide (Gb3Cer) receptors for Shiga toxin on the cell surface. In addition, Figure 14 shows the binding of Shiga toxin to neoglycolipids of a neoglycolipid composition of the present invention. The neoglycolipids have been formulated into liposomes. By the binding of the neoglycolipids of the neoglycolipid composition to the Shiga toxin the binding of the Shiga toxin to the globotriaosylceramide receptors of the cells is prevented. Therefore, the survival rate of the cells in presence of a Shiga toxin is enhanced.

For Stx inhibition experiments Vero-B4 cells were treated with either Stx, or Galₙ-PE-containing liposomes obtained in Example 6 in the absence and in the presence of Stx. Figures 17 and 18 demonstrate that on the one hand the neoglycolipid doped liposomes had *per se* no toxic effect and on the other hand Galₙ-PE inserted into liposomes substantially decrease Stx1 a- (Fig. 17) and Stx2a- (Fig. 18) mediated cytotoxicity towards Vero-B4 cells. In this regard, the left side of the bar chart of Figure 17 shows reference samples in which the cells have not been incubated with the Shiga toxin. In contrast, the right side of the bar chart of Figure 17 shows samples which have been incubated with Shiga toxin of subtype Stx1a. Similarly, the left side of the bar chart of Figure 18 shows reference samples in which the cells have not been incubated with the Shiga toxin. In contrast, the right side of the bar chart of Figure 18 shows samples which have been incubated with Shiga toxin of subtype Stx2a. The data of Figures 17 and 18 show that the survival rate of the cells in presence of a Shiga toxin is significantly increased when the cells are treated with a neoglycolipid composition of the present invention compared to the absence of such neoglycolipid composition. In this regard, the absence of any Shiga toxin inhibitor such as a neoglycolipid composition is indicated by "no inhibitor". "PE" denotes dihexadecylglycerophosphoethanolamine. Accordingly "Gb3-PE" denotes a globotriaosyl-phosphatidylethanolamine, wherein "PE" is dihexadecylglycerophosphoethanolamine.

Propagation of Vero-B4 cells and seeding into microtiter plates were performed as described in Example 5 above. Neoglycolipid-containing liposome preparations were diluted 1:2 with either medium or Stx-containing medium and preincubated for 1 h at 37 °C. 100 µl of the preincubated samples were added to the appropriate wells and the plate was incubated for 1 h at 37 °C and 5 % CO₂. The supernatants were discarded, and 200 µl of fresh medium was added to each well. All following steps were conducted as outlined for the cytotoxicity assay (see Example 5). Stx-containing bacterial supernatants with unknown concentrations of Stx have been used. However, within a given experiment the amount of Stx per well was the same. The amount of Gb3Cer was 20 µg/well. The amounts of Gb3-PE or Galₙ-PE were 16.5 to 19.5 µg/well (considering a content of 30 to 40 % of free PE in the respective Gb3-PE and Galₙ-PE preparations). Since comparable amounts of Gb3Cer, Gb3-PE and Galₙ-PE have been used in the experiments, the survival rates depicted in Figures 17 and 18 reflect the relative binding activity to the Shiga toxin.

## Claims

1. A method of preparing an oligosaccharide composition, said method comprising the steps of:
(a) providing a pectin; and
(b) performing, in any order, conversion of carboxylic acid groups to the corresponding alcohols and cleavage of glycosidic bonds under conditions suitable to give an oligosaccharide composition.

2. The method of preparing an oligosaccharide composition of claim 1, said method comprising the steps of:
(I)
(a) providing a pectin;
(b1) conversion of carboxylic acid groups of the pectin to the corresponding alcohols; and
(b2) cleavage of glycosidic bonds of the conversion product of step (b1) under conditions suitable to give an oligosaccharide composition;
or said method comprising the steps of:
(II)
(a) providing a pectin;
(b1) cleavage of glycosidic bonds of the pectin under conditions suitable to give an oligomeric pectin cleavage product; and
(b2) conversion of carboxylic acid groups of the cleavage product of step (b1) to the corresponding alcohols to give an oligosaccharide composition.

3. The method of any one of the preceding claims, wherein
(a) the method further comprises at least partial separation of oligosaccharides having non-reduced carboxylic acid groups; and/or
(b) the conversion of carboxylic acid groups to the corresponding alcohols comprises activating the carboxylic acid groups with a carbodiimide and reducing the obtained conjugate of the carboxylic acid groups and the carbodiimide using a borohydride.

4. The method of any one of the preceding claims, wherein the cleavage of glycosidic bonds is performed using hydrolysis of glycosidic bonds,
wherein preferably:
(a) the hydrolysis of glycosidic bonds is performed in presence of an acid, more preferably wherein the acid is trifluoroacetic acid;
(b) the hydrolysis is performed at a temperature of from 70 to 120 °C for 5 minutes to 3 hours, more preferably at a temperature of from 80 to 110 °C for 10 minutes to 60 minutes, still more preferably at a temperature of from 90 to 95 °C for 30 to 40 minutes, most preferably at a temperature of from 100 to 105 °C for 15 to 20 minutes;
(c) the pH of the reaction mixture is between 2 and 5, more preferably between 2.1 and 4, still more preferably between 2.2 and 3.5, most preferably between 2.3 and 3.0; and/or
(d) the hydrolysis of glycosidic bonds is performed using an enzyme, more preferably using a glycosidase.

5. An oligosaccharide composition obtainable or being obtained by the method of any one of claims 1 to 4.

6. The oligosaccharide composition of claim 5, wherein the oligosaccharide composition comprises one or more of an oligosaccharide selected from the group consisting of an oligosaccharide having 20 saccharide units, an oligosaccharide having 19 saccharide units, an oligosaccharide having 18 saccharide units, an oligosaccharide having 17 saccharide units, an oligosaccharide having 16 saccharide units, an oligosaccharide having 15 saccharide units, an oligosaccharide having 14 saccharide units, an oligosaccharide having 13 saccharide units, an oligosaccharide having 12 saccharide units, an oligosaccharide having 11 saccharide units, an oligosaccharide having 10 saccharide units, an oligosaccharide having 9 saccharide units, an oligosaccharide having 8 saccharide units, an oligosaccharide having 7 saccharide units, an oligosaccharide having 6 saccharide units, an oligosaccharide having 5 saccharide units, an oligosaccharide having 4 saccharide units, an oligosaccharide having 3 saccharide units, and an oligosaccharide having 2 saccharide units,
wherein preferably the oligosaccharides comprise one or more of a saccharide unit selected from the group consisting of galactose, galacturonic acid, and galacturonic acid alkyl ester.

7. A method of preparing a coupling product of an oligosaccharide composition, said method comprising the steps of:
(I)
(a) providing an oligosaccharide composition of any one of claims 5 to 6, in particular an oligosaccharide composition obtained by any one of the methods of preparing an oligosaccharide composition of any one of claims 1 to 4; and
(b) forming a coupling product of an oligosaccharide composition from the oligosaccharide composition and one or more of a coupling partner;
or said method comprising the steps of:
(II)
(a1) providing a pectin;
(a2) cleavage of glycosidic bonds of the pectin under conditions suitable to give an oligosaccharide composition;
(b) forming a coupling product of an oligosaccharide composition from the oligosaccharide composition and one or more of a coupling partner; and
(c) conversion of carboxylic acid groups of the coupling product of step (b) to the corresponding alcohols.

8. The method of claim 7, wherein the coupling partner is a phosphatidylethanolamine, said method of claim 7 item (I) comprising the steps of:
(I)
(a) providing an oligosaccharide composition of any one of claims 5 to 6, in particular an oligosaccharide composition obtained by any one of the methods of preparing an oligosaccharide composition of any one of claims 1 to 4; and
(b) forming a neoglycolipid composition from the oligosaccharide composition and one or more of a phosphatidylethanolamine having a structure of: 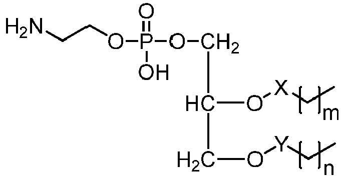 or any suitable salt thereof, wherein m and n is each, independently, an integer of from 8 to 20, wherein X and Y is each, independently, CH₂ or C=O; and wherein a carbon-nitrogen bond is formed between an oligosaccharide and a phosphatidylethanolamine;
or
said method of claim 7 item (II) comprising the steps of:
(II)
(a1) providing a pectin;
(a2) cleavage of glycosidic bonds of the pectin under conditions suitable to give an oligosaccharide composition;
(b) forming a neoglycolipid composition from the oligosaccharide composition and one or more of a phosphatidylethanolamine having a structure of: 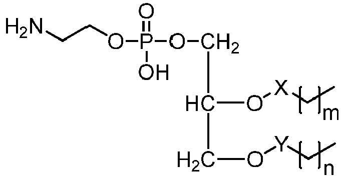 or any suitable salt thereof, wherein m and n is each, independently, an integer of from 8 to 20, wherein X and Y is each, independently, CH₂ or C=O; and wherein a carbon-nitrogen bond is formed between an oligosaccharide and a phosphatidylethanolamine; and
(c) conversion of carboxylic acid groups of the neoglycolipid composition of step (b) to the corresponding alcohols.

9. A coupling product of an oligosaccharide composition obtainable or being obtained by the method of any one of claims 7 to 8.

10. The coupling product of an oligosaccharide composition of claim 9, wherein the coupling product is a neoglycolipid composition obtainable or being obtained by the method of claim 8, wherein preferably the neoglycolipid composition comprises one or more of a neoglycolipid having the structure: 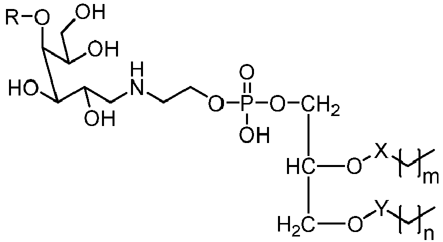 or any pharmaceutically acceptable salt thereof, wherein m and n is each, independently, an integer of from 8 to 20, wherein X and Y is each, independently, CH₂ or C=O; and wherein R is a residue comprising of from 1 to 19 saccharide units, wherein more preferably the residue R comprises one or more of a saccharide unit selected from the group consisting of galactose, galacturonic acid, and galacturonic acid alkyl ester.

11. A pharmaceutical composition comprising
(a) an oligosaccharide composition of any one of claims 5 to 6 and a pharmaceutically acceptable carrier, wherein preferably the oligosaccharide composition is formulated in lipid vesicles; or
(b) a coupling product of an oligosaccharide composition of any one of claims 9 to 10 and a pharmaceutically acceptable carrier, wherein preferably:
(i) the coupling product of an oligosaccharide composition is formulated in lipid vesicles; and/or
(ii) the coupling product of an oligosaccharide composition is a neoglycolipid composition of claim 10.

12. The oligosaccharide composition of any one of claims 5 to 6, the coupling product of an oligosaccharide composition of any one of claims 9 to 10 or the pharmaceutical composition of claim 11 for use in treatment of a disease.

13. The oligosaccharide composition of any one of claims 5 to 6, the coupling product of an oligosaccharide composition of any one of claims 9 to 10 or the pharmaceutical composition of claim 11 for use in treatment of a disease caused by a lectin or lectin producing pathogens, wherein preferably:
(a) the disease is caused by Shiga toxin or Shiga toxin producing pathogens;
(b) the disease is an infection by uropathogenic *Escherichia coli* (UPEC) or the disease is caused by an infection with uropathogenic *Escherichia coli*(UPEC); or
(c) the disease is an infection by *Streptococcus suis* or wherein the disease is caused by an infection with *Streptococcus suis.*

14. A method of determining a lectin in a sample comprising contacting a sample suspected to contain a lectin with a neoglycolipid composition as defined in claim 10, wherein preferably:
(a) the determining comprises determining of the absence or presence, the amount or the subtype of the lectin in the sample;
(b) the lectin which is to be determined is a Shiga toxin, more preferably wherein the Shiga toxin is a Shiga toxin 1 selected from the group consisting of Stx1a, Stx1 c and Stx1 d and/or a Shiga toxin 2 selected from the group consisting of Stx2a, Stx2b, Stx2c, Stx2d, Stx2e, Stx2f and Stx2g; and/or
(c) the lectin is an adhesin, more preferably wherein the adhesin is a PapG adhesin from uropathogenic *Escherichia coli* (UPEC) and/or the adhesin is SadP from *Streptococcus suis.*

15. A kit comprising a neoglycolipid composition of claim 10 and at least one lectin, wherein preferably the lectin is a Shiga toxin, wherein more preferably the Shiga toxin is a Shiga toxin 1 selected from the group consisting of Stx1a, Stx1 c and Stx1 d and/or the Shiga toxin is a Shiga toxin 2 selected from the group consisting of Stx2a, Stx2b, Stx2c, Stx2d, Stx2e, Stx2f and Stx2g.
